# EUROPEAN PATENT APPLICATION

(11) **EP 4 517 327 A2**
(43) Date of publication of application: **05.03.2025**
(21) Application number: 24206415.2
(22) Date of filing: 05.06.2020
(51) Int. Cl.: G01N 33/68

(54) **METHODS OF IDENTIFYING ATTRIBUTES OF THERAPEUTIC PROTEINS**

(30) Priority: 05.06.2019 US 201962857637 P; 31.01.2020 US 202062968682 P
(62) Divisional of application: 20750792.2
(71) Applicant: Amgen Inc., Thousand Oaks, CA 91320-1799 (US)
(72) Inventor: BONDARENKO, Pavel, Thousand Oaks, 91320-1799 (US); CROUSE-ZEINEDDINI, Jill A., Thousand Oaks, 91320-1799 (US); KUHNS, Scott Thomas, Thousand Oaks, 91320-1799 (US); NICHOLS, Andrew, Thousand Oaks, 91320-1799 (US); XIAO, Gang, Thousand Oaks, 91320-1799 (US); SHI, Rachel Liuqing, Thousand Oaks, 91320-1799 (US)
(74) Representative: Dörries, Hans Ulrich

(57) **Abstract**

Provided herein are methods of identifying structures, e.g., attributes, of a therapeutic protein or a target that affect an interaction between the therapeutic protein and the target. In exemplary embodiments, the method comprises: (a) applying a stress to a first sample comprising therapeutic proteins or targets; (b) contacting the first sample with a second sample comprising targets or therapeutic proteins to form a mixture comprising (i) therapeutic protein- target complexes, (ii) unbound therapeutic proteins, and (iii) unbound targets; and (c) separating the mixture into at least two fractions, wherein an unbound fraction comprises unbound therapeutic proteins or unbound targets and a bound fraction comprises therapeutic protein-target complexes; and (d) for each of the unbound fraction and bound fraction, identifying and quantifying the abundance of the structures, e.g., attribute, present on a species of the therapeutic protein or target.

## Description

### CROSS REFERENCE TO RELATED APPLICATIONS

The present Application claims the benefit of U.S. Provisional App. No. 62/857,637, filed June 5, 2019, entitled "Methods of Identifying Attributes of Therapeutic Proteins," and U.S. Provisional App. No. 62/968,682, filed January 31,2020, entitled "Methods of Identifying Attributes of Therapeutic Proteins," each of which is incorporated by reference in its entirety herein.

### INCORPORATION BY REFERENCE OF MATERIAL SUBMITTED ELECTRONICALLY

Incorporated by reference in its entirety is a computer-readable nucleotide/amino acid sequence listing submitted concurrently herewith and identified as follows: 268,171 byte ASCII (Text) file named "A-2296-WO-PCT_Seqlisting.txt"; created on June 3, 2020.

### BACKGROUND

The native structures of proteins adapt to changes within the protein's environment. Although this flexibility in structure is required for the biological function of most, if not all, proteins, it also presents many challenges during the development of therapeutic proteins for pharmaceutical applications. For example, therapeutic proteins endure various conditions during the many process steps that lead up to being administered to a patient. The many process steps include, for instance, one or more of protein production (e.g., recombinant production), harvest, purification, formulation, filling, packaging, storage, delivery, and final preparation immediately prior to administration to the patient. During each of these steps, a therapeutic protein is placed in one or more environments that may or may not lead to a change in its structure. The change in structure can lead to the formation of different species of the therapeutic protein that results in a heterogeneous product. While some species retain their ability to bind to their targets and therefore maintain therapeutic efficacy, others lose target binding ability and thus become functionally inactive. In order to maximize and maintain quality control of these pharmaceutical products, the biopharmaceutical industry has focused much effort to understand why some species lose activity while others retain activity. Current methodologies exist for analyzing species formation of therapeutic proteins. However, current techniques are impractical with regard to time and cost, while others fail to mirror therapeutic proteins in a physiologically-relevant context. Also, some current techniques use a covalent cross-linking agent or a labeling agent which can negatively impact the results of the analysis. Accordingly, there is a need for methods of identifying the species of therapeutic proteins that lead to the loss of binding, as well as identifying the conditions that lead to the formation of such species.

### SUMMARY

Described herein for the first time is the development of methods for identifying attributes of a therapeutic protein that affects an interaction (e.g., binding interaction) between the therapeutic protein and its target and the validation of such methods. As described herein, exemplary methods of the present disclosure led to the identification of fifteen attributes of a first therapeutic protein, which attributes were statistically significantly changed in the unbound fraction relative to the bound fraction, and thus such attributes were identified as relevant to the interaction (e.g., binding interaction) between the first therapeutic protein and its target. The exemplary methods of the present disclosure also led to the identification of 22 attributes of a second therapeutic protein, which attributes were statistically significantly changed in the unbound fraction relative to the bound fraction, and thus were identified as relevant to the interaction (e.g., binding interaction) between the second therapeutic protein and its target. The data provided herein support that the presently disclosed methods are more accurately and precisely identify attributes that impact the interaction between a therapeutic protein and its target (e.g., binding target).

Accordingly, the present disclosure provides methods of identifying structures, e.g., attributes, of a therapeutic protein or a target that affect an interaction between the therapeutic protein and the target. In exemplary embodiments, the method comprises (a) applying a stress to a first sample comprising therapeutic proteins or targets; (b) contacting the first sample with a second sample comprising targets or therapeutic proteins to form a mixture comprising (i) therapeutic protein- target complexes, (ii) unbound therapeutic proteins, and (iii) unbound targets; (c) separating the mixture into at least two fractions, wherein an unbound fraction comprises unbound therapeutic proteins or unbound targets and a bound fraction comprises therapeutic protein-target complexes; and (d) for each of the unbound fraction and bound fraction, identifying and quantifying the abundance of the structure, e.g., attribute, present on a species of the therapeutic protein or the target. For example, the abundance of each structure present on the species of the therapeutic protein or target can be identified and quantified. In exemplary aspects, when the abundance of a structure, e.g., attribute, in the unbound fraction is greater than the abundance of the structure, e.g., attribute, in the bound fraction, the structure, e.g., attribute, negatively affects the interaction between the therapeutic protein and the target.

In various aspects, the mixture is separated into at least two fractions using a technique that separates components of a mixture based on size, charge, hydrophobicity, affinity for a capture molecule, or a combination thereof. In exemplary instances, the technique is size exclusion chromatography (SEC), affinity chromatography, precipitation using beads or cells, free flow fractionation (FFF), ion exchange chromatography (IEX), hydrophobic interaction chromatography (HIC), or ultracentrifugation (UC). In additional or alternative aspects, the mixture is separated into at least two fractions using a technique that separates components of a mixture based on size, optionally, wherein the technique is size exclusion chromatography (SEC). In various instances, the mixture is separated into at least two fractions using a technique that separates components of a mixture based on affinity for a capture molecule bound to a solid support, optionally, a bead or a cell. The capture molecule in exemplary aspects is the target or an Fc receptor (e.g., FcRn, Fcy receptor).

The present disclosure also provides methods of determining an effect of a known structure, e.g., known attribute, present on a species of a therapeutic protein or a target on an interaction between the therapeutic protein and the target. In exemplary embodiments, the method comprises (a) contacting a first sample comprising a therapeutic protein or target with a known structure, e.g., attribute, with a second sample comprising targets or therapeutic proteins to form a mixture comprising (i) therapeutic protein- target complexes, (ii) unbound therapeutic proteins, and (iii) unbound targets; (b) separating the mixture into at least two fractions, wherein an unbound fraction comprises unbound therapeutic proteins or targets and a bound fraction comprises therapeutic protein-target complexes; and (c) for each of the unbound fraction and bound fraction, quantifying the abundance of the known structure, e.g., attribute. In exemplary aspects, when the abundance of the known structure, e.g., attribute, in the unbound fraction is greater than the abundance of the known structure, e.g., attribute, in the bound fraction, the known structure, e.g., attribute, has a negative effect on the interaction between the therapeutic protein and the target.

Additional descriptions and guidance, as well as exemplification, of the presently disclosed methods are provided herein.

### BRIEF DESCRIPTION OF THE DRAWINGS

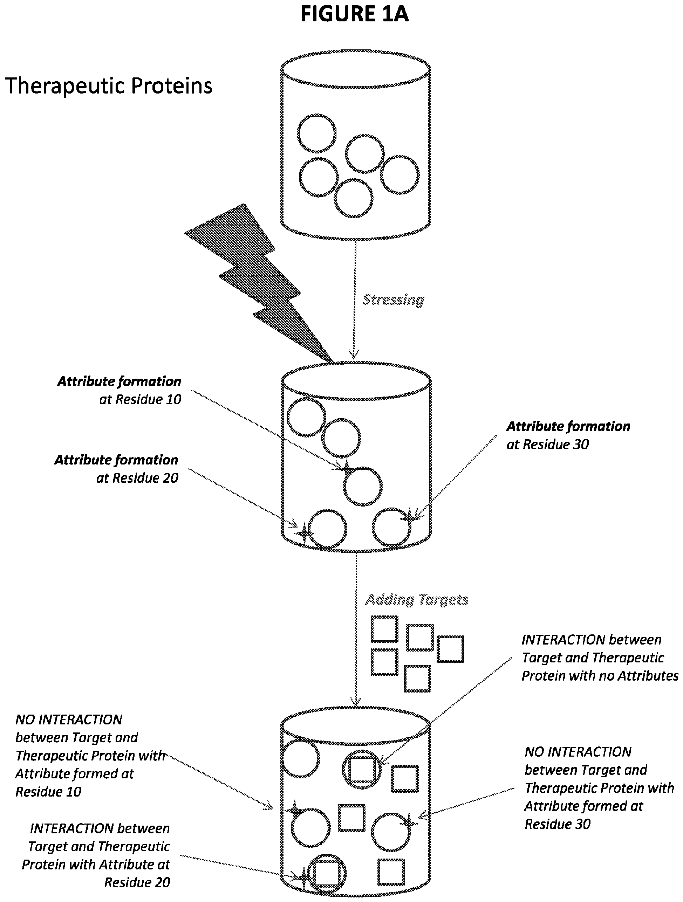

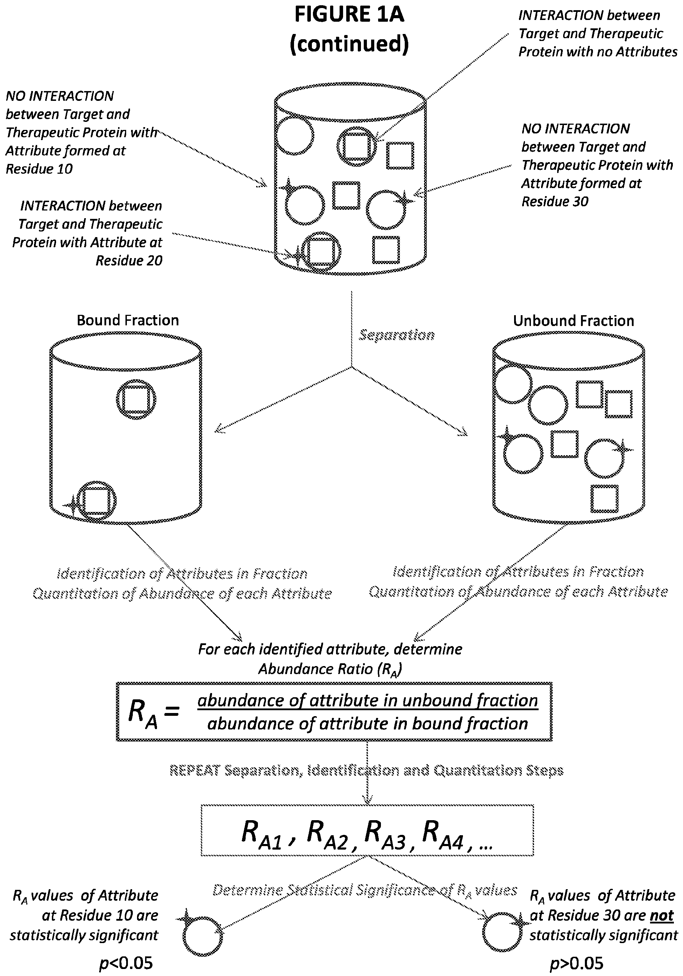

Figure 1A is an illustration of an exemplary workflow of a presently disclosed method of identifying attributes of a therapeutic protein that affect an interaction between the therapeutic protein and a target. Stress is applied to therapeutic proteins resulting in attribute formation at different amino acid residues. Each therapeutic protein having a different profile of attributes is considered a species. Upon adding targets to stressed therapeutic proteins, interactions between species of therapeutic proteins and target form. For some species, interactions between species of therapeutic proteins and target do not form. The mixture of therapeutic protein-target complexes, unbound therapeutic proteins, and unbound targets are separated into different fractions including a bound fraction and unbound fraction. Each attribute in each fraction is identified by attribute type (e.g., oxidation, deamidation, etc.) and location (e.g., amino acid residue position). The abundance of each attribute in each fraction is determined. For each attribute, a ratio of the abundance of an attribute in the unbound fraction to the abundance of an attribute in the bound fraction is determined. The method is repeated starting with the separation step and a second ratio is determined. The method is repeated again. Each time a new ratio is determined. The statistical significance of the ratios is determined. When the ratio is statistically significant, that attribute is considered as one that affects an interaction between the therapeutic protein and a target.

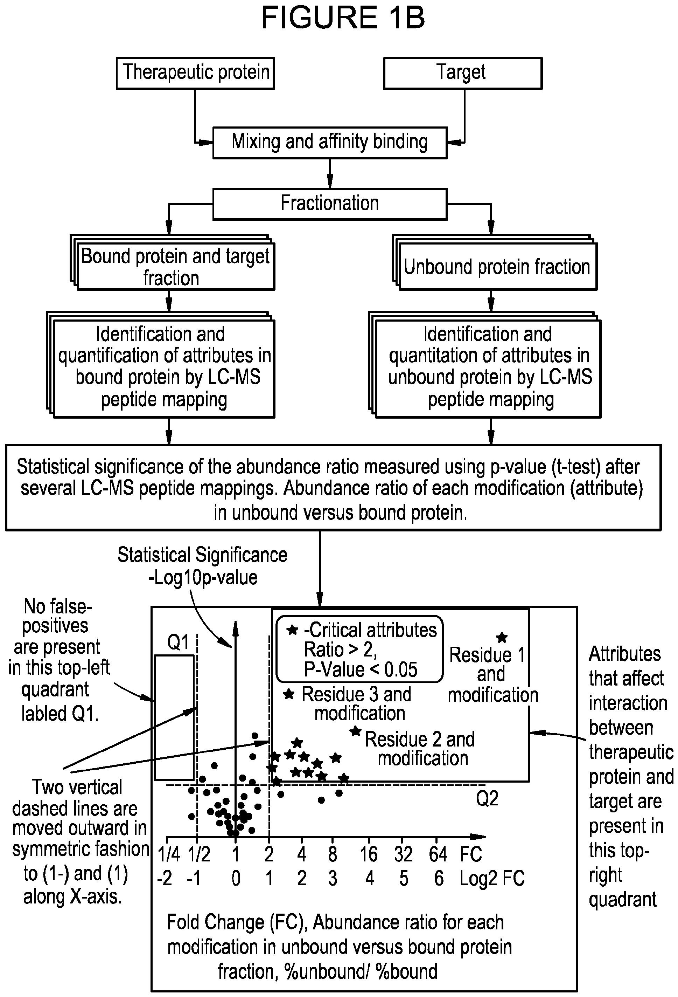

Figure 1B is an illustration of an exemplary workflow of a presently disclosed method of identifying attributes of a therapeutic protein that affect an interaction between the therapeutic protein and a target. In this illustration, the separation step of Figure 1A is labeled as "fractionation" and the identification and abundance determination is carried out by LC-MS peptide mapping. At least the LC-MS peptide mapping step is repeated several times and each time an abundance ratio for each attribute is determined, wherein the abundance ratio is the ratio of the abundance of an attribute in the unbound fraction to the abundance of an attribute in the bound fraction. The statistical significance of the abundance ratios is determined by determining the p-value upon a t-test. The statistical significance is plotted against the abundance ratio (or fold change) in a volcano plot. The round dots near fold change = 1 of the volcano plot represent (a) attributes that are not affecting interaction formation between target and therapeutic protein and (b) artificial attributes caused by the sample preparation. The attributes affecting binding are represented by stars on the top right corner.

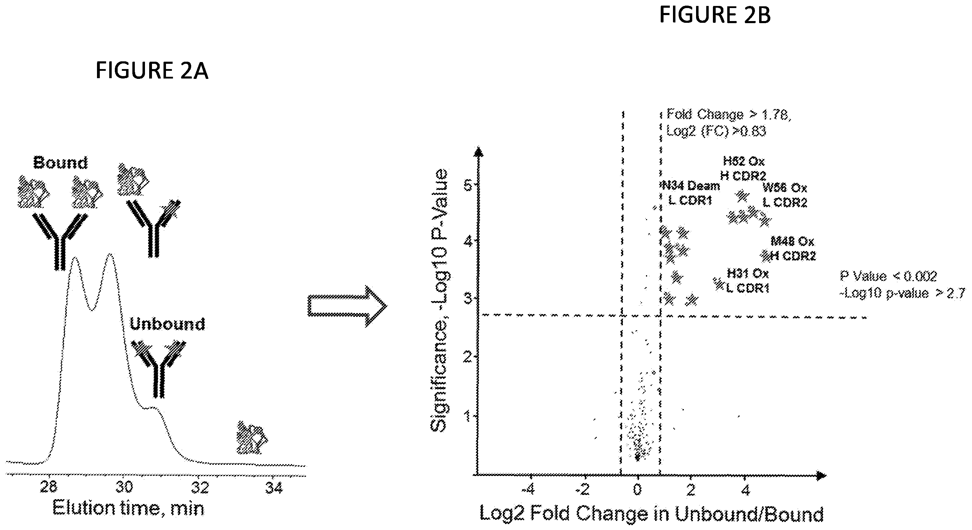

Figure 2A is an exemplary size exclusion chromatogram showing the elution of two targets bound to an arm of Antibody 1, one target bound to Antibody 1, Antibody 1 unbound to any targets, and unbound target. Figure 2B is a graph of the statistical significance of abundance ratios plotted as a function of abundance ratio (or fold change). p-value < 0.05 corresponds to -log10 p-value > 1.3; Fold Change > 1.78 corresponds to Log2 Fold Change > 0.83.

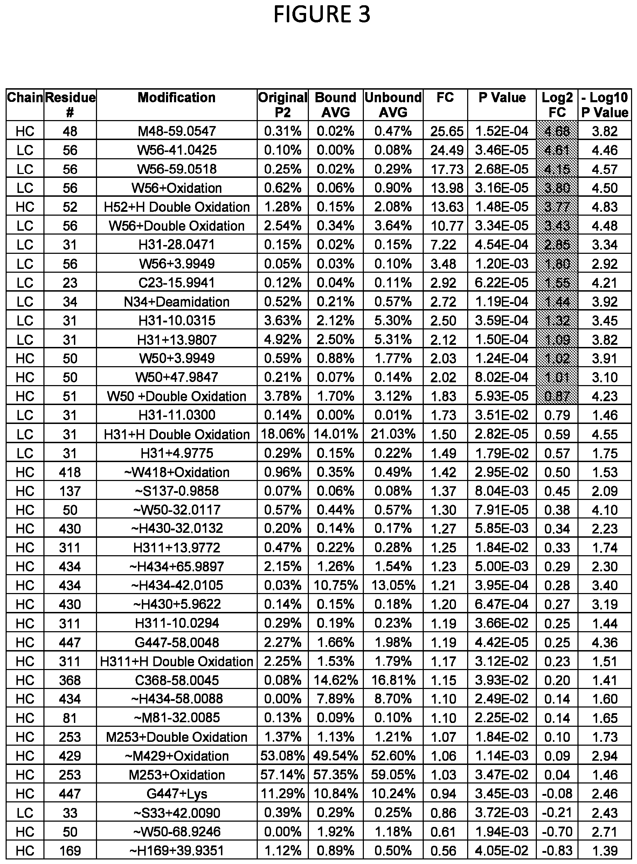

Figure 3 is an exemplary attribute profile listing 39 modifications with p-value < 0.05 out of total 188 modifications identified in Antibody 1. HC, heavy chain; LC, light chain; FC, fold change (ratio of the abundance of an attribute in the unbound fraction to the abundance of an attribute in the bound fraction) p-value as determined by t-test.

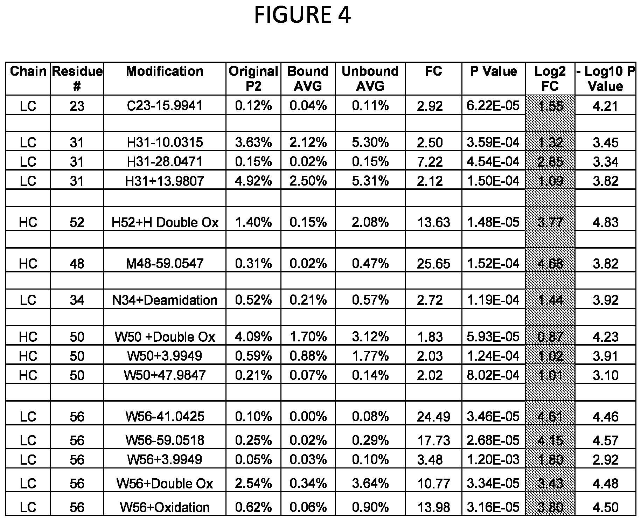

Figure 4 is an exemplary attribute profile listing 15 modifications on 7 residues of heavy and light chains of Antibody 1, which statistically significantly affect binding between Antibody 1 and its target. For these modifications, p-value < 0.002 (-log10 p-value > 2.7) and fold change > 1.78 (log2 fold change > 0.83. HC, heavy chain; LC, light chain; FC, fold change (ratio of the abundance of an attribute in the unbound fraction to the abundance of an attribute in the bound fraction) p-value as determined by t-test.

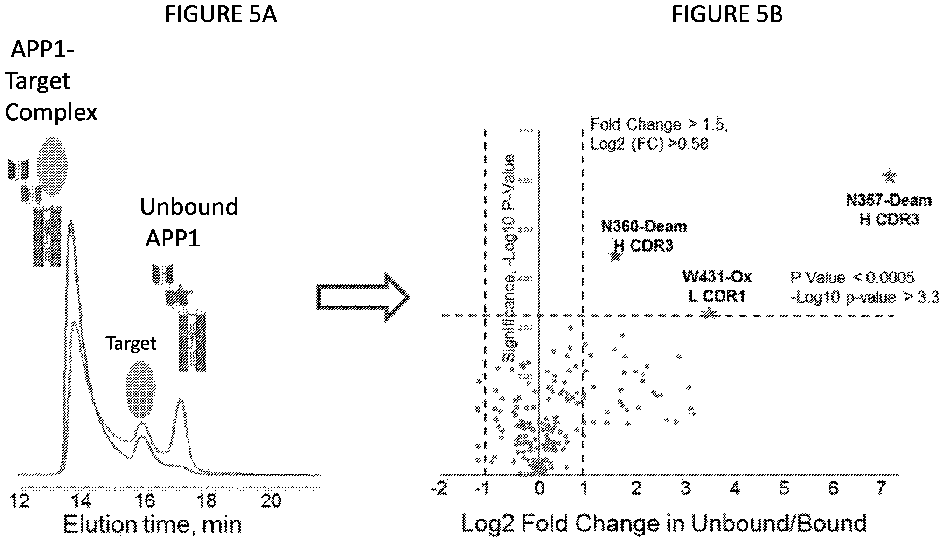

Figure 5A is an exemplary size exclusion chromatogram showing the elution of Antibody Protein Product 1 (APP1), which is an Fc fusion protein, bound to one target, unbound target, and unbound APP1. Figure 5B is a graph of the statistical significance of abundance ratios plotted as a function of abundance ratio (or fold change). p-value < 0.0005 corresponds to -log10 p-value > 3.3; Fold Change > 2 corresponds to Log2 Fold Change > 1.

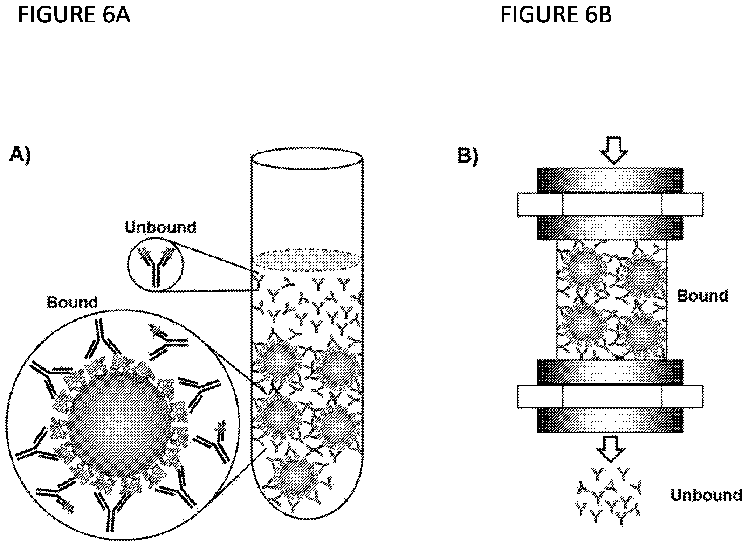

Figure 6A is an illustration of an exemplary workflow of a presently disclosed method of identifying attributes of a therapeutic protein that affect an interaction between the therapeutic protein and a target, wherein a bead bound to a capture molecule or a cell expressing on its surface a capture molecule is used. Stressed therapeutic proteins are contacted with the beads or cells. The capture molecule is the target. In the tube, a mixture of therapeutic protein-target complexes and unbound therapeutic proteins is formed. The beads or cells containing the bound therapeutic proteins may be separated from the unbound fraction by centrifuging. The beads or cells pellet with the bound therapeutic proteins and unbound proteins remain in supernatant. Figure 6B is an exemplary workflow of a presently disclosed method of identifying attributes of a therapeutic protein that affect an interaction between the therapeutic protein and a target, wherein a bead bound to a capture molecule. The capture molecule is the target. The beads bound to the target are loaded onto a column. The stressed therapeutic proteins are loaded onto the column and the flow-through contains the unbound fraction. The bound fraction is separately eluted and collected. This method is carried out for therapeutic proteins having a K_{D} of about 10⁻¹¹ to about 10⁻⁹ M for the target.

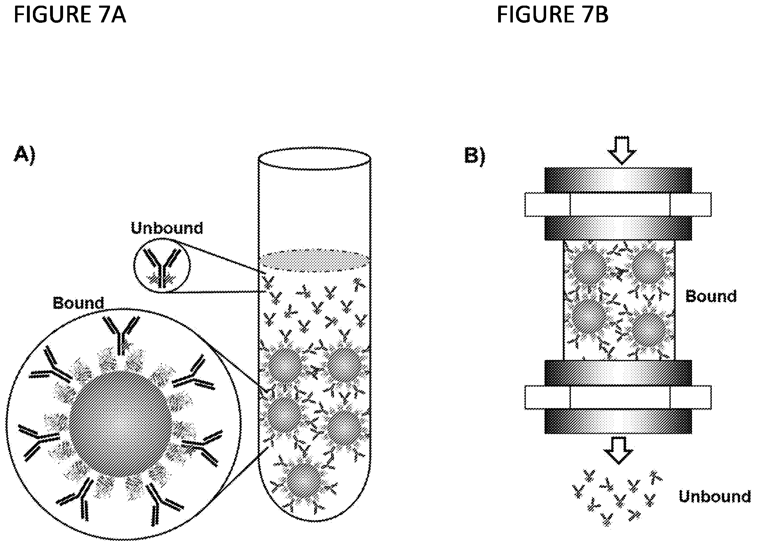

Figure 7A is an illustration of an exemplary method similar to that shown in Figure 6A, except that the beads are bound to Fc receptors or the cells express Fc receptors. This method is carried out for therapeutic proteins having a K_{D} of about 10⁻⁸ to about 10⁻⁶ M for the target.

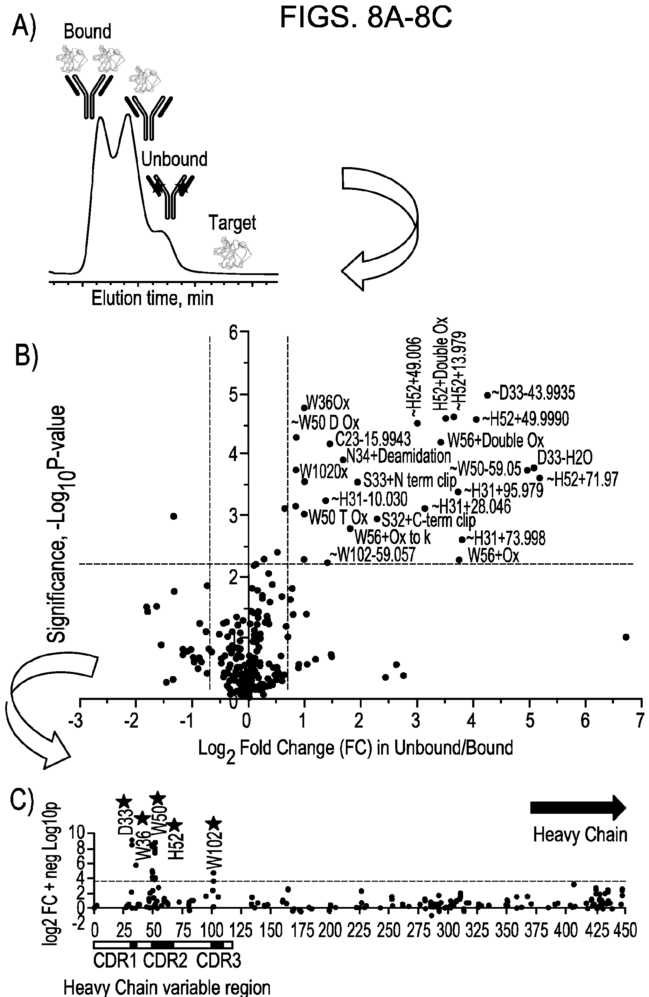

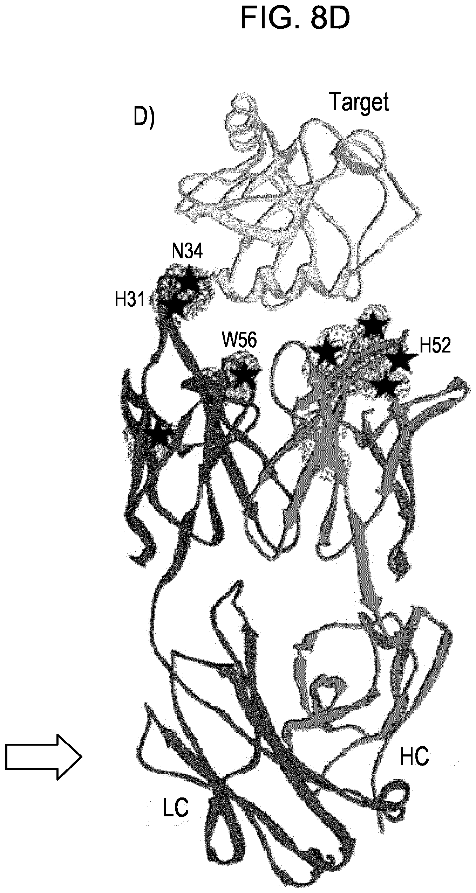

Figures 8A-8D show a workflow for identifying critical chemical modifications and paratope mapping by size exclusion chromatography (SEC) of stressed antibody-target complexes in accordance with some embodiments. A) Antibody is stressed in one or several of the following conditions: elevated temperature in the original formulation, for example at 40°C for 4 weeks (40C4W) in a mildly acidic formulation pH 5; physiological conditions (pH 7, 37°C); extremes of pH; UV light. UV light for 20 hours for total 200 watt hours per square meter was used in this study. Target protein was added and mixed with the stressed antibody in appropriate stoichiometric ratio, typically at antibody : target ratio of 1 : 2. After a short incubation, the mixture was injected into a SEC system and separated on bound and unbound antibody species as follows. Antibody bound to two target molecules eluted first, followed by antibody bound to one target molecule, and then unbound antibody and residual target. B) SEC fractions containing antibody with two bound targets (bound) and one bound target (partially unbound) were collected and subjected to peptide mapping. The fractions were chosen because they have similar protein concentration, which is important for equal sample preparation conditions. The proteins in the collected fractions were unfolded, disulfides reduced, alkylated, and protein backbone digested to peptides by an enzyme (trypsin) or a combination of enzymes. Chemical modifications on bound and unbound antibody species were identified and quantified by LC-MS/MS peptide mapping. The entire procedure was repeated several times (n=3), and statistical significance was calculated by performing the t-test using percentages of modifications in bound versus unbound antibody molecules. A graph (volcano plot) was plotted as statistical significance versus modifications abundance ratio (fold change) in unbound /bound antibody molecules. Statistically significant data were grouped in the top right corner bordered by the dashed lines for p-value < 0.006 (-log10 p-value > 2.2) and fold change > 1.78 (Log2 Fold Change > 0.83). Eleven (11) residues were modified with the specified statistical significance and modifications ratio in unbound/bound antibody. C) Paratope map was plotted as a modification score versus residue number. The modification score was chosen as a sum of logarithms for fold change and p-value (Log2 Fold Change + -log10 p-value). D) Correlation to available crystallography data of the antibody-target complex revealed that 8 out of 11 antibody residues with modifications identified by the method were within 5Å from the target, suggesting that these antibody residues are close to and likely to interact with the target.

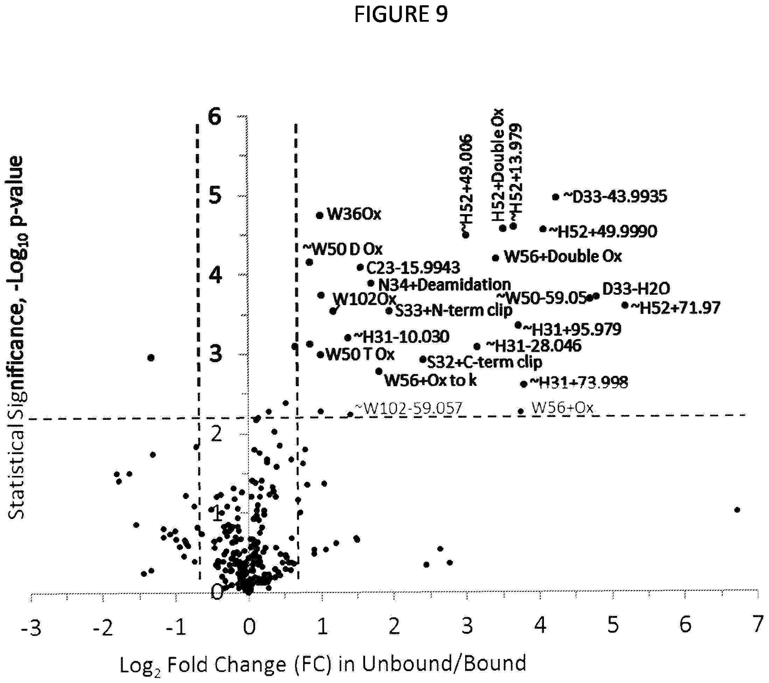

Figure 9 is a volcano plot of the statistical significance versus modifications abundance ratio (fold change) for unbound/bound antibody molecules according to some embodiments. Statistically significant data grouped in the top right corner bordered by the dashed lines for p-value < 0.006 (-log10 p-value > 2.2) and Fold Change > 1.78 (Log2 Fold Change > 0.83). The log scales are often used for volcano plots for better visualization. Positions of the two vertical dash lines for the fold change (log2 Fold Change) thresholds were selected by moving the lines symmetrically outward until the top-left quadrant was clear. The statistical significance (-log10 p-value) horizontal threshold line position was chosen by moving it up until the top-left quadrant was clear. These thresholds define the fold change and p-value for the top-right quadrant, in which the modifications reduce binding with statistical significance.

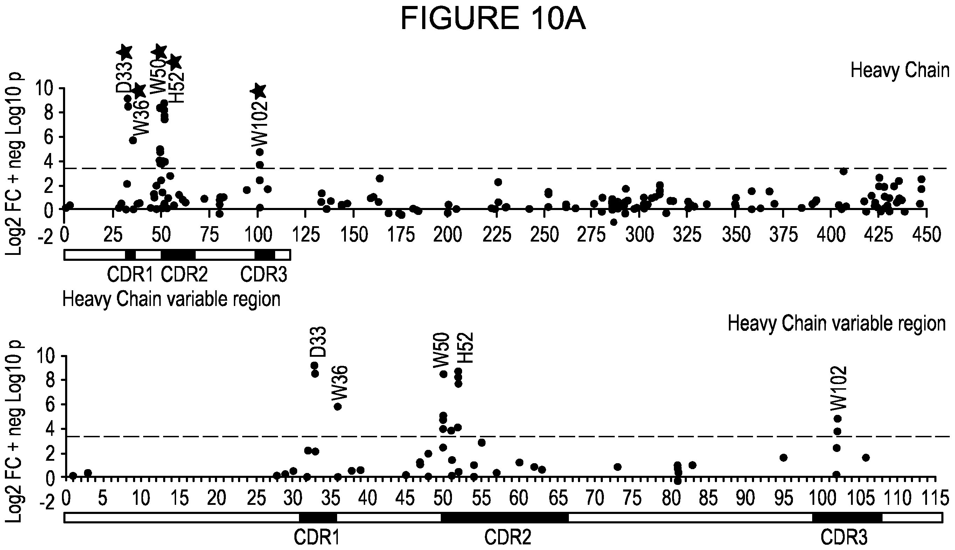

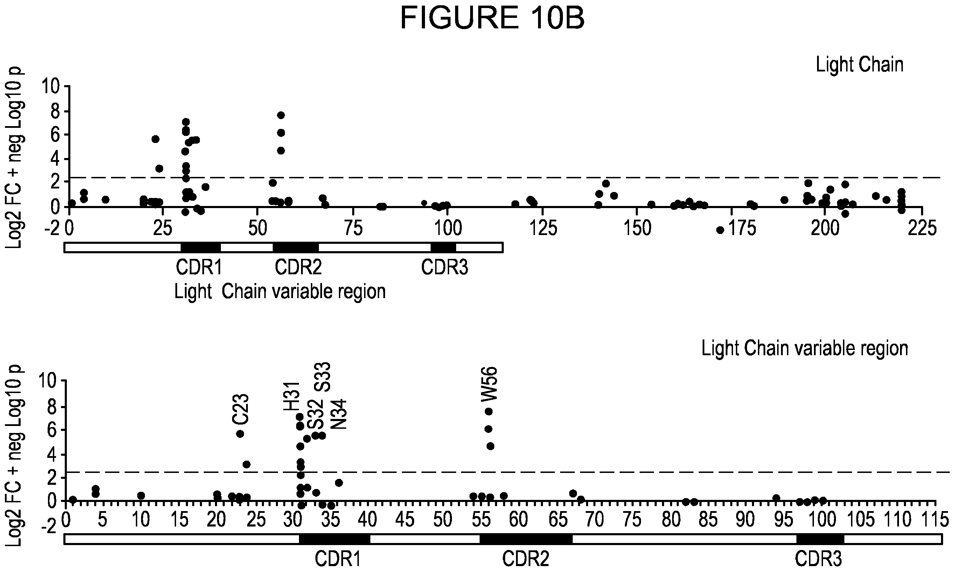

Figures 10A-10B show paratope mapping plots for A) heavy chain with a close view at its variable region and B) light chain with a close view at its variable region in accordance with some embodiments. Paratope map was plotted as a modification score versus residue number. The modification score was chosen as a sum of logarithms for fold change and p-value (Log2 Fold Change + -log10 p-value), because the scales have similar values and both are important for separating signal from noise. Out of 11 residues with highest score (labeled on the plots), 10 were from complementarity determining regions (CDRs), regions typically involved in binding. Residues in the conserved regions, typically not involved in binding, had significantly lower score. Correlation to available crystallography data of the antibody-target complex revealed that 8 out of 11 antibody residues with modifications identified by the method were within 5Å from the target, indicating that these antibody residues are close to and likely to interact with the target. These results supported validity of the method. A modification on a cysteine residue (C23) outside of CDRs was also detected. Without being limited by theory, this result suggested that long-range (allosteric) effects may play a role caused by the reduced disulfide bond. Among the 11 residues, clipping at LC S32/S33 was represented by two dots (for S32 and S33) for the two detected fragments significantly increased in the unbound SEC fraction. Although clipping reduced binding to target, and it is a critical attribute, these residues may not be involved in binding to target. Without being limited by theory, a part of antibody molecule could be simply lost as a result of the clipping. Paratope mapping can generally be performed without clipping data.

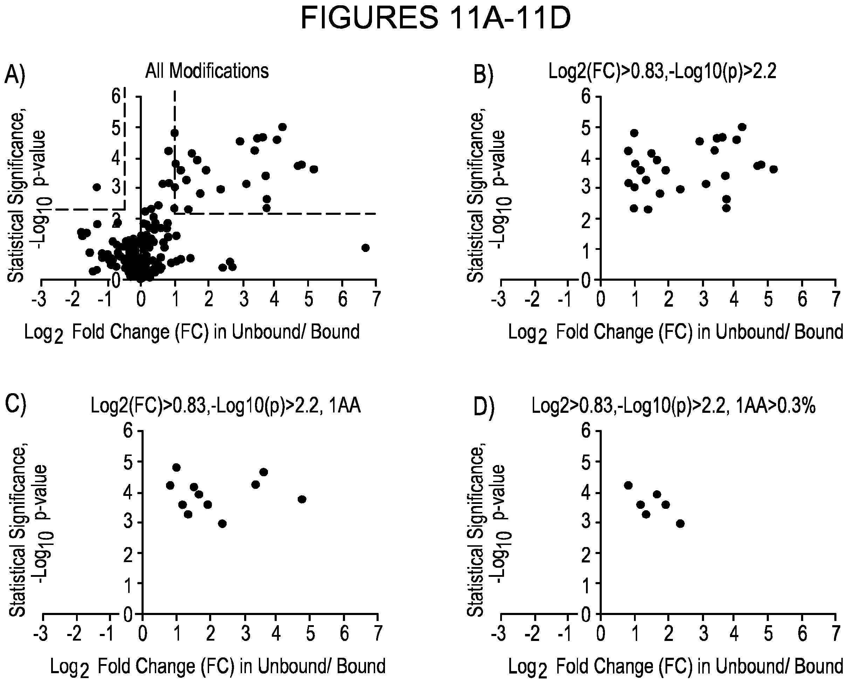

Figures 11A-11D show volcano plots for: A) All 275 chemical modifications uncovered by peptide mapping on amino acid residues of the stressed therapeutic antibody. B) 27 modifications on 11 residues statistically significantly impacting binding to the target and satisfying the following criteria: Log₂(FC)>0.83 and -Log₁₀(p)>2.2. C) 11 residues of the antibody identified as the residues involved in binding to the target (paratope). D) 6 residues with modifications exceeding 0.3% after 40°C stress in the liquid formulation for 4 weeks (40C4W). 40C4W accelerated stress was chosen to simulate the reasonable conditions of production and storage during shelf life of antibodies in mildly-acidic liquid formulation at 4°C for 2 years. Modifications on the 6 residues impact binding, have relatively large value under the reasonable conditions of production and storage and should be considered as critical attributes and monitored. 40C4W stress caused very minor modifications on other 5 residues (<0.01%). UV light caused higher modifications on these residues, which were useful for paratope mapping.

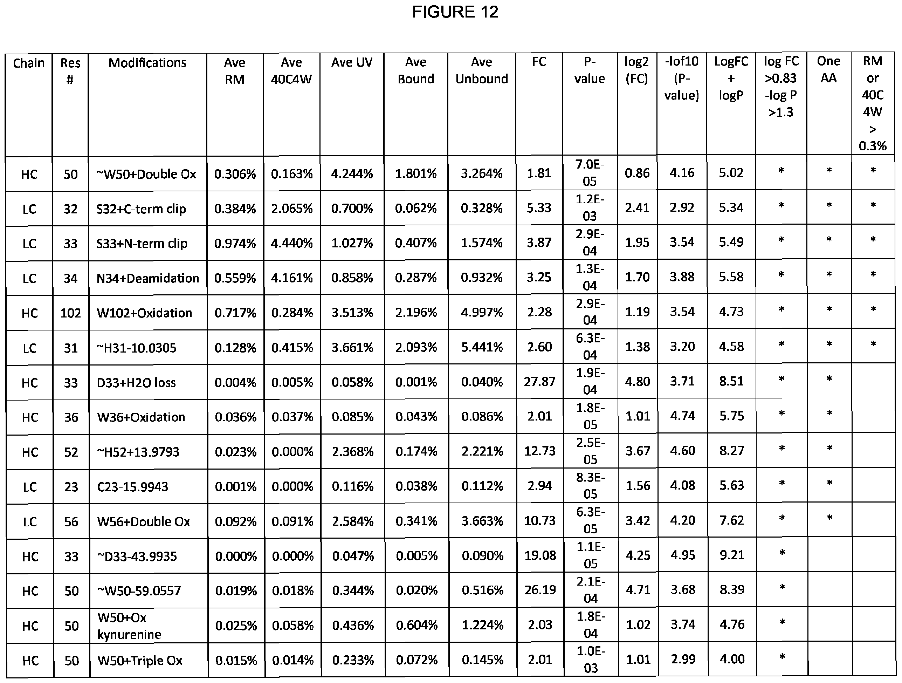

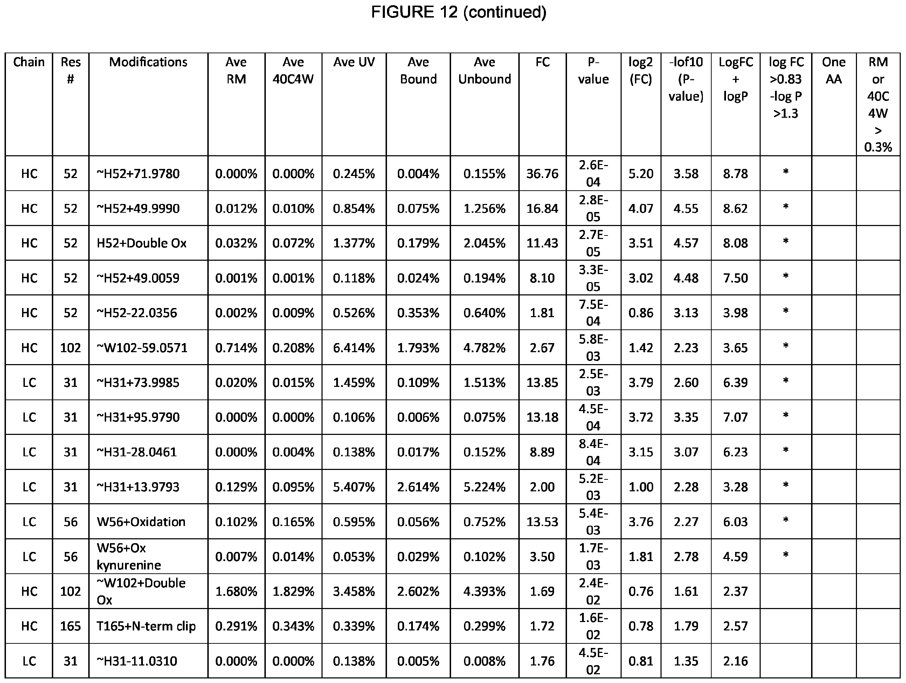

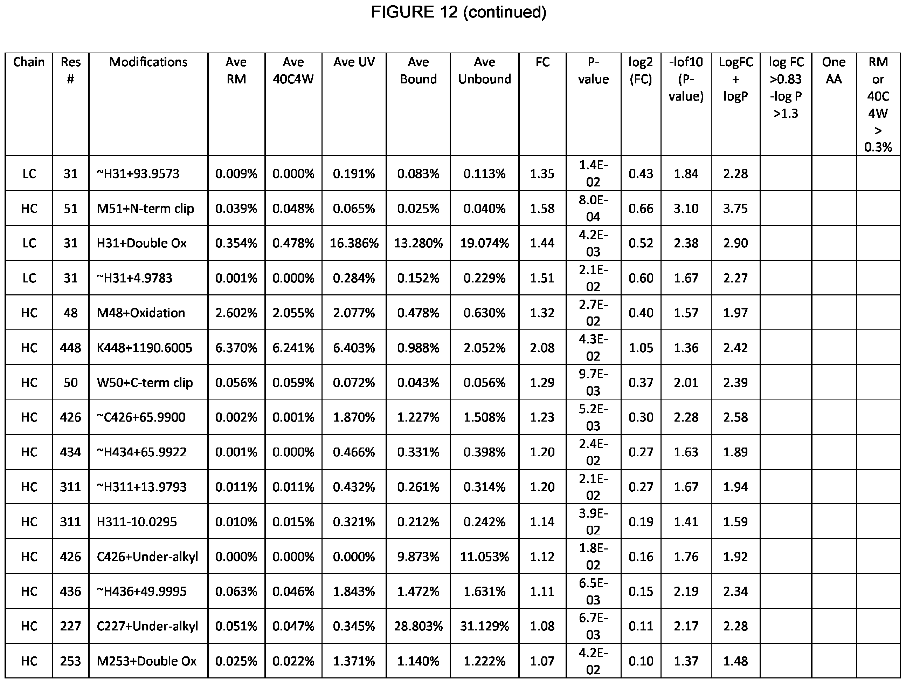

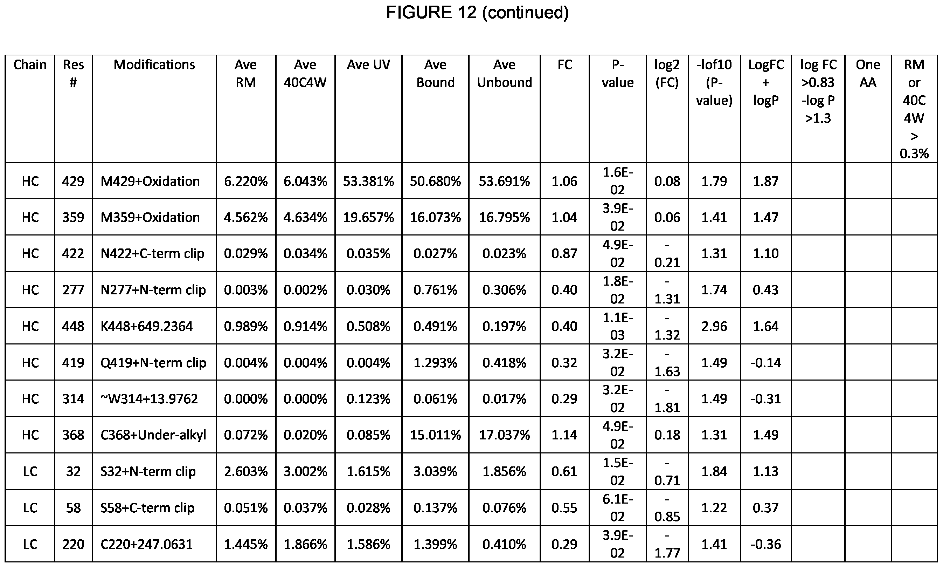

Figure 12 is a chemical modifications profile listing 56 modifications with p-value < 0.05 - Log₁₀(p)>1.3 out of total 275 modifications identified on the stressed antibody used in a study in accordance with some embodiments herein (See Example 6). Of them, 27 modifications satisfied criteria of statistical significance: Log₂(FC)>0.83 and -Log₁₀(p)>2.2. These modifications occurred on 11 residues, more than one modification on some residues. The 11 residues of the antibody were identified as the residues involved in binding to the target (paratope). On 6 residues (including two residues with a clip between them), the modifications exceeded 0.3% after 40°C stress in the liquid formulation for 4 weeks (40C4W).

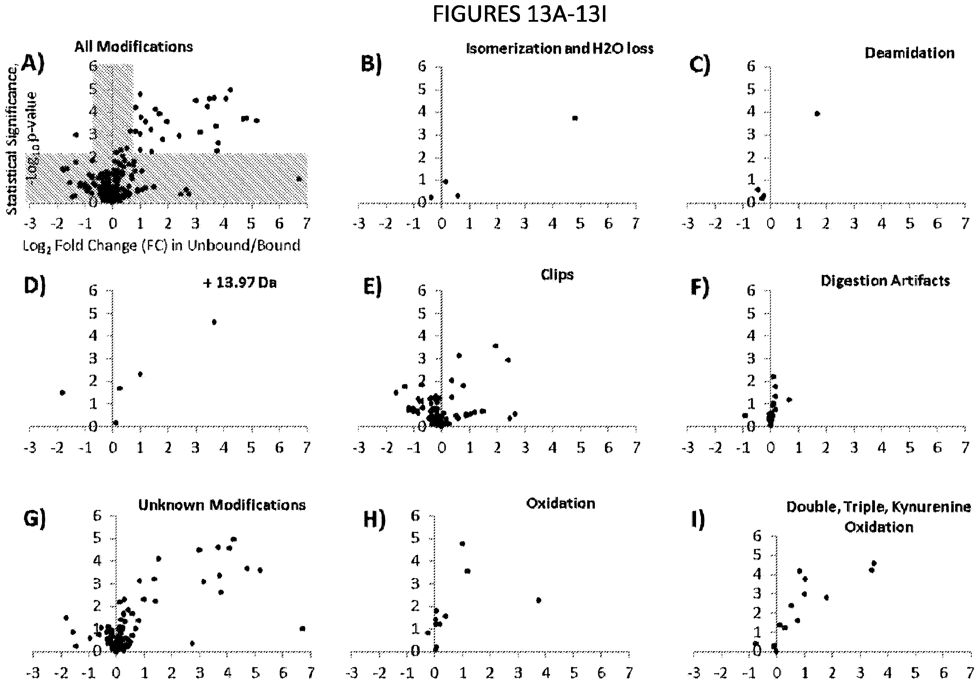

Figures 13A-13I are volcano plots for individual chemical modifications of statistical significance versus abundance ratio in unbound/bound antibody fractions according to some embodiments. The following modifications were plotted: A) All modifications. B) Isomerization and H20 loss, the later for succinimide. C) Deamidation; D) +13.97 Da, which is double oxidation and H2O loss modification on H, W; E) Clips; F) Digestion Artifacts including unintended carboxymethylation of H, M, and also under-alkylation of several C residues; G) Unknown Modifications including oxidation, hydroxylation, H2O loss identified on "unusual residues" and several other modifications identified only by mass change; H) Oxidation identified mainly on M and probably caused by dissolved oxygen; I) Double, Triple Oxidation, Oxidation to Kynurenine identified mainly on W, H and likely caused by radicals, metals and light. The plots were used for quality control applying the following rationale. If SEC unbound and bound antibody fractions were collected and processed similarly, then noise is expected to be symmetrical at the basis of the volcano and around the vertical axis Log2(FC) = 0. The noise includes the area with -log10(p-value) < 2.2, and also area -log10(p-value) > 2.2 and - 0.7<log2(FC)<0.83 (greyed on All Modifications plot). If the dots in the noise area are distributed not symmetrically with respect to the vertical axis, then bound and unbound fractions were treated differently. Majority of dots for Double, Triple Oxidation, oxidation to kynurenine are slightly shifted to the right, suggesting that unbound fraction may be exposed more to radicals, metals and light. Otherwise, both fractions were treated similarly with respect to factors causing other modifications.

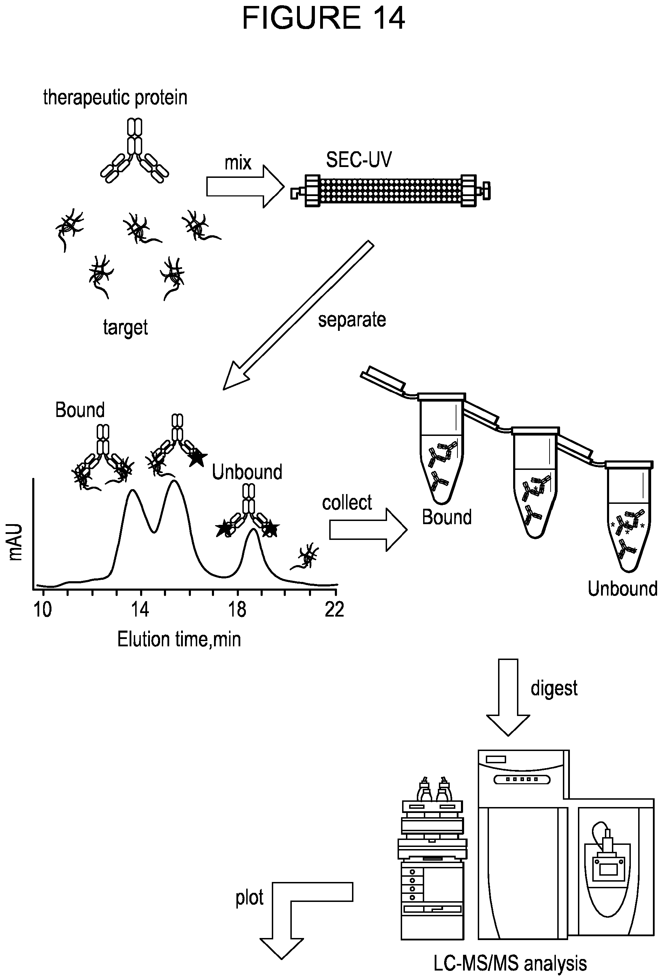

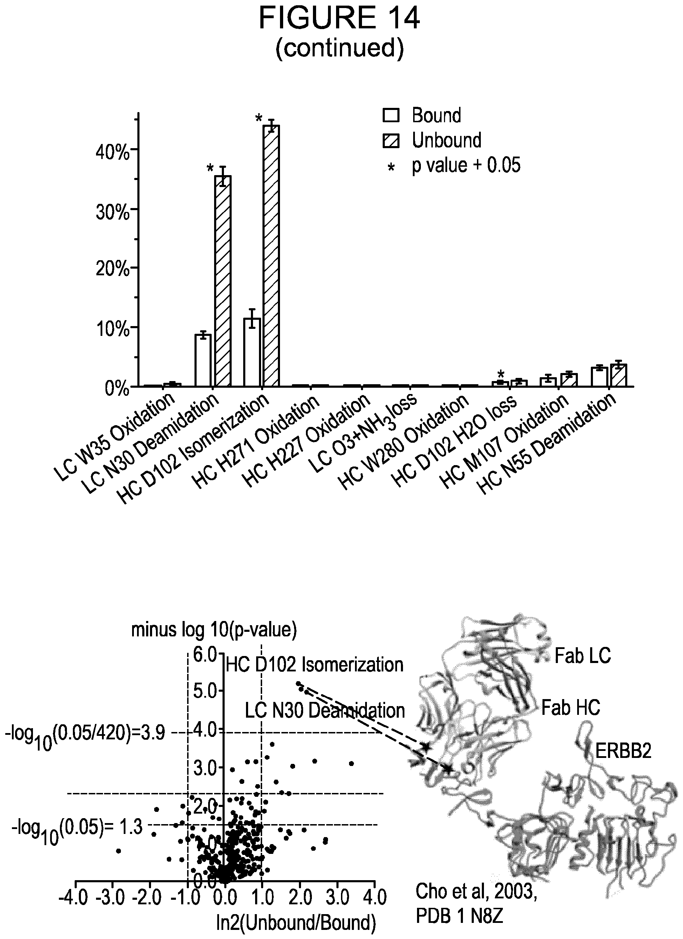

Figure 14 is schematic of the SEC binding affinity workflow according to some embodiments.

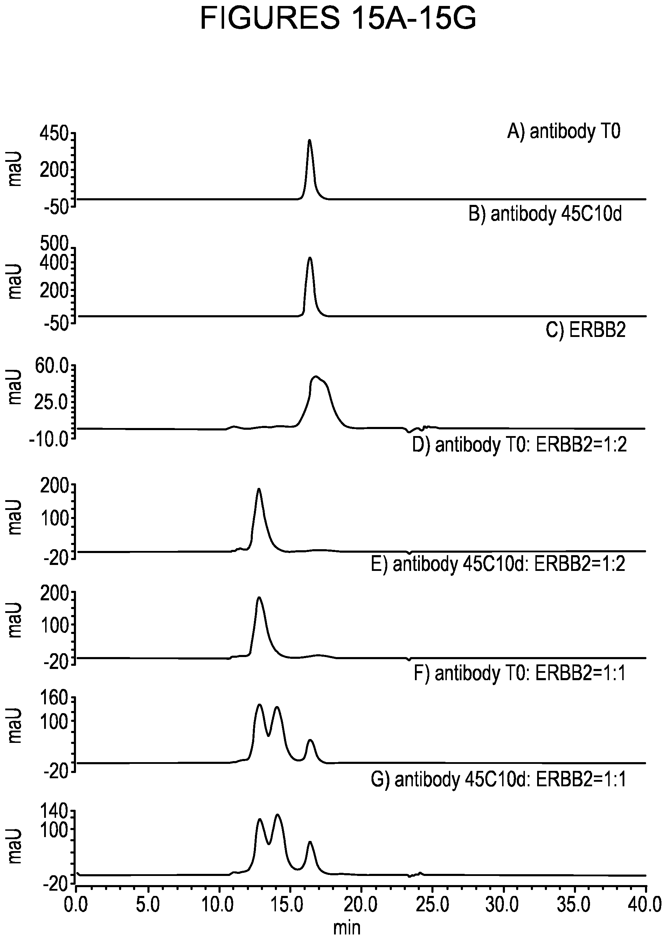

Figures 15A-15G are SEC-UV profiles (280 nm) of antibody, receptor, and their mixture at different ratios, in accordance with some embodiments. The binding ratio used for each experiment is indicated in each corresponding panel.

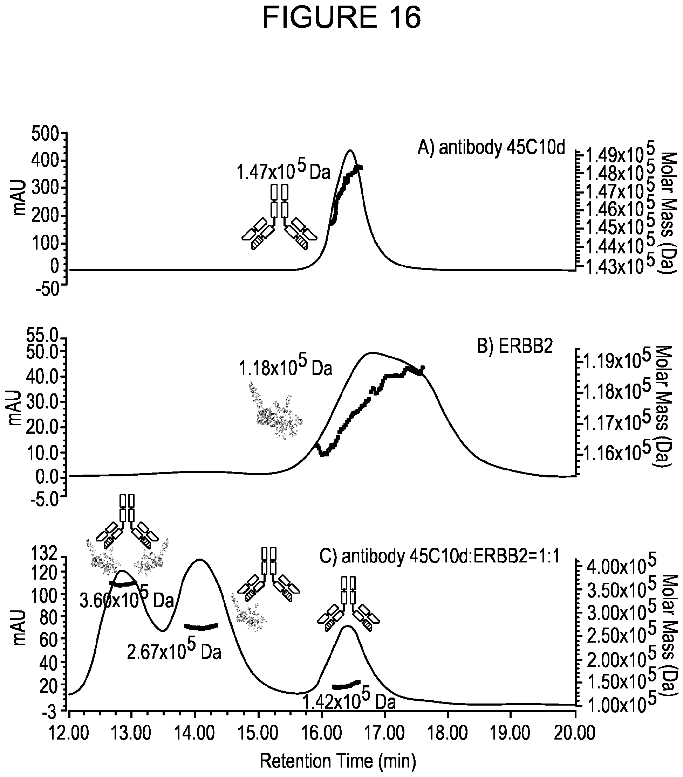

Figures 16A-16C are SEC-UV-MALS profiles of the stressed antibody (45C10d), antigen, and their mixture with a binding ratio of 1:1 in accordance with some embodiments. The MALS data are represented in dots, and the y-axis of the determined molecular mass is shown in the right.

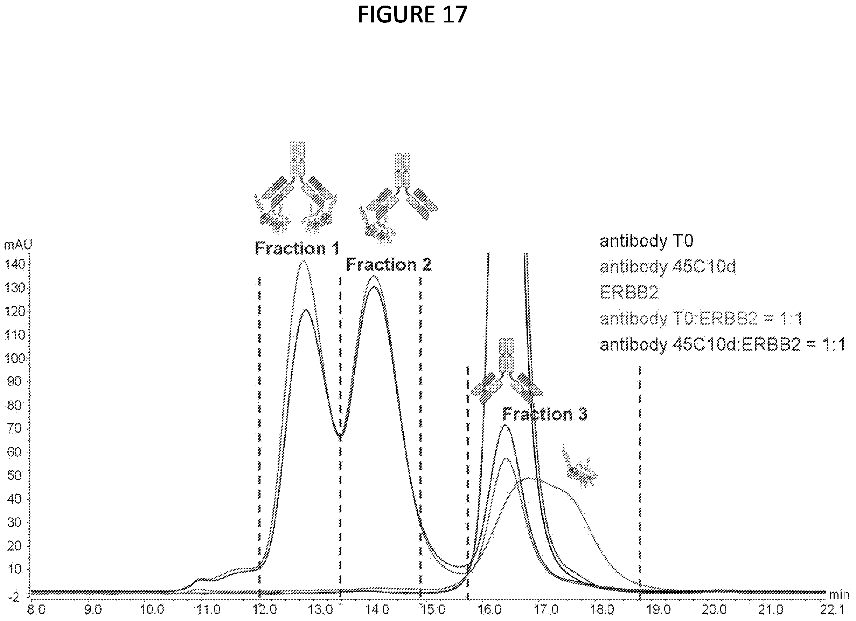

Figure 17 is a SEC-UV (280 nm) profile and collected fractions of the 45C10d antibody /antigen mixture with a ratio of 1:1 in accordance with some embodiments. The UV profile is shown in blue lines, and blue dashed lines delineate the regions for fraction collection of antibody 45C10d/antigen mixture. The SEC-UV profiles of antibody T0, antibody 45C10d, HER2, and antibody T0/antigen mixture are shown in black, red, magenta, and green for comparison.

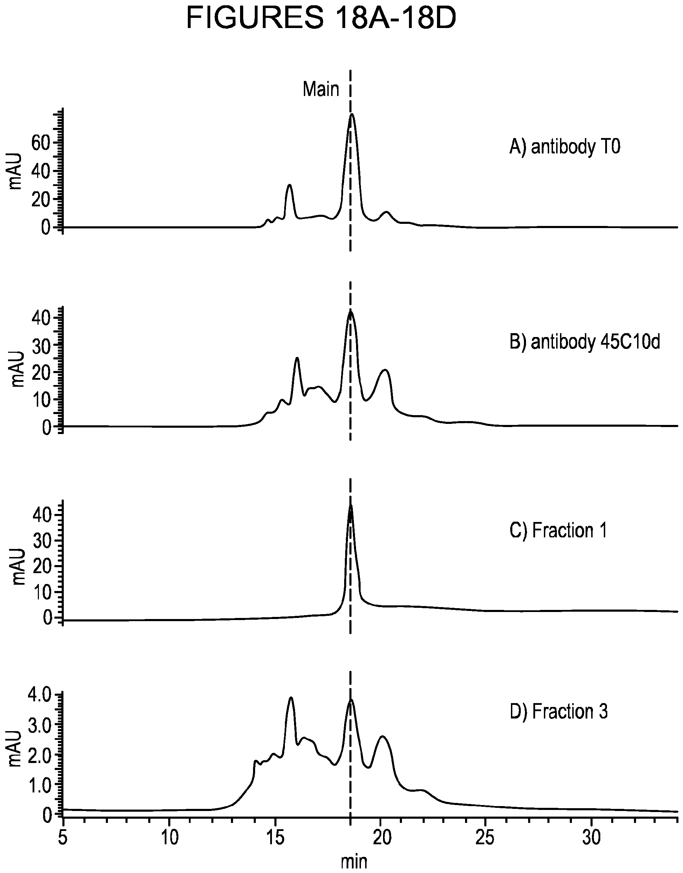

Figures 18A-18D are CEX-UV (280 nm) profiles of antibody T0, antibody 45C10d, the collected fraction 1, and the collected fraction 3 in accordance with some embodiments. Fraction 1 and 3 are collected based on the SEC-UV profile of antibody 45C10d/antigen mixture with a ratio of 1:1 shown in Figure 17.

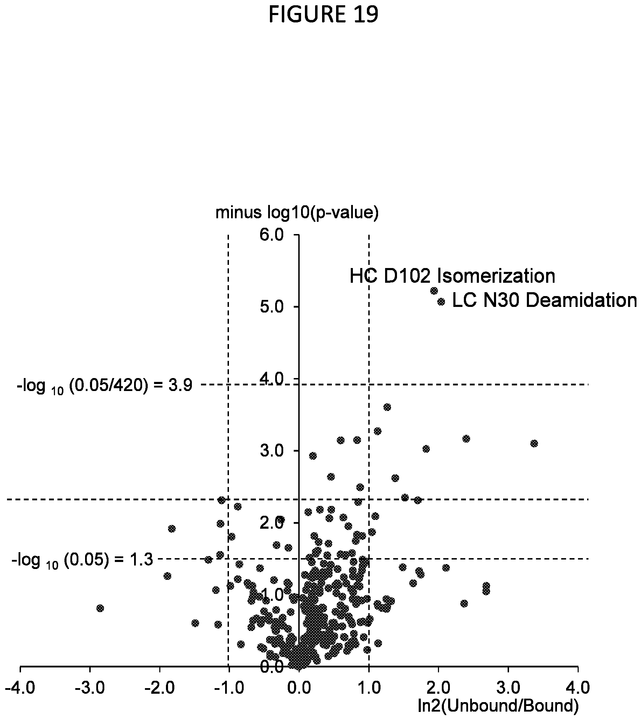

Figure 19 is a volcano plot for criticality assessment of attributes of antibody in accordance with some embodiments. The x-axis represents the In2 values of the ratio between unbound and bound, and the y-axis represents the log10 value of p-value. For reference, the vertical dashed lines corresponds to a ratio of 1.5, and three horizontal dashed lines represent three p-values: 0.05, 0.005, and 0.05/420

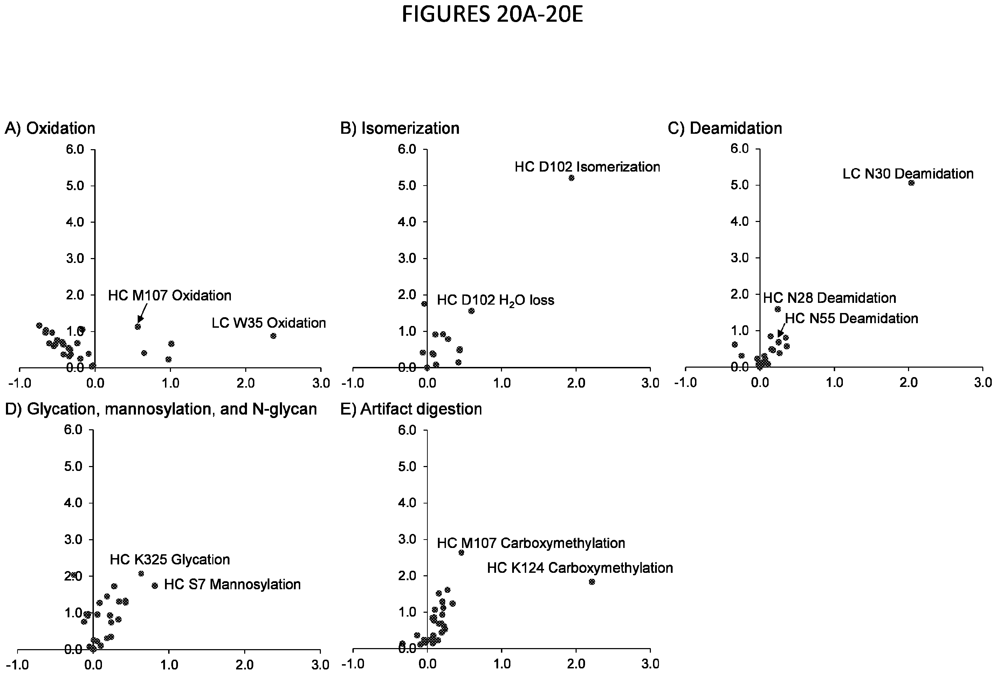

Figures 20A-20E are volcano plots for selective attributes of antibody by SEC of antibody and its target in accordance with some embodiments. These attributes include oxidation (A), isomerization (B), deamidation (C), glycation, mannosylation, and N-glycan (D), and artifact digestion (E).

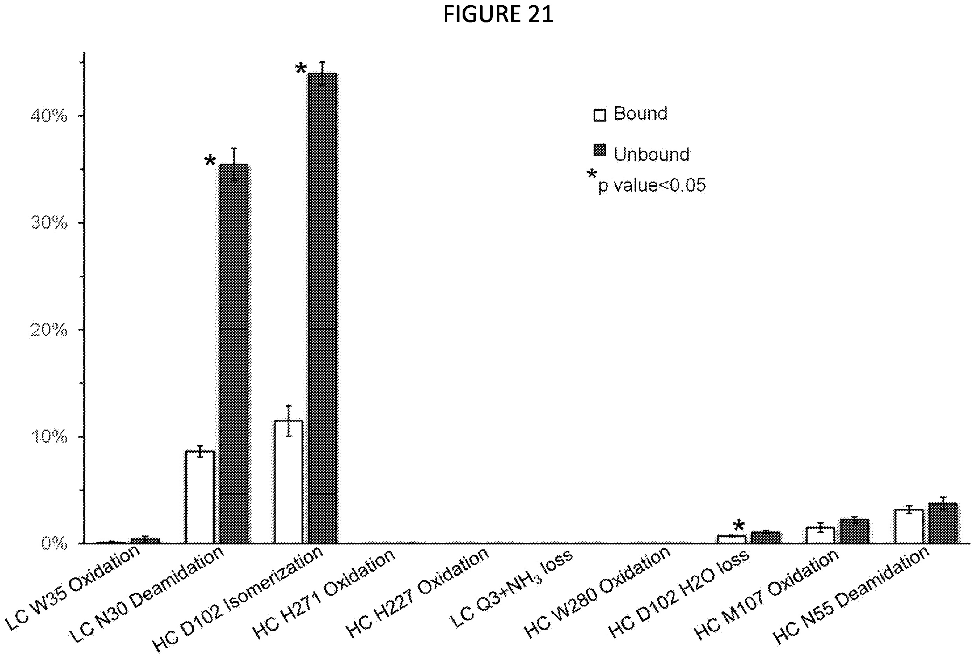

Figure 21 shows the relative abundance plots of selected attributes of antibody in bound (white) and unbound (blue) species according to some embodiments. The modifications with a p-value that is smaller than 0.05 are labeled with asterisk signs.

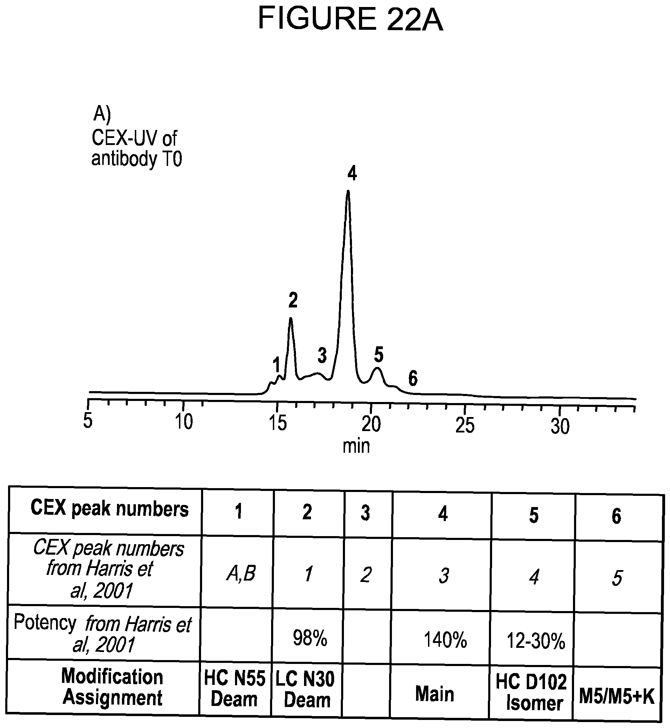

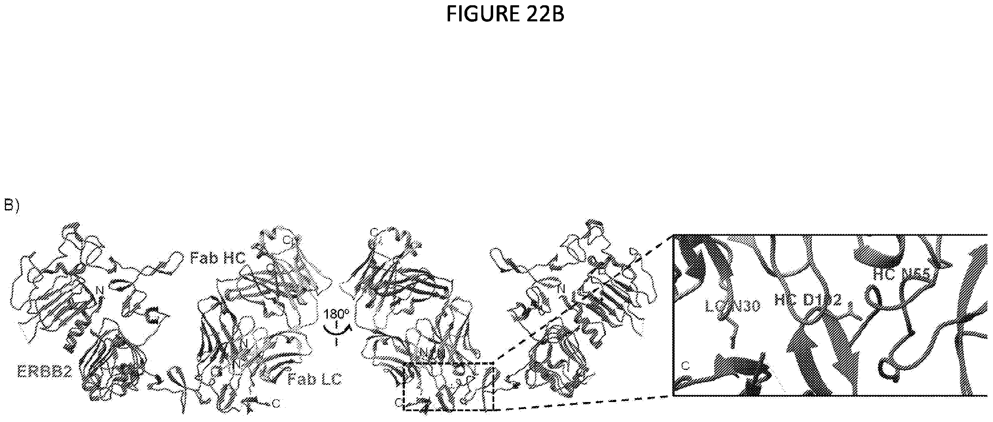

Figure 22A) CEX-UV profile of antibody and characterization of each proteoform observed in the CEX experiments according to some embodiments. Potency measurement of CEX peaks are also shown in the table on the right. Figure 22B) crystal structure of antibody-HER2 and the closer view of the binding region with critical attributes shown in the right box.

### DETAILED DESCRIPTION

The present disclosure provides methods of identifying structures of a therapeutic protein or target that affect an interaction between the therapeutic protein and the target. In exemplary embodiments, the method comprises (a) applying a stress to a first sample comprising therapeutic proteins or targets; (b) contacting the first sample with a second sample comprising targets or therapeutic proteins (e.g., the first sample can comprise the therapeutic protein and the second sample can comprises the target, or the first sample can comprise the target and the second sample can comprise the therapeutic proteins) to form a mixture comprising (i) therapeutic protein-target complexes, (ii) unbound therapeutic proteins, and (iii) unbound targets; (c) separating the mixture into at least two fractions, wherein an unbound fraction comprises unbound therapeutic proteins or unbound targets and a bound fraction comprises therapeutic protein-target complexes; and (d) for each of the unbound fraction and bound fraction, identifying and quantifying the abundance of a structure present on a species of the therapeutic protein or target. For example, the abundance of each structure present on the species of the therapeutic protein can be identified and quantified. In exemplary aspects, when the abundance of a structure in the unbound fraction is greater than the abundance of the structure in the bound fraction, the structure negatively affects the interaction between the therapeutic protein and the target. In exemplary instances, when the abundance of an structure in the unbound fraction is equal to or less than the abundance of the structure in the bound fraction, the structure does not negatively affect the interaction between the therapeutic protein and the target. Without being limited by theory, it can be inferred that if a structure negatively affects the interaction between the therapeutic protein and the target that the structure may be at or near a location that is involved in binding between the therapeutic protein and the target, and/or that allosterically affects binding between the therapeutic protein and the target. By way of example, in methods described herein, a therapeutic protein can comprise an antibody, and the target can comprise an antigen for the antibody.

The present disclosure also provides methods of determining an effect of a known structure (e.g., attribute) present on a species of a therapeutic protein or target on an interaction between the therapeutic protein and the target. In exemplary embodiments, the method comprises (a) contacting a first sample comprising a therapeutic protein or target with a known structure (e.g., attribute) with a second sample comprising targets or therapeutic proteins to form a mixture comprising (i) therapeutic protein- target complexes, (ii) unbound therapeutic proteins, and (iii) unbound targets; (b) separating the mixture into at least two fractions, wherein an unbound fraction comprises unbound therapeutic proteins or targets and a bound fraction comprises therapeutic protein-target complexes; and (c) for each of the unbound fraction and bound fraction, quantifying the abundance of the known structure (e.g., attribute). In exemplary aspects, when the abundance of the known structure (e.g., attribute) in the unbound fraction is greater than the abundance of the known structure in the bound fraction, the known structure (e.g., attribute) has a negative effect on the interaction between the therapeutic protein and the target. In exemplary instances, when the abundance of the known structure (e.g., attribute) in the unbound fraction is equal to or less than the abundance of the known structure in the bound fraction, the known structure does not have a negative effect on the interaction between the therapeutic protein and the target.

In various aspects, the method is a method of identifying attributes of a therapeutic protein that affect an interaction between the therapeutic protein and a target. In exemplary embodiments, the method comprises (a) applying a stress to a first sample comprising therapeutic proteins; (b) contacting the first sample with a second sample comprising targets to form a mixture comprising (i) therapeutic protein-target complexes, (ii) unbound therapeutic proteins, and (iii) unbound targets; (c) separating the mixture into at least two fractions, wherein an unbound fraction comprises unbound therapeutic proteins and a bound fraction comprises therapeutic protein-target complexes; and (d) for each of the unbound fraction and bound fraction, identifying and quantifying the abundance of an attribute present on a species of the therapeutic protein. For example, each attribute can be identified and quantified on the species of the therapeutic protein. In exemplary aspects, when the abundance of an attribute in the unbound fraction is greater than the abundance of the attribute in the bound fraction, the attribute negatively affects the interaction between the therapeutic protein and the target. In exemplary instances, when the abundance of an attribute in the unbound fraction is equal to or less than the abundance of the attribute in the bound fraction, the attribute does not negatively affect the interaction between the therapeutic protein and the target

In various instances, the method is a method of determining an effect of a known attribute present on a species of a therapeutic protein on an interaction between the therapeutic protein and a target. In exemplary embodiments, the method comprises (a) contacting a first sample comprising a therapeutic protein with a known attribute with a second sample comprising targets to form a mixture comprising (i) therapeutic protein- target complexes, (ii) unbound therapeutic proteins, and (iii) unbound targets; (b) separating the mixture into at least two fractions, wherein an unbound fraction comprises unbound therapeutic proteins and a bound fraction comprises therapeutic protein-target complexes; and (c) for each of the unbound fraction and bound fraction, quantifying the abundance of the known attribute. In exemplary aspects, when the abundance of the known attribute in the unbound fraction is greater than the abundance of the known attribute in the bound fraction, the known attribute has a negative effect on the interaction between the therapeutic protein and the target. In exemplary instances, when the abundance of the known attribute in the unbound fraction is equal to or less than the abundance of the known attribute in the bound fraction, the known attribute does not have a negative effect on the interaction between the therapeutic protein and the target.

As used herein, the term "therapeutic protein" refers to any molecule, which may be naturally-occurring or engineered or synthetic, comprising at least one polypeptide chain, which, when administered to a subject, is intended for achieving a therapeutic effect for treatment of a disease or medical condition. In various aspects, a therapeutic protein interacts with a target to form an interaction, e.g., a binding interaction. In various instances, the formation of the interaction between the therapeutic protein and target is important to the therapeutic protein's mechanism of action. In various aspects, the interaction is relevant to the therapeutic efficacy of the therapeutic protein in the context of the intended treatment. In exemplary instances, the therapeutic protein interacts, e.g., binds, to a target with a requisite affinity and specificity. The affinity is a measure of the strength of the interaction (e.g., binding interaction) between the therapeutic protein and target. In exemplary aspects, the therapeutic protein has high-affinity for the target and thus will bind a greater amount of the target in a shorter period of time than low-affinity therapeutic proteins. In exemplary aspects, the affinity of the therapeutic protein for the target is expressed in terms of an equilibrium association constant, K_{A}. In exemplary aspects, the therapeutic protein has an equilibrium association constant, K_{A}, for the target, which is at least 10⁵ mol⁻¹, at least 10⁶ mol⁻¹, at least 10⁷ mol⁻¹, at least 10⁸ mol⁻¹, at least 10⁹ mol⁻¹, or at least 10¹⁰ mol⁻¹ or at least 10¹⁰ mol⁻¹ least 10¹⁰ mol⁻¹. As understood by the artisan of ordinary skill, K_{A} can be influenced by factors including pH, temperature and buffer composition.

In exemplary embodiments, the strength of interaction between the therapeutic protein and target may be described in terms of its sensitivity. K_{D} is the equilibrium dissociation constant, a ratio of k_{off}/kₒₙ, between the therapeutic protein and target. K_{D} and K_{A} are inversely related. The K_{D} value relates to the concentration of the therapeutic protein (the amount of therapeutic protein needed for a particular experiment) and so the lower the K_{D} value (lower concentration) the higher the affinity of the therapeutic protein. In exemplary aspects, the binding strength of the therapeutic protein to the target may be described in terms of K_{D}. In exemplary aspects, the K_{D} of the therapeutic protein for the target is about 10⁻¹, about 10⁻², about 10⁻³, about 10⁻⁴, about 10⁻⁵, about 10⁻⁶, or less. In exemplary aspects, the K_{D} of the therapeutic protein for the target is micromolar, nanomolar, picomolar or femtomolar. In exemplary aspects, the K_{D} of the therapeutic protein is within a range of about 10⁻⁴ to 10⁻⁶ or 10⁻⁷ to 10⁻⁹ or 10⁻¹⁰ to 10⁻¹² or 10⁻¹³ to 10⁻¹⁵. In exemplary aspects, the K_{D} of the therapeutic protein is within a range of about 10⁻¹² M to about 10⁻⁶ M, about 10⁻¹¹ M to about 10⁻⁶ M, about 10⁻¹⁰ M to about 10⁻⁶ M, about 10⁻⁹ M to about 10⁻⁶ M, about 10⁻⁸ M to about 10⁻⁶ M, about 10⁻⁷ M to about 10⁻⁶ M, about 10⁻¹² M to about 10⁻⁷ M, about 10⁻¹² M to about 10⁻⁸ M, about 10⁻¹² M to about 10⁻⁹ M, about 10⁻¹² M to about 10⁻¹⁰ M, or about 10⁻¹² M to about 10⁻¹¹ M. In exemplary aspects, the K_{D} of the therapeutic proteins is within a range of about 10⁻¹¹ M to about 10⁻⁹ M, and in some such aspects, the interaction is considered as a strong interaction (e.g., strong binding interaction). In some aspects, the K_{D} of the therapeutic protein for its target is about 10⁻⁸ to about 10⁻⁶ M and, in some such instances, the interaction is considered a weak interaction (e.g., weak binding interaction).

In various aspects, the therapeutic protein is placed in a condition that leads to a change in its structure, for example, a change in the structure of an amino acid of the therapeutic protein, leading to the formation of a species of the therapeutic protein. In exemplary aspects, the changed structure of an amino acid is referred to as an "attribute" and may be characterized in terms of its chemical identity or attribute type and location within the amino acid sequence of the therapeutic protein, e.g., the position of the amino acid on which the attribute is present. For example, asparagine and glutamine residues are susceptible to deamidation. A deamidated asparagine at position 10 of a therapeutic protein amino acid sequence is an example of an attribute. A list of exemplary attribute types for particular amino acids is provided in TABLE A. As such, a "structure" as used herein can comprise, consist essentially of, or consisting of an attribute type listed in Table A, or a combination of two or more attribute types listed in Table A. It will be understood that attributes are examples of structures, and unless stated otherwise, wherever a "structure" is mentioned herein, an attribute is contemplated as an example of the structure.

**TABLE A**

| **Exemplary Attribute Type** | **Amino acid residue** |
|---|---|
| deamidation | Asn, Gln |
| deamination | Glu, Ser, Gly |
| glycation, hydroxylysine | Lys |
| glycosylation | Asn |
| cyclization | N-terminal Gln, N-terminal Glu |
| oxidation | Met, Trp, His |
| isomerization | Asp |
| fragmentation/clipping | Asp/Pro |

As a therapeutic protein comprises multiple amino acids, a therapeutic protein may have more than one attribute (e.g., more than one amino acid having a changed structure) and may be described in terms of its attribute profile. As used herein, the term "attribute profile" refers to a listing of a therapeutic protein's attributes. In various instances, the attribute profile provides the chemical identity or attribute type, e.g., deamidation, optionally, relative to the native structure of the therapeutic protein. In various instances, the attribute profile provides the location of the attribute, e.g., the position of the amino acid on which the attribute is present. An exemplary attribute profile of a therapeutic protein (e.g., a therapeutic protein attribute profile) is provided in Figures 3 and 4. An attribute profile in some aspects, provides a description of all attributes present on the therapeutic protein. In other aspects, an attribute profile provides a description of a subset of attributes present on the therapeutic protein. For example, an attribute profile may provide only those attributes that are present in a particular portion of the therapeutic protein, e.g., the extracellular domain, the variable region, the CDR. A species of a therapeutic protein is characterized by the attribute(s) present on the therapeutic protein. A species of a therapeutic protein may differ from another species of the same therapeutic protein by having a different attribute profile. When two therapeutic proteins have differing attribute profiles, the therapeutic proteins represent two different species of the therapeutic protein. When two therapeutic proteins have identical attribute profiles, the therapeutic proteins are considered as the same species of the therapeutic protein.

In various instances, the therapeutic protein is placed in a condition that leads to a change in its structure, e.g., formation of one or more attributes, and the change in structure alters the affinity of the therapeutic protein for its target. In various aspects, the therapeutic protein is placed in a condition that leads to a change in its structure, e.g., formation of one or more attributes, and the change in structure reduces the affinity of the therapeutic protein for its target. The reduced affinity in some aspects leads to a partial or total loss of the ability of the therapeutic protein to interact with (e.g., bind to) a target. In various instances, the partial or total loss of the ability of the therapeutic protein to interact with (e.g., bind to) a target ultimately reduces the therapeutic protein's efficacy. In alternative instances, the therapeutic protein is placed in a condition that leads to a change in its structure, e.g., formation of one or more attributes, and the change in structure does not alter the affinity of the therapeutic protein for its target. In various aspects, the change in structure does not reduce the therapeutic protein for its target. Without being bound to any particular theory, the methods of the present disclosure advantageously distinguish with precision and accuracy those attributes of a therapeutic protein that affect an interaction between the therapeutic protein and the target from attributes that do not affect the interaction.

In various aspects, the therapeutic protein is placed in a condition that leads to a change in its structure, for example, a change in the structure of an amino acid of the therapeutic protein, leading to the formation of one or more attributes, thereby creating a species of the therapeutic protein. In various aspects, the change in the therapeutic protein's structure may be caused by a post-translational modification involving one or more intracellular enzymes, and thus, the change in the therapeutic protein's structure may be caused by an enzymatic modification. Enzymatic modifications are known in the art and include for example, lipidation (e.g., palmitoylation, myristoylation), phosphorylation, glycosylation, ubiquitination, nitrosylation, methylation, acetylation, and proteolysis. In various aspects, an amino acid sequence of a therapeutic protein with the post-translational modification is considered to be the same as the unmodified therapeutic protein, though one or more amino acid residues are changed by way of comprising a moiety, e.g., lipid, sugar, peptide, phosphate group, ubiquitin tag, methyl, acetyl group. In various instances, the change in the therapeutic protein's structure may be caused by a change in the protein's environment. For example, the protein's environment may change in pH, salinity, osmolality, pressure, temperature, exposure to light, UV light, or other, exposure to air or oxygen, agitation/shaking, exposure to chemicals or materials (e.g., metals, plastics), or long-term storage), and such environmental change leads to a change in the structure of one or more amino acids. The change in the environment may be a change in culture media, for example, or a change in formulation components, a change in storage conditions, and the like. The change in environment in some aspects occurs during any one or more of the process steps that lead up to being administered to a patient, e.g., one or more of protein production (e.g., recombinant production), harvest, purification, formulation, filling, packaging, storage, delivery, and final preparation immediately prior to administration to the patient. Exemplary modifications that may occur during manufacture include, e.g., the removal of a residue from a polypeptide of the therapeutic protein, and/or cleavage mediated by proteases. Exemplary changes that may occur post-production but prior to administration (e.g., during packaging and/or filling, storage and/or shipping/transport) of the therapeutic protein include, e.g., oxidation, reduction, deamidation, deamination, aggregation, denaturation, precipitation, hydrolysis, aspartate isomerization, N-terminal and C-terminal modification. Accordingly, in various aspects, the change in the therapeutic protein's structure may be caused by a non-enzymatic modification (e.g., chemical modification) not involving any intracellular enzymes, and thus, the change in the therapeutic protein's structure may be caused by a non-enzymatic modification (e.g., chemical modification). In various embodiments, the modification occurs post-administration of the therapeutic protein and occurs *in vivo* (relative to the subject to whom the therapeutic protein was administered).

In various aspects, the target is analyzed by the methods of the present disclosure. Thus, in various aspects, the target is placed in a condition that leads to a change in its structure, for example, a change in the structure of an amino acid of the target, leading to the formation of a species of the target. In exemplary aspects, the changed structure of an amino acid is referred to as an "attribute" and may be characterized in terms of its chemical identity or attribute type and location within the amino acid sequence of the target, e.g., the position of the amino acid on which the attribute is present. For example, asparagine and glutamine residues are susceptible to deamidation. A deamidated asparagine at position 10 of a target amino acid sequence is an example of an attribute. A list of exemplary attribute types for particular amino acids is provided in TABLE A. Accordingly, for methods as described herein, an attribute can comprise, consist essentially or, or consist of an attribute type shown in TABLE A, or a combination of two or more attribute types shown in TABLE A.

As a target comprises multiple amino acids, a target may have more than one attribute (e.g., more than one amino acid having a changed structure) and may be described in terms of its attribute profile. As used herein, the term "attribute profile" may refer to a listing of a target's attributes. In various instances, the attribute profile provides the chemical identity or attribute type, e.g., deamidation, optionally, relative to the native structure of the target. In various instances, the attribute profile provides the location of the attribute, e.g., the position of the amino acid on which the attribute is present. An attribute profile in some aspects, provides a description of all attributes present on the target. In other aspects, an attribute profile provides a description of a subset of attributes present on the target. For example, an attribute profile may provide only those attributes that are present in a particular portion of the target, e.g., the extracellular domain. A species of a target is characterized by the attribute(s) present on the target. A species of a target may differ from another species of the same target by having a different attribute profile. When two targets have differing attribute profiles, the targets represent two different species of the target. When two targets have identical attribute profiles, the targets are considered as the same species of the target.

In various instances, the target is placed in a condition that leads to a change in its structure, e.g., formation of one or more attributes, and the change in structure alters the affinity between a therapeutic protein and the target. In various aspects, the target is placed in a condition that leads to a change in its structure, e.g., formation of one or more attributes, and the change in structure reduces the affinity of the target for its target. The reduced affinity in some aspects leads to a partial or total loss of the ability of the target to interact with (e.g., bind to) a therapeutic protein. In various instances, the partial or total loss of the ability of the target to interact with (e.g., bind to) a therapeutic protein ultimately reduces the target's efficacy. In alternative instances, the target is placed in a condition that leads to a change in its structure, e.g., formation of one or more attributes, and the change in structure does not alter the affinity between a therapeutic protein and the target. In various aspects, the change in structure does not reduce the affinity between the therapeutic protein and the target. Without being bound to any particular theory, the methods of the present disclosure advantageously distinguish with precision and accuracy those attributes of a target that affect an interaction between the target and the therapeutic protein from attributes that do not affect the interaction.

In various aspects, the target is placed in a condition that leads to a change in its structure, for example, a change in the structure of an amino acid of the target, leading to the formation of one or more attributes, thereby creating a species of the target. In various aspects, the change in the target's structure may be caused by a post-translational modification involving one or more intracellular enzymes, and thus, the change in the target's structure may be caused by an enzymatic modification. Enzymatic modifications are known in the art and include for example, lipidation (e.g., palmitoylation, myristoylation), phosphorylation, glycosylation, ubiquitination, nitrosylation, methylation, acetylation, and proteolysis. In various aspects, an amino acid sequence of a target with the post-translational modification is considered to be the same as the unmodified target, though one or more amino acid residues are changed by way of comprising a moiety, e.g., lipid, sugar, peptide, phosphate group, ubiquitin tag, methyl, acetyl group. In various instances, the change in the target's structure may be caused by a change in the protein's environment. For example, the protein's environment may change in pH, salinity, osmolality, pressure, temperature, exposure to light, UV light, or other, exposure to air or oxygen, agitation/shaking, exposure to chemicals or materials (e.g., metals, plastics), or long-term storage), and such environmental change leads to a change in the structure of one or more amino acids. The change in the environment may be a change in culture media, for example, or a change in formulation components, a change in storage conditions, and the like. The change in environment in some aspects occurs during any one or more of the process steps that lead up to being administered to a patient, e.g., one or more of protein production (e.g., recombinant production), harvest, purification, formulation, filling, packaging, storage, delivery, and final preparation immediately prior to administration to the patient. Exemplary modifications that may occur during manufacture include, e.g., the removal of a residue from a polypeptide of the target, and/or cleavage mediated by proteases. Exemplary changes that may occur post-production but prior to administration (e.g., during packaging and/or filling, storage and/or shipping/transport) of the target include, e.g., oxidation, reduction, deamidation, deamination, aggregation, denaturation, precipitation, hydrolysis, aspartate isomerization, N-terminal and C-terminal modification. Accordingly, in various aspects, the change in the target's structure may be caused by a non-enzymatic modification (e.g., chemical modification) not involving any intracellular enzymes, and thus, the change in the target's structure may be caused by a non-enzymatic modification (e.g., chemical modification).

### Samples and Stresses

With regard to the presently disclosed methods of identifying attributes of a therapeutic protein or target that affect an interaction between the therapeutic protein and the target, the method in exemplary embodiments comprises applying a stress to a first sample comprising therapeutic proteins or targets and contacting the first sample with a second sample comprising targets or the therapeutic proteins to form a mixture comprising (i) therapeutic protein-target complexes, (ii) unbound therapeutic proteins, and (iii) unbound targets.

In various aspects, the first sample comprises a population of species of the therapeutic protein. In various instances, the population is a homogenous population of the therapeutic protein, optionally, each of the therapeutic proteins present in the first sample are the same species. In various instances, the population is a heterogeneous population comprising at least two different species of the therapeutic protein. In various aspects, the heterogeneous population comprises at least 3, at least 4, at least 5, at least 6 or more different species of the therapeutic protein. Optionally, the heterogeneous population comprises more than 7, more than 8, more than 9, more than 10, more than 20, more than 30, more than 40, more than 50 different species of the therapeutic protein. Each species of the population in some aspects has a unique attribute profile. In exemplary instances, the species of the therapeutic protein are the only proteins present in the first sample. In some aspects, the first sample comprises (i) the population of therapeutic proteins and (ii) a pharmaceutically-acceptable carrier, diluent, excipient, or a combination thereof. In various aspects, the first sample comprises acceptable formulation components which preferably are nontoxic to patients. The first sample optionally comprises agents for modifying, maintaining or preserving, for example, the pH, osmolarity, viscosity, clarity, color, isotonicity, odor, sterility, stability, rate of dissolution or release, adsorption or penetration of the therapeutic protein in the first sample. In general, excipients can be classified on the basis of the mechanisms by which they stabilize proteins against various chemical and physical stresses. Some excipients alleviate the effects of a specific stress or regulate a particular susceptibility of a specific polypeptide. Other excipients more generally affect the physical and covalent stabilities of proteins. Common excipients of liquid and lyophilized therapeutic protein formulations are shown in TABLE B (see also (Kamerzell, Esfandiary, Joshi, Middaugh, & Volkin, Adv Drug Deliv Rev 63(13): 1118-1159 (2011))).

**TABLE B**

| Examples of excipient components for polypeptides formulations | | |
|---|---|---|
| **Component** | **Function** | **Examples** |
| Buffers | Maintaining solution pH | Citrate |
| | Mediating buffer-ion specific interactions with polypeptides | Succinate |
| | | Acetate |
| | | glutamate |
| | | Aspartate |
| | | histidine |
| | | Phosphate |
| | | Tris |
| | | Glycine |
| Sugars and carbohydrates | Stabilizing polypeptides | Sucrose |
| | Tonicifying agents | Trehalose |
| | Acting as carriers for inhaled drugs (e.g., lactose) | Sorbitol |
| | | Mannitol |
| | Providing dextrose solutions during IV | Glucose |
| | administration | Lactose |
| | | Cyclodextrin derivatives |
| Stabilizers and bulking agents | Enhancing product elegance and preventing blowout | Mannitol |
| | | Glycine |
| Osmolytes | Providing structural strength to a lyo cake Stabilizing against environmental stress (temperature, dehydration) | Sucrose |
| | | Trehalose |
| | | Sorbitol |
| | | Glycine |
| | | proline |
| | | glutamate |
| | | Glycerol |
| | | Urea |
| Amino acids | Mediating specific interactions with polypeptides | histidine |
| | | arginine |
| | Providing antioxidant activity (e.g., His, Met) | Glycine |
| | | proline |
| | Buffering, tonicifying | lysine |
| | | Methionine |
| | | Aa mixtures (e.g., Glu/Arg) |
| Polypeptides and polymers | Acting as competitive inhibitors of polypeptide adsorption | HSA |
| | | PVA |
| | Providing bulking agents for lyophilization | PVP |
| | Acting as drug delivery vehicles | PLGA |
| | | PEG |
| | | Gelatin |
| | | Dextran |
| | | Hydroxyethyl starch |
| | | HEC |
| | | CMC |
| Anti-oxidants | Preventing oxidative polypeptides damage | Reducing agents |
| | | Oxygen scavengers |
| | Metal ion binders (if a metal is included as a co-factor or is required for protease activity) | Free radical scavengers |
| | | Chelating agents (e.g., EDTA, EGTA, DTPA) |
| | Free radical scavengers | Ethanol |
| Metal ions | Polypeptides co-factors | Magnesium |
| | Coordination complexes (suspensions) | Zinc |
| Specific ligands | Stabilizers of native conformation against stress-induced unfolding | Metals |
| | | Ligands |
| | Providing conformation flexibility | Amino acids |
| | | Polyanions |
| Surfactants | Acting as competitive inhibitors of polypeptides adsorption | Polysorbate 20 |
| | | Polysorbate 80 |
| | Acting as competitive inhibitor of polypeptides surface denaturation | Poloxamer 188 |
| | | Anionic surfactants (e.g., |
| | Providing liposomes as drug delivery vehicles | sulfonates and |
| | | sulfosuccinates) |
| | Inhibiting aggregation during lyophilization | Cationic surfactants |
| | | Zwitterionic surfactants |
| | Acting as reducer of reconstitution times of lyophilized products | |
| Salts | Tonicifying agents | NaC! |
| | Stabilizing or destabilizing agents for polypeptidess, especially anions | KCl |
| | | NaSO₄ |
| Preservatives | Protecting against microbial growth | Benzyl alcohol |
| | | M-cresol |
| | | Phenol |

Other excipients are known in the art (e.g., see (Powell, Nguyen, & Baloian, PDA J Pharm Sci Technol 52(5): 238-311 (1998)).

In various aspects, the first sample comprises the population of therapeutic proteins and a buffer. In exemplary aspects, the buffer is a buffer listed in TABLE C.

**TABLE C**

| Commonly used buffering agents and their pKa values | | |
|---|---|---|
| **Buffer** | **pKₐ** | **Example drug product** |
| Acetate | 4.8 | Neupogen^{®}, Neulasta^{®} |
| Succinate | pKₐ₁ = 4.8, pKₐ₂ = 5.5 | Actimmune^{®} |
| Citrate | pKₐ₁ = 3.1, pKₐ₂ = 4.8, pKₐ₃ = 6.4 | Humira^{®} |
| histidine (imidazole) | 6.0 | Xolair^{®} |
| phosphate | pKₐ₁ = 2.15, pKₐ₂ = 7.2, pKₐ₃ = 12.3 | Enbrel^{®} (liquid formulation) |
| Tris | 8.1 | Leukine |

In various instances, the first sample comprises one or more species of the therapeutic protein and serum, or a serum fraction. The term "serum" as used herein refers to the fraction of blood remaining after clotting proteins and blood cells have been removed. A "serum fraction" refers to a portion of serum remaining after one or more components are removed from serum. In some aspects, the serum fraction is a depleted fraction of serum. A "depleted fraction of serum" or "depleted fraction" as used herein means a fraction of serum from which one or more components have been removed. The term "whole serum" is serum from which no components have been removed. In exemplary aspects, the first sample comprises whole serum. In exemplary aspects, the whole serum is human serum, bovine serum (including fetal bovine serum), rabbit serum, mouse serum, rat serum, cynomolgus monkey serum, horse serum, or pig serum. In preferred embodiments, the whole serum is human serum. In exemplary aspects, the depleted fraction of serum is an IgG-depleted serum fraction or a molecular weight range-depleted serum ("depleted fraction serum" or "depleted fraction of serum"). In exemplary aspects, an IgG-depleted serum fraction is one obtained by removing IgG from serum by using Protein A, such as in Protein A affinity chromatography. In exemplary aspects, a depleted fraction of serum is a fraction depleted of molecules having a pre-selected molecular weight range. In exemplary instances, the pre-selected molecular weight range is about 30 kDa to about 300 kDa or higher. In various aspects, the depleted fraction is obtained by size-based filtration or centrifugation or ultra-filtration methods (see, e.g., Kornilov et al.., J Extracell Vesicles 7(1): 1422674 (2018). In various aspects, the depleted serum is obtained through commercial vendors, e.g., Thermo Fisher Scientific (Waltham, MA), CalBiochem^{®} (Millipore Sigma, Burlington, MA), Quidel (San Diego, CA), and Complement Technologies (Tyler, TX). In exemplary aspects, the depleted fraction is a twice-depleted fraction, optionally, a fraction twice-depleted of IgG or a fraction twice-depleted of molecules having a pre-selected molecular weight. "Twice-depleted" refers to a fraction of serum that has undergone the depletion or removal technique two times. In exemplary instances, the first sample comprising serum or a serum fraction comprises one or more serum proteins.

In various aspects, the first sample comprises a population of species of the target. In various instances, the population is a homogenous population of the target, optionally, each of the targets present in the first sample are the same species. In various instances, the population is a heterogeneous population comprising at least two different species of the target. In various aspects, the heterogeneous population comprises at least 3, at least 4, at least 5, at least 6 or more different species of the target, including ranges between any two of the listed values, for example 2-4, 2-6, or 4-6 different species of the target. Optionally, the heterogeneous population comprises more than 7, more than 8, more than 9, more than 10, more than 20, more than 30, more than 40, more than 50 different species of the target, including ranges between any two of the listed values. Each species of the population in some aspects has a unique attribute profile. In exemplary instances, the species of the target are the only proteins present in the first sample.

In exemplary embodiments, the method comprises applying a stress to the first sample. In various instances, the stress may be any condition which leads to at least one change in structure of an amino acid of the therapeutic protein or target, e.g., the stress may be any condition which leads to the formation of at least one attribute at an amino acid of the therapeutic protein or target. Optionally, the stress leads to a change in structure in more than one amino acid of the therapeutic protein or target, e.g., the stress leads to the formation or more than one attribute (e.g., at least or about 2, at least or about 3, at least or about 4, at least or about 5, at least or about 6, at least or about 7, at least or about 8, at least or about 9, at least or about 10, or more attributes). The stress in various instances leads to the formation of one or more attributes that are not present in the therapeutic protein or target prior to the application of the stress. Accordingly, in some aspects, the application of stress leads to the formation of species of the therapeutic protein or target that were not present in the first sample prior to the application of stress.

In various aspects, the stress that is applied is an (A) exposure to visible light, ultra-violet (UV) light, heat, air/oxygen, freeze/thaw cycle, shaking/agitation, chemicals and materials (e.g., metals, metal ions, chaeotropic salts, detergents, preservatives, organic solvents, plastics), molecules and cells (e.g., immune cells), or (B) change in pH (e.g., a change of greater than 1.0, 1.5, or 2.0), pressure, temperature, osmolality, salinity, or (C) long-term storage. The change in temperature in some aspects, is a change of at least or about 1 degree C, at least or about 2 degrees C, at least or about 3 degrees C, at least or about 4 degrees C, at least or about 5 degrees C, or more. The methods of the present disclosure are not limited to any particular types of stresses. In exemplary aspects, the stress is an exposure to elevated temperatures to, e.g., 25 degrees C, 40 degrees C, 50 degrees C optionally, in formulation. In exemplary instances, such exposure to elevated temperatures mimics an accelerated stress program. In some aspects, the stress is exposure to visible and/or ultra-violet light; oxidizing reagents (e.g., hydrogen peroxide); air/oxygen, freeze/thaw cycle, shaking, long-term storage in formulation under the intended product storage conditions; mildly acidic pH (e.g., pH of 3-4) or elevated pH (e.g., pH of 8-9) simulate exposure to some purification conditions/steps. In some aspects, the stress is a change in pH of greater than 1.0, 1.5, 2.0 or 3.0. In exemplary aspects, the stress is an exposure to ultra-violet light, heat, air, freeze/thaw cycle, shaking, long-term storage, change in pH, or change in temperature, optionally, wherein the change in pH is greater than about 1.0 or greater than about 2.0, optionally, wherein the change in temperature is greater than or about 2 degrees Celsius or greater than or about 5 degrees Celsius. In various aspects, the stress applied to the first sample leads to formation of at least one species of the therapeutic protein or target having a unique attribute profile relative to the attribute profile of the therapeutic protein or target prior the applied stress. In various aspects, the attribute forms as a result of a chemical modification, optionally, wherein the chemical modification alters the mass-to-charge ratio (m/z) of charged ions of an amino acid of the therapeutic protein or target. Optionally, the chemical modification is e.g., a glycosylation, hydroxylation, glycation, deamidation, oxidation, reduction, isomerization, aggregation, degradation, acetylation, or clipping due to hydrolysis proteolysis.

Optionally, the stress causes, about 5% to about 30%, about 10% to about 30%, about 15% to about 30%, about 20% to about 30%, about 25% to about 30%, about 5% to about 25%, about 5% to about 20%, about 5% to about 15%, or about 5% to about 10% of complexes formed between the therapeutic protein and the target to degrade or dissociate. In various aspects, the stress causes a reduced level of interactions between the therapeutic protein and its target. In some aspects, the stress causes an about 10% to about 50% (e.g., about 10% to about 45%, about 10% to about 40%, about 10% to about 35%, about 10% to about 30%, about 10% to about 25%, about 10% to about 20%, about 10% to about 15%, about 10% to about 40%, about 10% to about 35%, about 10% to about 30%, about 10% to about 25%, about 10% to about 20%, or about 10% to about 15%) reduction in interactions, relative to interactions in corresponding conditions lacking the stress. In some aspects, the stress causes an increase in the K_{D} of the therapeutic protein for its target which K_{D} is associated with weaker binding. In some aspects, the stress causes a 10% to about 50% increase (e.g., about 10% to about 45%, about 10% to about 40%, about 10% to about 35%, about 10% to about 30%, about 10% to about 25%, about 10% to about 20%, about 10% to about 15%, about 10% to about 40%, about 10% to about 35%, about 10% to about 30%, about 10% to about 25%, about 10% to about 20%, or about 10% to about 15%) in the amount of unbound therapeutic protein. Without being bound to a particular theory, the stress applied in the presently disclosed methods leads to the generation of therapeutic protein species in a quicker and more robust, reproducible manner to obtain an abundance and variety of species for enhanced detection of species which might be created during manufacturing, storage and in human circulation (intravenous space or subcutaneous space in a human subject).

With regard to the presently disclosed methods of determining an effect of a known attribute present on a species of a therapeutic protein or target on an interaction between the therapeutic protein and the target, the method in exemplary embodiments comprises contacting a first sample comprising a therapeutic protein with the known attribute or a target with a known attribute with a second sample comprising targets or therapeutic proteins (e.g., the first sample can comprise therapeutic proteins and the second sample can comprise targets, or the first sample can comprise targets and the second sample can comprise therapeutic proteins) to form a mixture comprising (i) therapeutic protein- target complexes, (ii) unbound therapeutic proteins, and (iii) unbound targets. Optionally, the therapeutic protein with a known attribute is a therapeutic protein engineered to have a particular attribute at a particular amino acid at a particular location within the amino acid sequence of the therapeutic protein. In various aspects, the known attribute was engineered at a known amino acid position of the therapeutic protein prior to being contacted with the second sample. Optionally, the therapeutic protein underwent an application of a stress known to cause the known attribute. Optionally, the target underwent an application of a stress known to cause the known attribute. The stress may be any of those described herein. In some aspects, the therapeutic protein or target has an engineered attribute (e.g., oxidation (for M, W, H), deamidation (for N, Q), isomerization (for D)) at a site suspected to be part of the binding site of the therapeutic protein at which the target binds or the binding site of the target at which the therapeutic protein binds.

In various aspects, the therapeutic protein comprises more than one known attribute. In exemplary aspects, the therapeutic protein comprise 2, 3, 4, 5, 6, 7, 8, 9, 10, or more known attributes, including ranges between any two of the listed values, for example 2-10. Optionally, the therapeutic protein comprises one or more known attributes at locations hypothesized as being a part of a paratope or binding site located within the therapeutic protein, wherein the paratope or binding site is the site at which the target interacts with or binds to the therapeutic protein. In various aspects, the therapeutic protein comprises only one known attribute, but the method of determining an effect of a known attribute present on a species of a therapeutic protein on an interaction between the therapeutic protein and a target is carried out multiple times, each time with the same therapeutic protein having a different known attribute. In various aspects, the methods of determining an effect of a known attribute present on a species of a therapeutic protein on an interaction between the therapeutic protein and a target enables the mapping of a paratope or binding site on the therapeutic protein for a target. Thus, the present disclosure additionally provides a method of mapping a paratope for an interaction between a therapeutic protein and a target, wherein the paratope is on the therapeutic protein, comprising identifying attributes of a therapeutic protein that affect an interaction between the therapeutic protein and a target or determining an effect a known attribute of a therapeutic protein has on an interaction between the therapeutic protein and a target in accordance with any of the presently disclosed methods of determining an effect of a known attribute. It is contemplated that when a structure (e.g., attribute) on a therapeutic protein negatively affects the interaction between the therapeutic protein and a target, the structure is a candidate for being part of (e.g., being on a residue of) a paratope of the therapeutic protein. In some embodiments, the method as described herein further comprises generating a paratope map of the therapeutic protein. The paratope map may comprise a graphical representation and/or a coordinate map, and optionally may be electronic.

In various aspects, the target comprises more than one known attribute. In exemplary aspects, the target comprises 2, 3, 4, 5, 6, 7, 8, 9, 10, or more known attributes, including ranges between any two of the listed values, for example 2-10. Optionally, the target comprises one or more known attributes at locations hypothesized as being a part of an epitope or binding site located within the target, wherein the epitope or binding site is the site at which the therapeutic protein interacts with or binds to the target. In various aspects, the target comprises only one known attribute, but the method of determining an effect of a known attribute present on a species of a target on an interaction between the target and a therapeutic protein is carried out multiple times, each time with the same target having a different known attribute. In various aspects, the methods of determining an effect of a known attribute present on a species of a target on an interaction between the target and a therapeutic protein enables the mapping of an epitope or binding site on the target for a therapeutic protein. Thus, the present disclosure additionally provides a method of mapping an epitope for an interaction between a therapeutic protein and a target, wherein the epitope is on the target, comprising identifying attributes of a target that affect an interaction between the therapeutic protein and a target or determining an effect a known attribute of a target has on an interaction between the therapeutic protein and a target in accordance with any of the presently disclosed methods of determining an effect of a known attribute.

With regard to the methods of the present disclosure, the first sample is contacted with a second sample comprising the binding partner of the protein present in the first sample. In various aspects, the first sample comprises therapeutic proteins and the second sample comprises targets. Optionally, the second sample comprises a population of targets. In exemplary instances, the targets are proteins or are polypeptides or peptides. In various instances, the population of targets is a homogenous population, optionally, each of the targets present in the second sample are the same (e.g., chemically identical, comprising the same amino acid sequence, when the target is a protein). In some aspects, each of the targets of the second sample has the same amino acid sequence. In various instances, the population is a heterogeneous population comprising at least two different targets. In various aspects, the heterogeneous population comprises at least 3, at least 4, at least 5, at least 6 or more different targets. Optionally, the heterogeneous population comprise more than 7, more than 8, more than 9, more than 10, more than 20, more than 30, more than 40, more than 50 different targets. In exemplary instances, the targets are the only proteins present in the second sample. In various aspects, the targets are the only proteins present in the second sample. Optionally, the second sample comprises a pharmaceutically-acceptable carrier, diluent, excipient, or a combination thereof, e.g., one or more substances in Table B and/or Table C. In some aspects, the second sample comprises the targets and serum or a serum fraction. Optionally, the second sample comprises serum or serum fraction comprises one or more serum proteins. In various instances, the first sample comprises targets and the second sample comprises therapeutic proteins. In exemplary instances, the targets are proteins or are polypeptides or peptides. In various instances, the population of therapeutic proteins is a homogenous population, optionally, each of the therapeutic proteins present in the second sample are the same (e.g., chemically identical, comprising the same amino acid sequence, when the target is a protein). In some aspects, each of the therapeutic proteins of the second sample has the same amino acid sequence. In various instances, the population is a heterogeneous population comprising at least two different targets. In various aspects, the heterogeneous population comprises at least 3, at least 4, at least 5, at least 6 or more different therapeutic proteins. Optionally, the heterogeneous population comprise more than 7, more than 8, more than 9, more than 10, more than 20, more than 30, more than 40, more than 50 different therapeutic proteins. In exemplary instances, the therapeutic proteins are the only proteins present in the second sample. In various aspects, the therapeutic proteins are the only proteins present in the second sample.

### Separation

In exemplary embodiments, the methods of the present disclosure comprise separating the mixture into at least two fractions. In some aspects, the mixture is separated into several (e.g., 5, 6, 7, 8, 9, 10, or more) fractions. In some aspects, the separation step of the presently disclosed methods preserves native folding, high-order structure and binding ability of the therapeutic protein and its target. In various aspects, the mixture is separated into an unbound fraction comprises unbound therapeutic proteins or targets and a bound fraction comprises therapeutic protein-target complexes.

Suitable methods and techniques for separating mixtures into fractions are known in the art. See, e.g., Coskun, North Clin Istanb 3(2): 156-160 (2016); Snyder et al., Practical HPLC Method Development, 2nd ed., John Wiley & Sons, Inc. 1997; Snyder et al., Introduction to Modern Liquid Chromatography, John Wiley & Sons, Inc., Hoboken, NJ, 2010; Heftmann, Chromatography: Fundamentals and applications of chromatography and related differential migration methods, 6th ed., Volume 69A, Elsevier, Amsterdam, Netherlands, 2004; Mori and Barth, Size Exclusion Chromatography, Springer-Verlag, Berlin, 1999. In some aspects, the separation is based on charge, such as, e.g., ion exchange chromatography, capillary isoelectric focusing (clEF) and/or capillary zone electrophoresis (CZE) or is based on hydrophobicity, such as, e.g., separation in reverse phase (RP; e.g., RP-HPLC) and hydrophobic interaction chromatography (HIC-HPLC). In various aspects, the separation is based on size such as, e.g., size exclusion chromatography (SEC; e.g., SE-HPLC), sodium dodecyl sulfate-polyacrylamide gel electrophoresis (SDS-PAGE), capillary electrophoresis with sodium dodecyl sulfate (CE-SDS). The methods described herein are used for detecting product oxidation of Met or Trp residues, fragmentation/clipping, isomerization of Asp, deamidation, formation of pyroglutamic acid at the N-terminus. In exemplary aspects, the mixture is separated into at least two fractions using a technique that separates components of a mixture based on size, charge, hydrophobicity, affinity for a capture molecule, or a combination thereof. In various instances, the technique is size exclusion chromatography (SEC), affinity chromatography, precipitation using beads or cells, free flow fractionation (FFF), ion exchange chromatography (IEX), hydrophobic interaction chromatography (HIC), or ultracentrifugation (UC). Optionally, the mixture is separated into at least two fractions using a technique that separates components of a mixture based on size, optionally, wherein the technique is size exclusion chromatography (SEC).

In various aspects, the mixture is separated into at least two fractions using a technique that separates components of a mixture based on affinity for a capture molecule bound to a solid support, optionally, a bead or a cell. In various instances, the mixture is separated by (i) adding the mixture to a container, e.g., a tube, comprising beads bound to the capture molecule or cells expressing at its surface the capture molecule, (ii) centrifuging the container (e.g., tube) to obtain a supernatant and a pellet, (iii) collecting the supernatant from the pellet to obtain the unbound fraction, (iv) releasing the bound fraction from the pellet with a solution, (v) centrifuging the container (e.g., tube) comprising the pellet and the solution to obtain a second supernatant comprising the bound fraction and a second pellet comprising the beads or cells, and (vi) collecting the second supernatant to obtain the bound fraction. The mixture in some aspects is separated by (i) adding the mixture to a column comprising beads bound to the capture molecule to obtain a flow-through and a bound fraction (ii) collecting the flow-through to obtain the unbound fraction, (iii) releasing the bound fraction from the beads with a solution and collecting the solution comprising the bound fraction. Suitable solid supports include, for example, beads, resin, paper, optionally, made of cellulose, silica, alumina, glass, plastic, or a combination thereof. In exemplary aspects the capture molecule bound to the solid support is a protein. The capture molecule may be identical to the target. Advantageously, the capture molecule is not limited to any particular molecule.

In exemplary aspects, the method comprises contacting the first sample with a second sample comprising targets, wherein the targets in the second sample are bound to a solid support, e.g., resin, beads, wall of a multi-well plate, etc., or the targets are expressed at the cell surface of cells in the second sample. Contacting the first sample with the second sample leads to the formation of a mixture comprising therapeutic protein-target complexes (which are in turn bound to the solid support) and unbound therapeutic proteins or targets. In exemplary aspects, the mixture is separated into at least two fractions by centrifuging the mixture (e.g., in a container, e.g., a tube) and collecting the unbound fraction upon collection of the supernatant. In various aspects, the bound fraction is collected by dissociating the therapeutic protein-target complexes from the solid support, centrifuging the solid support and the complexes and collecting the bound fraction upon collection of the supernatant. An illustration of this method is shown in Figure 6A.

In exemplary instances, the method comprises applying the first sample to a column (e.g., a chromatography column) comprising targets bound to a solid support (e.g., resin, beads). In various aspects, the first sample is applied to the column and the therapeutic proteins of the first sample bind to the targets bounds to the solid support to form therapeutic protein-target complexes (which are in turn bound to the solid support). In some aspects, the flow-through represents the unbound fraction. In various instances, the bound fraction is collected by dissociating the therapeutic protein-target complexes from the solid support and collecting the complexes. An illustration of this method is shown in Figure 6B. In exemplary instances, the method comprises applying the first sample to a column (e.g., a chromatography column) comprising therapeutic proteins bound to a solid support (e.g., resin, beads). In various aspects, the first sample is applied to the column and the targets of the first sample bind to the therapeutic proteins bounds to the solid support to form therapeutic protein-target complexes (which are in turn bound to the solid support). In some aspects, the flow-through represents the unbound fraction. In various instances, the bound fraction is collected by dissociating the therapeutic protein-target complexes from the solid support and collecting the complexes.

In exemplary aspects, the therapeutic protein comprises an Fc domain of an antibody and the target is an Fc receptor. See Figures 7A and 7B.

### Identifying and Quantifying Attributes

In exemplary embodiments of the method of identifying attributes of a therapeutic protein or target that affect an interaction between the therapeutic protein and the target, for each of the unbound fraction and bound fraction, the method comprises identifying and quantifying the abundance of each attribute present on a species of the therapeutic protein or target, wherein, when the abundance of an attribute in the unbound fraction is greater than the abundance of the attribute in the bound fraction, the attribute negatively affects the interaction between the therapeutic protein and the target. In various aspects, the method comprises using a mass spectrometer to identify and quantify the abundance of each attribute of the species of the therapeutic protein or target in each of the unbound fraction and bound fraction.

In exemplary embodiments of the method of determining an effect of a known attribute present on a species of a therapeutic protein or target on an interaction between the therapeutic protein and the target, the method comprises for each of the unbound fraction and bound fraction, quantifying the abundance of the known attribute, wherein, when the abundance of the known attribute in the unbound fraction is greater than the abundance of the known attribute in the bound fraction, the known attribute has a negative effect on the interaction between the therapeutic protein and the target. In various aspects, the method comprises using a mass spectrometer to quantify the abundance of the known attribute in each of the unbound fraction and bound fraction.

In exemplary aspects, each of the unbound fraction and bound fraction are subjected to a peptide digestion step, optionally, a trypsin digestion step, prior to the quantifying step or identifying and quantifying step. Optionally, the method comprises carrying out liquid chromatography/mass spectrometry (LC/MS). Protocols for LC-MS peptide mapping are known in the art. See, e.g., Ren et al., Anal. Biochem. 392: 12-21 (2009), Mouchahoir and Schiel, Anal Bioanl Chem 410(8): 2111-2126 (2018). Briefly, the therapeutic protein undergoes a digestion process comprising reduction, alkylation, and digestion to produce peptides with attributes. In some instances, the reduction is carried out with dithiothreitol (DTT), the alkylation is carried out with 2-iodoacetamide (IAA) and the digestion is carried out with trypsin such that all peptides produced terminate in Lys or Arg, or the C-terminal residue of the therapeutic protein. Once produced, the peptides are loaded onto the column for the subsequent chromatography steps. In exemplary embodiments, LC-MS is carried out with a labeling reagent for quantification of abundances of attributes. In exemplary embodiments, the methods do not utilize a labeling reagent in the LC-MS peptide mapping, e.g., LC-MS peptide mapping is carried out in the absence of a labeling agent. In exemplary instances, the measurement of abundances via LC-MS peptide mapping is carried out as two analyses: one for the unbound fraction and another for the bound fraction. In exemplary instances, the two analyses are carried out side-by-side or simultaneously. Such embodiments differ from prior art methods which comprise labeling the unbound fraction with one labeling reagent, labeling the bound fraction with second reagent of a different mass, mixing two samples and performing a single sample preparation procedure and single LC-MS analysis.

In exemplary aspects, the data obtained from LC-MS peptide mapping leads to the identity and quantification of the abundance of each species of each fraction. In exemplary instances, masses of the peptides are accurately measured in on-line mass-spectrometer (MS). Optionally, the peptides are subjected to fragmentation in the mass-spectrometer, and the masses of fragments are also measured (MS/MS) to confirm amino acid sequence and identify and quantify the attributes on amino acids of the peptide. In various aspects, fragmentation at every residue takes place providing single amino acid sequence coverage. If different residues are modified on the same peptide, they have different elution time or mass or both in various aspects. Hence, in some aspects, they will be selected to the fragmentation (MS/MS), and modifications of different residues on the same peptide will be identified and quantified. In exemplary instances, if two attributes happen on the same peptide, modern instrumentation and data processing software are able to identify and quantify them. Attributes in some instances are statistically independent. In some aspects attributes pairing effects were reported, but they are less common (non-stochastic, non-random distribution of modifications). In some aspects, the attributes are random.

In exemplary embodiments, a cross-linking agent is not used during any step of the method. In prior art methods, cross-linking, as well as HDX, Fast Photochemical Oxidation of Proteins (FPOP), are used for assessing higher order structures of proteins and detect presence or absence of protein-bone hydrogen bonds or amino acids side-chain hydrogen bonds or other non-covalent interactions.

### Abundance Ratios and Statistical Significance

In exemplary embodiments of the presently disclosed methods, for each attribute, a ratio of the abundance of the attribute in the unbound fraction to the abundance of the attribute in the bound fraction is determined. In various instances, when the ratio is greater than about 1, the attribute negatively affects the interaction between the therapeutic protein and the target. In various instances, when the ratio is greater than about 1.5, the attribute affects the interaction between the therapeutic protein and the target. In various instances, when the ratio is greater than about 2.0, the attribute affects the interaction between the therapeutic protein and the target. Optionally, the ratio is expressed as log₂. In certain aspects, when the log₂ ratio is greater than about 0.58, the attribute affects the interaction between the therapeutic protein and the target. Optionally, when the log₂ ratio is greater than about 1.0, the attribute affects the interaction between the therapeutic protein and the target.

The method in exemplary embodiments comprises repeating steps of the method, optionally, repeating the separating and quantifying steps, wherein, each time the steps are repeated, a ratio is determined for each attribute to obtain a plurality of ratios and the method comprises determining the statistical significance of the plurality of ratios. In some aspects, the method comprises repeating the steps at least two, three, or four times, optionally, at least five or more times. In exemplary aspects, the method comprises determining the statistical significance of the plurality of ratios comprises calculating the p-value, optionally, wherein the p-value is calculated by a t-test. Methods of calculating the p-value via a t-test are known in the art. See, e.g., Greenland et al., Eur J Epidemiol 31: 37-350 (2016). In various instances, when the *p*-value is less than 0.05 or the negative log₁₀ *p*-value is greater than 1.3, the attribute negatively affects the interaction (e.g., binding interaction) between the therapeutic protein and the target. In various instances, when the *p*-value is less than 0.0005 or the negative log₁₀ *p*-value is greater than 3.3, the attribute negatively affects the interaction (e.g., binding interaction) between the therapeutic protein and the target. In some aspects, the method comprises determining the statistical significance of the plurality of ratios comprises calculating the corrected *p*-value (*p**) of the ratios, wherein *p** = *p*/N, wherein *p* is the *p-*value and N is the total number of tested attributes. In exemplary instances, the method further comprises plotting the average ratio or mean ratio for each attribute in a volcano plot, wherein the statistical significance of the ratio is plotted as a function of the ratio. In various aspects, the abundance is expressed as a ratio and then plotted onto a graph (e.g., a scatter plot graph). Volcano plots are known in the art. See, e.g., The abundance ratios are in some aspects plotted for each attribute and the graph contains all abundance ratios. In exemplary aspects, the abundance ratios are expressed on a log scale, e.g., on a log₂ scale, and the abundance ratios are expressed as a ratio of log₂ abundances. Figure 1B compares the instance wherein abundance ratios are expressed as absolute values vs log₂. As shown in Figure 1B, the log₂ scale is more convenient and often used in statistics, because it provides symmetrical scale for ratios above and below 1. In exemplary aspects, the species is identified as relevant to the interaction between the therapeutic protein and the target, when the ratio is greater than 1.5 (or greater than about 0.58 on a log₂ scale). In exemplary aspects, the species is identified as relevant to the interaction, when the ratio is greater than 2.0 (or greater than about 1 on a log₂ scale). In some aspects, the threshold or cut-off for determining a relevant attribute is made by selecting a cut-off where there are no false-positive results located in the top-left quadrant of a volcano plot. See area bordered by the dashed lines on Figure 1B. For example, in Figure 1B, the two vertical dash lines for the fold change thresholds should be moved symmetrically outward until the top-left quadrant is clear. The (-log₁₀ p-value) horizontal threshold line should be moved up until the top-left quadrant is clear. These thresholds in some instances define the ratio and p-value for the top right quadrant above which the attributes/modifications reduce binding with statistical significance.

### Therapeutic Protein Type And Affinity

Advantageously, the presently disclosed methods are not limited to any particular type of therapeutic protein which participates in an interaction with a target, e.g., a binding interaction. In exemplary instances, the therapeutic protein interacts with a target which is a protein. In various aspects, the therapeutic protein comprises a protein native or endogenously produced in nature, e.g., by a mammal, e.g., a human. Alternatively, in some aspects, the therapeutic protein is not found in nature or endogenously produced in nature by an organism, and is a synthetic or engineered protein. In some aspects, the therapeutic protein is a hormone, growth factor, cytokine, a cell-surface receptor, or any ligand thereof. In some instances, the therapeutic protein is a fusion protein, a chimeric protein, a protein fragment or a conjugate comprising a protein.

In exemplary aspects, the therapeutic protein comprises an antibody, an antigen-binding antibody fragment, or an antibody protein product. Collectively, antibodies form a family of plasma proteins known as immunoglobulins and comprise of immunoglobulin domains. (Janeway et al., Immunobiology: The Immune System in Health and Disease, 4th ed., Elsevier Science Ltd./Garland Publishing, 1999. As used herein, the term "antibody" refers to a protein having a conventional immunoglobulin format, comprising heavy and light chains, and comprising variable and constant regions. For example, an antibody may be an IgG which is a "Y-shaped" structure of two identical pairs of polypeptide chains, each pair having one "light" (typically having a molecular weight of about 25 kDa) and one "heavy" chain (typically having a molecular weight of about 50-70 kDa). An antibody has a variable region and a constant region. In IgG formats, the variable region is generally about 100-110 or more amino acids, comprises three complementarity determining regions (CDRs), is primarily responsible for antigen recognition, and substantially varies among other antibodies that bind to different antigens. The constant region allows the antibody to recruit cells and molecules of the immune system. The variable region is made of the N-terminal regions of each light chain and heavy chain, while the constant region is made of the C-terminal portions of each of the heavy and light chains. (Janeway et al., "Structure of the Antibody Molecule and the Immunoglobulin Genes", Immunobiology: The Immune System in Health and Disease, 4th ed. Elsevier Science Ltd./Garland Publishing, (1999)).

The general structure and properties of CDRs of antibodies have been described in the art. Briefly, in an antibody scaffold, the CDRs are embedded within a framework in the heavy and light chain variable region where they constitute the regions largely responsible for antigen binding and recognition. A variable region typically comprises at least three heavy or light chain CDRs (Kabat et al., 1991, Sequences of Proteins of Immunological Interest, Public Health Service N.I.H., Bethesda, Md.; see also Chothia and Lesk, 1987, J. Mol. Biol. 196:901-917; Chothia et al., 1989, Nature 342: 877-883), within a framework region (designated framework regions 1-4, FR1, FR2, FR3, and FR4, by Kabat et al., 1991; see also Chothia and Lesk, 1987, supra).

Antibodies can comprise any constant region known in the art. Human light chains are classified as kappa and lambda light chains. Heavy chains are classified as mu, delta, gamma, alpha, or epsilon, and define the antibody's isotype as IgM, IgD, IgG, IgA, and IgE, respectively. IgG has several subclasses, including, but not limited to IgG1, IgG2, IgG3, and IgG4. IgM has subclasses, including, but not limited to, IgM1 and IgM2. Embodiments of the present disclosure include all such classes or isotypes of antibodies. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region. Accordingly, in exemplary embodiments, the antibody is an antibody of isotype IgA, IgD, IgE, IgG, or IgM, including any one of IgG1, IgG2, IgG3 or IgG4.

The antibody can be a monoclonal antibody or a polyclonal antibody. In some embodiments, the antibody comprises a sequence that is substantially similar to a naturally-occurring antibody produced by a mammal, e.g., mouse, rabbit, goat, horse, chicken, hamster, human, and the like. In this regard, the antibody can be considered as a mammalian antibody, e.g., a mouse antibody, rabbit antibody, goat antibody, horse antibody, chicken antibody, hamster antibody, human antibody, and the like. In certain aspects, the antibody is a human antibody. In certain aspects, the antibody is a chimeric antibody or a humanized antibody. The term "chimeric antibody" refers to an antibody containing domains from two or more different antibodies. A chimeric antibody can, for example, contain the constant domains from one species and the variable domains from a second, or more generally, can contain stretches of amino acid sequence from at least two species. A chimeric antibody also can contain domains of two or more different antibodies within the same species. The term "humanized" when used in relation to antibodies refers to antibodies having at least CDR regions from a non-human source which are engineered to have a structure and immunological function more similar to true human antibodies than the original source antibodies. For example, humanizing can involve grafting a CDR from a non-human antibody, such as a mouse antibody, into a human antibody. Humanizing also can involve select amino acid substitutions to make a non-human sequence more similar to a human sequence.

An antibody can be cleaved into fragments by enzymes, such as, e.g., papain and pepsin. Papain cleaves an antibody to produce two Fab fragments and a single Fc fragment. Pepsin cleaves an antibody to produce a F(ab')₂ fragment and a pFc' fragment. In exemplary aspects of the present disclosure, the therapeutic protein comprises an antigen binding antibody fragment. As used herein, the term "antigen binding antibody fragment refers to a portion of an antibody that is capable of binding to the antigen of the antibody and is also known as "antigen-binding fragment" or "antigen-binding portion". In exemplary instances, the antigen binding antibody fragment is a Fab fragment or a F(ab')₂ fragment.

The architecture of antibodies has been exploited to create a growing range of alternative formats that span a molecular-weight range of at least about 12-150 kDa and has a valency (n) range from monomeric (n = 1), to dimeric (n = 2), to trimeric (n = 3), to tetrameric (n = 4), and potentially higher; such alternative formats are referred to herein as "antibody protein products". Antibody protein products include those based on the full antibody structure and those that mimic antibody fragments which retain full antigen-binding capacity, e.g., scFvs, Fabs and VHH/VH (discussed below). The smallest antigen binding antibody fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria- and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ~15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al., mAbs 4(5): 586-591 (2012).

Other antibody protein products include a single chain antibody (SCA); a diabody; a triabody; a tetrabody; bispecific or trispecific antibodies, and the like. Bispecific antibodies can be divided into five major classes: BsIgG, appended IgG, BsAb fragments, bispecific fusion proteins and BsAb conjugates. See, e.g., Spiess et al., Molecular Immunology 67(2) Part A: 97-106 (2015).

In exemplary aspects, the therapeutic protein comprises any one of these antibody protein products. In exemplary aspects, the therapeutic protein comprises any one of an scFv, Fab VHH/VH, Fv fragment, ds-scFv, scFab, dimeric antibody, multimeric antibody (e.g., a diabody, triabody, tetrabody), miniAb, peptibody VHH/VH of camelid heavy chain antibody, sdAb, diabody; a triabody; a tetrabody; a bispecific or trispecific antibody, BsIgG, appended IgG, BsAb fragment, bispecific fusion protein, and BsAb conjugate.

In exemplary aspects, the therapeutic protein is a bispecific T cell engager (BiTE) molecule. A BiTE molecule is a bispecific antibody construct or bispecific fusion protein comprising two antibody binding domains (or targeting regions) linked together. BiTEs are constructed of two single chain variable fragments (scFv) connected in tandem by a flexible linker (scFc-scFc, Huehls et al, Immunol Cell Biol 93(3): 290-296 (2015)). One arm of the molecule is engineered to bind with a protein found on the surface of cytotoxic T cells, and the other arm is designed to bind to a specific protein found primarily on tumor cell. When both targets are engaged, the BiTE molecule forms a bridge between the cytotoxic T cell and the tumor cell, which enables the T cell to recognize the tumor cell and fight it through an infusion of toxic molecules. The tumor-binding arm of the molecule can be altered to create different BiTE antibody constructs that target different types of cancer. The term "binding domain" in regards to a BiTE molecule refers to a domain which (specifically) binds to / interacts with / recognizes a given target epitope or a given target site on the target molecules (antigens). The structure and function of the first binding domain (recognizing the tumor cell antigen), and preferably also the structure and/or function of the second binding domain (cytotoxic T cell antigen), is/are based on the structure and/or function of an antibody, e.g. of a full-length or whole immunoglobulin molecule. For example, the BiTE molecule comprises a first binding domain characterized by the presence of three light chain CDRs (i.e. CDR1, CDR2 and CDR3 of the VL region) and three heavy chain CDRs (i.e. CDR1, CDR2 and CDR3 of the VH region). The second binding domain preferably also comprises the minimum structural requirements of an antibody which allow for the target binding. More preferably, the second binding domain comprises at least three light chain CDRs (i.e. CDR1, CDR2 and CDR3 of the VL region) and/or three heavy chain CDRs (i.e. CDR1, CDR2 and CDR3 of the VH region). It is envisaged that the first and/or second binding domain is produced by or obtainable by phage-display or library screening methods rather than by grafting CDR sequences from a pre-existing (monoclonal) antibody into a scaffold. A binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of (modified) antigen-binding antibody fragments include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv) , the latter being preferred (for example, derived from an scFV-library).

In exemplary aspects, the therapeutic protein is a bi-specific antibody, a tri-specific antibody, a molecule, or an Fc fusion protein. In some aspects, the therapeutic protein comprises a single chain Fragment variable (scFv) which binds to a first target, optionally, wherein the therapeutic protein comprises a second scFV which binds to a second target (optionally different from the first target).

In exemplary instances, the Kd of the therapeutic protein - target interaction (e.g., binding interaction) is about 10⁻¹¹ M to about 10⁻⁹ M and the affinity purification technique is SEC, precipitation using binding target-labeled beads, or precipitation with cells with on-surface expressed targets. Fc-containing therapeutic proteins also interact with Fc receptors and the interactions are about 10⁻⁸ to about 10⁻⁶ M. FcRn and Fc gamma are perhaps the most important Fc receptors. FcRn and Fc gamma receptors bind weakly to Fc-containing proteins with Kd at about 10⁻⁸ to about 10⁻⁶ M. FcRn receptors are present on surface of many cells and allow internalization and then recycling to the surface of Fc-containing proteins, extending their life in circulation. Fc gamma receptors are present on the cells of immune system and involved in the antibody-dependent cellular cytotoxicity (ADCC), recognition and killing of the target by the immune cells. Precipitation with FcRn-labeled beads, Fc gamma receptor-labeled beads, or precipitation with cells with surface expressed FcRn and Fc gamma receptors. The complexes of Fc-containing proteins and Fc receptors are weakly bond, typically dissociate on SEC column after injection on the column, become diluted on the column, do not associate and elute separately. Once dissociated after injection on SEC column, the mixture is diluted and possibility of association is diminished. On the other hand, interaction in a tube (container) or on affinity column with the Fc receptors immobilized on beads maintains the original, high concentration of the protein and Fc receptor mixture and allows both, dissociation and association.

In exemplary embodiments, the therapeutic protein comprises an antibody, antigen-binding antibody fragment, or antibody protein product, optionally, a Bi-specific T cell engager (BiTE), bispecific antibody, trispecific antibody, Fc fusion protein. In some embodiments, the therapeutic protein is a BiTE^{®} molecule. BiTE^{®} molecules are engineered bispecific monoclonal antibodies which direct the cytotoxic activity of T cells against cancer cells. They are the fusion of two single-chain variable fragments (scFvs) of different antibodies, or amino acid sequences from four different genes, on a single peptide chain of about 55 kilodaltons. One of the scFvs binds to T cells via the CD3 receptor, and the other to a tumor cell via a tumor specific molecule. Blinatumomab (BLINCYTO^{®}) is an example of a BiTE^{®} molecule, specific for CD19. BiTE^{®} molecules that are modified, such as those modified to extend their half-lives, can also be used in the disclosed methods.

In various aspects, the therapeutic protein is Activase^{®} (Alteplase); alirocumab, Aranesp^{®} (Darbepoetin-alfa), Epogen^{®} (Epoetin alfa, or erythropoietin); Avonex^{®} (Interferon β-Ia); Bexxar^{®} (Tositumomab); Betaseron^{®} (Interferon-β); bococizumab (anti-PCSK9 monoclonal antibody designated as L1L3, see US8080243); Campath^{®} (Alemtuzumab); Dynepo^{®} (Epoetin delta); Velcade^{®} (bortezomib); MLN0002 (anti-α4β7 mAb); MLN1202 (anti-CCR2 chemokine receptor mAb); Enbrel^{®} (etanercept); Eprex^{®} (Epoetin alfa); Erbitux^{®} (Cetuximab); evolocumab; Genotropin^{®} (Somatropin); Herceptin^{®} (Trastuzumab); Humatrope^{®} (somatropin [rDNA origin] for injection); Humira^{®} (Adalimumab); Infergen^{®} (Interferon Alfacon-1); Natrecor^{®} (nesiritide); Kineret^{®} (Anakinra), Leukine^{®} (Sargamostim); LymphoCide^{®} (Epratuzumab); BenlystaTM (Belimumab); Metalyse^{®} (Tenecteplase); Mircera^{®} (methoxy polyethylene glycol-epoetin beta); Mylotarg^{®} (Gemtuzumab ozogamicin); Raptiva^{®} (efalizumab); Cimzia^{®} (certolizumab pegol); SolirisTM (Eculizumab); Pexelizumab (Anti-C5 Complement); MEDI-524 (Numax^{®}); Lucentis^{®} (Ranibizumab); Edrecolomab (,Panorex^{®}); Trabio^{®} (lerdelimumab); TheraCim hR3 (Nimotuzumab); Omnitarg (Pertuzumab, 2C4); Osidem^{®} (IDM-I); OvaRex^{®} (B43.13); Nuvion^{®} (visilizumab); Cantuzumab mertansine (huC242-DMI); NeoRecormon^{®} (Epoetin beta); Neumega^{®} (Oprelvekin); Neulasta^{®} (Pegylated filgastrim, pegylated G-CSF, pegylated hu-Met-G-CSF); Neupogen^{®} (Filgrastim); Orthoclone OKT3^{®} (Muromonab-CD3), Procrit^{®} (Epoetin alfa); Remicade^{®} (Infliximab), Reopro^{®} (Abciximab), Actemra^{®} (anti-IL6 Receptor mAb), Avastin^{®} (Bevacizumab), HuMax-CD4 (zanolimumab), Rituxan^{®} (Rituximab); Tarceva^{®} (Erlotinib); Roferon-A^{®}-(lnterferon alfa-2a); Simulect^{®} (Basiliximab); StelaraTM (Ustekinumab); Prexige^{®} (lumiracoxib); Synagis^{®} (Palivizumab); 146B7-CHO (anti-IL15 antibody, see US7153507), Tysabri^{®} (Natalizumab); Valortim^{®} (MDX-1303, anti-B. anthracis Protective Antigen mAb); ABthraxTM; Vectibix^{®} (Panitumumab); Xolair^{®} (Omalizumab), ETI211 (anti-MRSA mAb), IL-I Trap (the Fc portion of human IgGl and the extracellular domains of both IL-I receptor components (the Type I receptor and receptor accessory protein)), VEGF Trap (Ig domains of VEGFR! fused to IgGl Fc), Zenapax^{®} (Daclizumab); Zenapax^{®} (Daclizumab), Zevalin^{®} (Ibritumomab tiuxetan), Zetia (ezetimibe), Atacicept (TACI-Ig), anti-α4β7 mAb (vedolizumab); galiximab (anti-CD80 monoclonal antibody), anti-CD23 mAb (lumiliximab); BR2-Fc (huBR3 / huFc fusion protein, soluble BAFF antagonist); SimponiTM (Golimumab); Mapatumumab (human anti-TRAIL Receptor-1 mAb); Ocrelizumab (anti-CD20 human mAb); HuMax-EGFR (zalutumumab); M200 (Volociximab, anti-α5β1 integrin mAb); MDX-010 (Ipilimumab, anti-CTLA-4 mAb and VEGFR-I (IMC-18F1); anti-BR3 mAb; anti-C. difficile Toxin A and Toxin B C mAbs MDX-066 (CDA-I) and MDX-1388); anti-CD22 dsFv-PE38 conjugates (CAT-3888 and CAT-8015); anti-CD25 mAb (HuMax-TAC); anti-TSLP antibodies; anti-TSLP receptor antibody (US8101182); anti-TSLP antibody designated as A5 (US7982016); (anti-CD3 mAb (NI-0401); Adecatumumab (MT201, anti-EpCAM-CD326 mAb); MDX-060, SGN-30, SGN-35 (anti-CD30 mAbs); MDX-1333 (anti- IFNAR); HuMax CD38 (anti-CD38 mAb); anti-CD40L mAb; anti-Cripto mAb; anti-CTGF Idiopathic Pulmonary Fibrosis Phase I Fibrogen (FG-3019); anti-CTLA4 mAb; anti-eotaxin! mAb (CAT-213); anti-FGF8 mAb; anti-ganglioside GD2 mAb; anti-sclerostin antibodies (see, US8715663 or US7592429) anti-sclerostin antibody designated as Ab-5 (US8715663 or US7592429); anti-ganglioside GM2 mAb; anti-GDF-8 human mAb (MYO-029); anti-GM-CSF Receptor mAb (CAM-3001); anti-HepC mAb (HuMax HepC); MEDI-545, MDX-1103 (anti-IFNα mAb); anti-IGFIR mAb; anti-IGF-IR mAb (HuMax-Inflam); anti-IL12/IL23p40 mAb (Briakinumab); anti-IL-23p19 mAb (LY2525623); anti-IL13 mAb (CAT-354); anti-IL-17 mAb (AIN457); anti-IL2Ra mAb (HuMax-TAC); anti-IL5 Receptor mAb; anti-integrin receptors mAb (MDX-OI8, CNTO 95); anti-IPIO Ulcerative Colitis mAb (MDX- 1100); anti-LLY antibody; BMS-66513; anti-Mannose Receptor/hCGβ mAb (MDX-1307); anti-mesothelin dsFv-PE38 conjugate (CAT-5001); anti-PD!mAb (MDX-1106 (ONO- 4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; anti- ZP3 mAb (HuMax-ZP3); NVS Antibody #1; NVS Antibody #2; or an amyloid-beta monoclonal antibody, or an antibody listed in TABLE D. Optionally, the therapeutic protein is Other examples of suitable antibodies include infliximab, bevacizumab, cetuximab, ranibizumab, palivizumab, abagovomab, abciximab, actoxumab, adalimumab, afelimomab, afutuzumab, alacizumab, alacizumab pegol, ald518, alemtuzumab, alirocumab, altumomab, amatuximab, anatumomab mafenatox, anrukinzumab, apolizumab, arcitumomab, aselizumab, altinumab, atlizumab, atorolimiumab, tocilizumab, bapineuzumab, basiliximab, bavituximab, bectumomab, belimumab, benralizumab, bertilimumab, besilesomab, bevacizumab, bezlotoxumab, biciromab, bivatuzumab, bivatuzumab mertansine, blinatumomab, blosozumab, brentuximab vedotin, briakinumab, brodalumab, canakinumab, cantuzumab mertansine, cantuzumab mertansine, caplacizumab, capromab pendetide, carlumab, catumaxomab, cc49, cedelizumab, certolizumab pegol, cetuximab, citatuzumab bogatox, cixutumumab, clazakizumab, clenoliximab, clivatuzumab tetraxetan, conatumumab, crenezumab, cr6261, dacetuzumab, daclizumab, dalotuzumab, daratumumab, demcizumab, denosumab, detumomab, dorlimomab aritox, drozitumab, duligotumab, dupilumab, ecromeximab, eculizumab, edobacomab, edrecolomab, efalizumab, efungumab, elotuzumab, elsilimomab, enavatuzumab, enlimomab pegol, enokizumab, enoticumab, ensituximab, epitumomab cituxetan, epratuzumab, erenumab, erlizumab, ertumaxomab, etaracizumab, etrolizumab, evolocumab, exbivirumab, fanolesomab, faralimomab, farletuzumab, fasinumab, fbta05, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gemtuzumab ozogamicin, gevokizumab, girentuximab, glembatumumab vedotin, golimumab, gomiliximab, gs6624, ibalizumab, ibritumomab tiuxetan, icrucumab, igovomab, imciromab, imgatuzumab, inclacumab, indatuximab ravtansine, infliximab, intetumumab, inolimomab, inotuzumab ozogamicin, ipilimumab, iratumumab, itolizumab, ixekizumab, keliximab, labetuzumab, lebrikizumab, lemalesomab, lerdelimumab, lexatumumab, libivirumab, ligelizumab, lintuzumab, lirilumab, lorvotuzumab mertansine, lucatumumab, lumiliximab, mapatumumab, maslimomab, mavrilimumab, matuzumab, mepolizumab, metelimumab, milatuzumab, minretumomab, mitumomab, mogamulizumab, morolimumab, motavizumab, moxetumomab pasudotox, muromonab-cd3, nacolomab tafenatox, namilumab, naptumomab estafenatox, narnatumab, natalizumab, nebacumab, necitumumab, nerelimomab, nesvacumab, nimotuzumab, nivolumab, nofetumomab merpentan, ocaratuzumab, ocrelizumab, odulimomab, ofatumumab, olaratumab, olokizumab, omalizumab, onartuzumab, oportuzumab monatox, oregovomab, orticumab, otelixizumab, oxelumab, ozanezumab, ozoralizumab, pagibaximab, palivizumab, panitumumab, panobacumab, parsatuzumab, pascolizumab, pateclizumab, patritumab, pemtumomab, perakizumab, pertuzumab, pexelizumab, pidilizumab, pintumomab, placulumab, ponezumab, priliximab, pritumumab, PRO 140, quilizumab, racotumomab, radretumab, rafivirumab, ramucirumab, ranibizumab, raxibacumab, regavirumab, reslizumab, rilotumumab, rituximab, robatumumab, roledumab, romosozumab, rontalizumab, rovelizumab, ruplizumab, samalizumab, sarilumab, satumomab pendetide, secukinumab, sevirumab, sibrotuzumab, sifalimumab, siltuximab, simtuzumab, siplizumab, sirukumab, solanezumab, solitomab, sonepcizumab, sontuzumab, stamulumab, sulesomab, suvizumab, tabalumab, tacatuzumab tetraxetan, tadocizumab, talizumab, tanezumab, taplitumomab paptox, tefibazumab, telimomab aritox, tenatumomab, tefibazumab, teneliximab, teplizumab, teprotumumab, TGN1412, tremelimumab, ticilimumab, tildrakizumab, tigatuzumab, TNX-650, tocilizumab, toralizumab, tositumomab, tralokinumab, trastuzumab, TRBS07, tregalizumab, tucotuzumab celmoleukin, tuvirumab, ublituximab, urelumab, urtoxazumab, ustekinumab, vapaliximab, vatelizumab, vedolizumab, veltuzumab, vepalimomab, vesencumab, visilizumab, volociximab, vorsetuzumab mafodotin, votumumab, zalutumumab, zanolimumab, zatuximab, ziralimumab, zolimomab aritox.

**TABLE D**

| Examples of therapeutic antibodies | | | | | |
|---|---|---|---|---|---|
| **Target (informal name)** | **HC* Type (including allotypes)** | **LC* Type** | **pl** | **LC* SEQ ID NO:** | **HC* SEQ ID NO:** |
| anti-amyloid | IgG1 (f) (R;EM) | Kappa | 9.0 | 1 | 2 |
| GMCSF (247) | IgG2 | Kappa | 8.7 | 3 | 4 |
| CGRPR | IgG2 | Lambda | 8.6 | 5 | 6 |
| RANKL | IgG2 | Kappa | 8.6 | 7 | 8 |
| Sclerostin (27H6) | IgG2 | Kappa | 6.6 | 9 | 10 |
| IL-1R1 | IgG2 | Kappa | 7.4 | 11 | 12 |
| Myostatin | IgG1 (z) (K;EM) | Kappa | 8.7 | 13 | 14 |
| B7RP1 | IgG2 | Kappa | 7.7 | 15 | 16 |
| Amyloid | IgG1 (za) (K;DL) | Kappa | 8.7 | 17 | 18 |
| GMCSF (3.112) | IgG2 | Kappa | 8.8 | 19 | 20 |
| CGRP (32H7) | IgG2 | Kappa | 8.7 | 21 | 22 |
| CGRP (3B6.2) | IgG2 | Lambda | 8.6 | 23 | 24 |
| PCSK9 (8A3.1) | IgG2 | Kappa | 6.7 | 25 | 26 |
| PCSK9 (492) | IgG2 | Kappa | 6.9 | 27 | 28 |
| CGRP | IgG2 | Lambda | 8.8 | 29 | 30 |
| Hepcidin | IgG2 | Lambda | 7.3 | 31 | 32 |
| TNFR p55) | IgG2 | Kappa | 8.2 | 33 | 34 |
| OX40L | IgG2 | Kappa | 8.7 | 35 | 36 |
| HGF | IgG2 | Kappa | 8.1 | 37 | 38 |
| GMCSF | IgG2 | Kappa | 8.1 | 39 | 40 |
| Glucagon R | IgG2 | Kappa | 8.4 | 41 | 42 |
| GMCSF (4.381) | IgG2 | Kappa | 8.4 | 43 | 44 |
| Sclerostin (13F3) | IgG2 | Kappa | 7.8 | 45 | 46 |
| CD-22 | IgG1 (f) (R;EM) | Kappa | 8.8 | 47 | 48 |
| INFgR | IgG1 (za) (K;DL) | Kappa | 8.8 | 49 | 50 |
| Ang2 | IgG2 | Kappa | 7.4 | 51 | 52 |
| TRAILR2 | IgG1 (f) (R;EM) | Kappa | 8.7 | 53 | 54 |
| EGFR | IgG2 | Kappa | 6.8 | 55 | 56 |
| IL-4R | IgG2 | Kappa | 8.6 | 57 | 58 |
| IL-15 | IgG1 (f) (R;EM) | Kappa | 8.8 | 59 | 60 |
| IGF1R | IgG1 (za) (K;DL) | Kappa | 8.6 | 61 | 62 |
| IL-17R | IgG2 | Kappa | 8.6 | 63 | 64 |
| Dkk1 (6.37.5) | IgG2 | Kappa | 8.2 | 65 | 66 |
| Sclerostin | IgG2 | Kappa | 7.4 | 67 | 68 |
| TSLP | IgG2 | Lambda | 7.2 | 69 | 70 |
| Dkk1 (11H10) | IgG2 | Kappa | 8.2 | 71 | 72 |
| PCSK9 | IgG2 | Lambda | 8.1 | 73 | 74 |
| GIPR (2G10.006) | IgG1 (z) (K;EM) | Kappa | 8.1 | 75 | 76 |
| Activin | IgG2 | Lambda | 7.0 | 77 | 78 |
| Sclerostin (2B8) | IgG2 | Lambda | 6.7 | 79 | 80 |
| Sclerostin | IgG2 | Kappa | 6.8 | 81 | 82 |
| c-fm s | IgG2 | Kappa | 6.6 | 83 | 84 |
| α4β7 | IgG2 | Kappa | 6.5 | 85 | 86 |
| PD-1 | IgG2 | Kappa | | 87 | 88 |

| | | | | | |
|---|---|---|---|---|---|
| *HC - antibody heavy chain; LC - antibody light chain. | | | | | |

In various aspects, the therapeutic protein comprises an Fc domain. In various aspects, the therapeutic protein has a relatively weak affinity for the target. In various aspects, the therapeutic protein has an equilibrium dissociation constant (K_{D}) for the target that is about 10⁻⁸ to about 10⁻⁶ M. Optionally, the mixture is separated into at least two fractions by precipitation using beads or cells bound to capture molecule. In some aspects, the capture molecule is FcRn or Fc gamma receptor. Fc-containing therapeutic proteins also interact with Fc receptors and the interactions are about 10⁻⁸ to about 10⁻⁶ M. FcRn and Fc gamma are in some aspects very important Fc receptors. FcRn and Fc gamma receptors bind weakly to Fc-containing proteins with Kd of about 10⁻⁸ to about 10⁻⁶ M. FcRn receptors are present on the surface of many cells and allow internalization and then recycling to the surface of Fc-containing proteins, extending their life in circulation. Fc gamma receptors are present on the cells of immune system and are involved in antibody-dependent cellular cytotoxicity (ADCC), the recognition and killing of the target by the immune cells. Precipitation can also be accomplished, for example, with FcRn-labeled beads, Fc gamma receptor-labeled beads, or precipitation with cells with surface expressed FcRn and Fc gamma receptors. The complexes of Fc-containing proteins and Fc receptors are weakly bond, typically dissociate on SEC column after injection on the column, become diluted on the column, do not associate and elute separately. Once dissociated after injection on SEC column, the mixture is diluted and possibility of association is diminished. On the other hand, interaction in a tube (container) or on affinity column with the Fc receptors immobilized on beads maintains the original, high concentration of the protein and Fc receptor mixture and allows both, dissociation and association. In exemplary instances for Fc-containing therapeutic proteins that weakly interact with Fc receptors having a K_{D} of about 10⁻⁸ to about 10⁻⁶ M, the method comprises adding the mixture to a container, e.g., tube, comprising beads bound to the capture molecule or cells expressing at its surface the capture molecule, (ii) centrifuging the tube comprising the mixture and the beads or cells, and (iii) removing the supernatant from the pellet to obtain a first fraction and a second fraction, optionally, wherein the capture molecule is Fc receptor (FcRn or Fc gamma).

In various aspects, the therapeutic protein comprises a single chain Fragment variable (scFv) which binds to a target, and in some aspects, the therapeutic protein has a K_{D} for the target that is about 10⁻¹¹ to about 10⁻⁹ M. Optionally, the mixture is separated into at least two fractions by SEC or by precipitation using beads or cells. In exemplary instances, the Kd of the therapeutic protein - target interaction (e.g., binding interaction) is about 10⁻¹¹ M to about 10⁻⁹ M and the separation technique is SEC, precipitation using binding target-labeled beads, or precipitation with cells with on-surface expressed targets.

### Epitope mapping procedures

The described methods can be also used for epitope mapping. "Epitope mapping" refers to the identification of binding residues on a target protein. For that, the target protein is stressed under the same or similar stress conditions as the therapeutic antibody. The rest of the experiment flow can be the same as described for methods of paratope mapping, except bound target (in antibody-target complex) and unbound target fractions are collected and compared. It is contemplated that when a structure (e.g., attribute) on a target negatively affects the interaction between the target and a therapeutic protein, the structure is a candidate for being part of (e.g., being on a residue of) an epitope of the target.

### Alternative approaches for epitope/paratope mapping

Epitope/paratope mapping results in identification of residues in the antigen (epitope) that are bound by the residues in antibody (paratope). The mapping is a useful tool for understanding antibody-target interactions, elucidation of stoichiometry of the interaction and mechanism of drug action. This knowledge facilitates design optimization for next generation of therapeutic antibodies including optimization of binding strength, stoichiometry, mechanism of blocking or internalization together with the receptor from cell surface. Several techniques have been traditionally utilized for epitope/paratope mapping (Abbott *et al.,* 2014), (Opuni *et al.,* 2018), (Goswami et *al.,* 2018), including X-ray crystallography (Luo *et al.,* 2015); nuclear magnetic resonance (NMR) spectroscopy (Blech *et al.,* 2013); scanning mutagenesis, followed by a binding assay (Blech *et al.,* 2013); and mass spectrometry-based techniques including hydrogen deuterium exchange (HDX) (Englander, 2006), (Sevy *et al.,* 2013); fast photochemical oxidation of proteins (FPOP) (Jones *et al.,* 2011); differential chemical modification (Dhungana *et al.,* 2009).

X-ray crystallography provides most accurate determination of interactions between both side chains and main chain atoms of individual amino acids in both the epitope and paratope. Amino acids that are within 5Å of each other are generally considered to be contacting residues. The close proximity is not a guaranty of binding, however, and another technique, like NMR (Blech *et al.,* 2013) or scanning mutagenesis (Englander, 2006) are required to more confidently identify the biding residues. Also, obtaining diffraction quality crystals is often laborious and lengthy process, and is sometimes impossible for certain antibody-antigen pairs. NMR is another technique for high-resolution epitope mapping (Blech *et al.,* 2013). It is a complex method, however, and requires a previous crystal structure determination of the free antigen and full resonance assignment of a ¹⁵N-, ¹³C- and potentially ²H-labelled protein that will need to be expressed in mammalian or microbial systems with the isotopically labeled nutrients.

Scanning mutagenesis, followed by a binding assay of either antigen or antibody is an analytically and technically simpler technique. It analyzes binding to mutated forms of the antigen or antibody. Loss of binding to a particular mutant is interpreted that the mutated amino acid constitutes an epitope or paratope. The mutagenesis also provides an advantage to screen many hundreds of proteins by using alanine scanning or shotgun mutagenesis. Although the technique can potentially provide epitope/paratope information at the amino acid level, it suffers from the uncertainty of not knowing whether the mutation genuinely impacted a key interacting residue or just disrupted the folding of the mutated protein (Abbott *et al.,* 2014). Without being limited by theory, in the case of paratope mapping of antibodies, scanning mutagenesis (or the chemical modifications of amino acids) should not change folding, and it should be more accurate than on antigen. This is because paratopes are typically located on CDRs, which are unfolded, unstructured, flexible loops. Therefore, substituting amino acids or chemically modifying the side chains is expected to have lower impact on folding. Described herein is paratope/epitope mapping by SEC of stressed antibody-target complexes followed by mass spectrometry characterization is somewhat similar technique to mutagenesis, but with the following key differences. Instead of mutations, side chains of amino acid residues are altered by chemical modifications. And, instead of one-by-one binding assay measurements, the method allows parallel characterization of molecules with chemical modifications on different residues.

During epitope/paratope mapping by HDX-MS, hydrogen-deuterium exchange rate of each backbone amide hydrogen in the antibody-antigen complex is compared to that in the free antigen or antibody. The residues with slower exchange rate in the complex are due to extra protection caused by the binding. In FPOP method, a laser is used to photolyze hydrogen peroxide, which is added at low concentration to the protein solution, to form hydroxyl radicals and chemically modify side chains of surface-exposed residues. The residues with slower modification rate in the complex are due to extra protection caused by the binding. The use of a pulsed laser and a radical scavenger limits radical lifetimes, ensuring labeling on a microsecond time scale, faster than most protein unfolding. This is to ensure that loss of interaction is not caused by the unfolding. The principle of differential chemical modification method is the differential surface-exposure and reagent-accessibility of a protein antigen in the presence and absence of an antibody that determines the rate and the extent of chemical modification of amino acid residues on the antigen (Dhungana *et al.,* 2009). In all mass spectrometry-based techniques, the amino acid modifications are performed after binding, not before, as in the newly proposed method.

The following examples are given merely to illustrate the present invention and not in any way to limit its scope.

### EXAMPLES

### EXAMPLE 1

This example describes an exemplary method of the present disclosures wherein the therapeutic protein is an IgG antibody specific for Antigen 1 and the target is a soluble form of the carbohydrate binding domain (CBD) of Antigen 1.

### Materials and Methods

### Materials

Antibody 1 is an IgG antibody with a molecular weight of 149 kDa. Antibody 1 was formulated at a concentration of 70 mg/ml in a solution comprising 10 mM acetate (pH 5.2) and 9% sucrose. The target of Antibody 1 used in the experiments was a 17,043.5 Da soluble portion of the carbohydrate binding domain (CBD) of Antigen 1 tagged with six His residues. The target was formulated at a concentration of 0.81 mg/mL in a solution comprising 30 mM HEPES (pH 7.6), 0.15M NaCl, and 3mM CaCl₂ and stored at -70°C before use.

### Photo-Stress Protocol (P2)

Antibody 1 was photo-stressed by exposing to cool white light and ultraviolet (UV) light to cause instability causing attribute formation and thus the formation of different species of Antibody 1. The sample was subjected to Photo-Stress Protocol P2 according to International Conference on Harmonization of Technical Requirements for Registration of Pharmaceuticals for Human Use (ICH) guideline ICH Q1B "Stability testing, photostability testing of new drug substances and products" (available at http://academy.gmp-compliance.org/guidemgr/files/MEDIA412.PDF). Briefly, Antibody 1 samples in glass vials were exposed to an illumination of approximately 1.2 million lux hours consisting of both cool white fluorescence and at least 200 watt hours/square meter of UV irradiance. All samples were maintained at 25°C. A cool white fluorescent lamp was set at 8 klux, and time of incubation was 1.2 million lux hours / 8 klux = 150 hours. For UV irradiation, UV lamp was set at 10 watt per square meter, and time of incubation was 200 watt hours per square meter / 10 watt per square meter = 20 hours. The Photo-Stress Protocol P2 including the UV irradiation was harsh and more than the typical stress therapeutic proteins experience during production, purification, formulation, storage and in human circulation. However, it is capable of producing light-induced oxidation on solvent exposed tryptophan, methionine and histidine residues and therefore useful.

### Mixing Antibody 1 with its Target

Antibody 1 (38 µL of 0.25 mg/mL Antibody 1 solution) was mixed with the target (2 µL of 0.81 mg/mL AGSR1 solution) at 1:2 molar ratio. It was previously established that unstressed antibody molecule binds two target molecules, which is typical for bivalent antibodies (such as those having two Fab arms). The mixture was incubated at 37 °C in an HPLC vial for 10 minutes. Then a 20 µL aliquot of the mixture was injected onto an HPLC column for SEC separation, as described below.

### Size Exclusion Chromatography (SEC) and Fraction Collection

After incubation, the Antibody 1-target mixture was separated by SEC using a G3000SWxl TOSOH Bioscience, 7.8mm ID x 30cm column (Catalog # 08541, TOSOH Bioscience, San Francisco, CA) and the mobile-phase included 100 mM sodium phosphate and 250 mM NaC! (pH 6.8). The flow rate was set at 0.5 ml/min, the column temperature was set at 37°C, the run time was 35 minutes, and the auto sampler was set at 4°C. Ultraviolet/Visible spectrometry (UV/VIS) detection was performed at 214 nm and 280 nm. The eluting fractions were collected using a filter with a molecular weight cut-off of above 10 kDa and eluted with a 7.5 M guanidine elution buffer. The eluted fractions were subjected to sample preparation for peptide mapping described below.

### Peptide Mapping

Peptide mapping of the collected fractions was performed using the sample preparation procedure including refolding with guanidine, reduction and alkylation of disulfide bonds, buffer exchange and digestion with trypsin on peptides suitable for LC-MS analysis as described in (Ren et al., Anal. Biochem. 392: 12-21 (2009)). Briefly, a sample comprising Antibody 1 was diluted to about 1 mg/ml in 0.5 ml of pH 7.5 denaturation buffer (7.5 M guanidine hydrochloride (GdnHCl) and 0.25 M Tris). Reduction was accomplished with the addition of 3 µl of 0.5 M dithiothreitol (DTT) followed by 30 min of incubation at room temperature. Carboxy-methylation was achieved with the addition of 7 µl of 0.5 M iodoacetic acid (IAA). The reaction was carried out in the dark for 15 min at room temperature. Excess IAA was quenched with the addition of 4 µl of 0.5 M DTT. Reduced and alkylated Antibody 1 samples were buffer-exchanged into a pH 7.5 digestion buffer (0.1 M Tris or 0.1 M ammonium bicarbonate) using a NAP-5 column (GE Healthcare, Piscataway, NJ, USA). Lyophilized trypsin was dissolved in water to a final concentration of 1 mg/ml. Digestion was started with the addition of the 1-mg/ml trypsin solution to the reduced, alkylated, and buffer-exchanged Antibody 1 samples to achieve a 1:25 enzyme/substrate ratio. Digestion was carried out at 37 °C for 30 min. The final digest was quenched with the addition of 5 tl of 20% FA. LC-MS/MS peptide mapping analysis of the digested Antibody 1 samples was performed on an Agilent 1290 UHPLC system connected to a Thermo Scientific Q-Exactive Biopharma mass spectrometer as described in (Ren *et al.,* 2009, *supra*). Acquired LC-MS/MS raw data and sequences of Antibody 1 and target were used to identify and quantify modifications by MassAnalyzer software (Zhang, Anal.Chem. 81: 8354-8364 (2009)).

### Results

To assess the relevance of the formed attributes to the interaction between Antibody 1 and its target, the Antibody 1 sample was mixed with its target (Figure 2). The first eluting SEC peak (28-29 minutes) contained two target molecules bound to Antibody 1 (bound fraction), while the second eluting SEC peak (29-30 minutes) contained Antibody 1 with one arm unbound to target, disabled by a attribute (Figure 2A) (partially unbound fraction). The third SEC peak (30.5 - 31.5 minutes) was assigned as completely unbound antibody, because its elution time was the same as that of the control -Antibody 1 reference material (completely unbound fraction). The amount of material in the third fraction (completely unbound antibody) was much lower than the first fraction (bound fraction), so the first (bound) fraction and second (partially unbound) fractions, containing similar amounts of the antibody, were compared for better quantitation. Equal sample amounts and sample preparation and analysis conditions improve quality of label-free quantitation used in the method. Unconsumed target (target alone) was the smallest species and eluted around 33 minutes. Because the target) has a small size and mass compared to Antibody 1 (-17 kDa vs. ~150 kDa), complexes of Antibody 1 bound to target showed relatively small shift in elution time, and the SEC peaks partially co-eluted (Figure 2A).

The SEC-separated first fraction (bound) and second (partially unbound) fractions were subjected to tryptic digestion and LC-MS peptide mapping to identify and quantify the amino acid residues and modifications affecting binding. The resulting statistical significance of multiple LC-MS peptide mapping analyses and the fold change (FC) values for modifications in unbound versus bound antibody fractions were presented as the volcano plot (Figure 2B). The plot contained 188 dots for total 188 modifications identified in Antibody 1. Thirty-nine (39) modifications were statistically different in unbound versus bound fractions with a p-value below 0.05. They were located above the horizontal line in Figure 2B and in Figure 3. The rest of the modifications were at similar levels indicating that they did not impact binding or were caused by the sample preparation. The criterion for unbound/bound FC was set up as follows. Since the most negative log2 FC value was at -0.83 on the bottom of Figure 3, positive Log2 FC threshold should be set up at 0.83, and all modifications between were considered not impacting binding. Modifications with log2 FC > 0.83 (FC > 1.78) were considered as relevant to binding. This criterion, together with a requirement of p-value < 0.05, led to the identification of 15 modifications with statistically significant change (Figure 3). Grouping the modifications by residues, seven (7) residues were identified on variable region of heavy and light chains of Antibody 1, which statistically significantly affected binding between Antibody 1 and target (Figure 4). A majority of the identified residues are in the light chain CDR1 (near residue 30) and CDR2 (near residue 50) and heavy chain CDR2 (near residue 50) (Figure 2) and in close proximity to the target in agreement with the crystal structure (data not shown). One residue effecting the Antibody 1-target binding the most was outside of CDRs - cysteine 23 (C23, Figure 4). This finding suggests that long-range (allosteric) effects may play a role caused by the modification of C23, which suggests reduction of the disulfide bond involving C23 and modification of this residue with mass decrease by 15.9941. It is possible that reduced disulfide bond decreases the energy of unfolding (melting temperature), weakens protein structure and leads to loss of binding. It is also possible that the weaker structure without the disulfide bond made the surrounding CDR residues more susceptible to modifications, and the later modifications broke the binding. In any case, modification of C23 correlates with loss of binding, although it is not in close proximity to the target. Only a very minor (but statistically significant) level of C23 modification was observed on the photo stressed sample (<0.12%).

In alternative embodiments, for higher confidence of attribute identification, the corrected p-value (p*) may be considered, which includes the total number of tested modifications as p*=p/N,; where p is the p-value for a test of a single modification, and N is the number of modifications tested (see [0073]). For example, if 188 modifications were in the list identified and quantified by the LC-MS peptide mapping, corrected p-value (p*) should be set to 0.05/188 = 0.000266, negative log10 (0.000266) = 3.6. In practice, the significance threshold (horizontal dash line in Figure 2) is set between negative log10 (p) = 1.3 and negative log10 (p*) = 3.6. Also, there should not be any dots (false-positive results) located in the top-left quadrant of the volcano plot bordered by the dash lines (Figure 2). The two vertical dash lines for the fold change thresholds should be moved symmetrically outward and the horizontal significance line should be moved up until the top-left quadrant is clear. These thresholds will define the modifications will affect binding.

Even after the harsh photo stress of the P2 protocol, the modifications levels were relatively low on the residues affecting binding, below 5% (column "Original P2" in Figures 3, 4). The modifications were below 1% on the non-stressed reference material (data not shown). In general, the relevance of attributes is defined not only by their impact on binding, but also if the modification occurs at any significant level during a typical production process, storage and/or while in human circulation. Other stress conditions for accelerated stability studies typically include: 1) elevated temperature stress in formulation buffer (for example, 37°C for several weeks) and 2) pH increase from mildly acidic formulation buffer to physiological buffer (PBS at pH 7) and incubation at 37°C for several weeks

### EXAMPLE 2

This example describes an exemplary method of the present disclosures wherein the therapeutic protein is Antibody Protein Product 1 (APP1), an Fc fusion protein which binds to Antigen 2.

### Materials and Methods

APP1 is an Fc fusion protein, which binds to two different antigens, one of which is Antigen 2. Antigen 2 is expressed by T-cells. The Fc portion of APP1 is aglycosylated. The molecular weight of APP1 is 106 kDa. APP1 was formulated at a concentration of 1.98 mg/ml in 10 mM glutamic acid and 9% sucrose and having a pH of 4.2. The target of APP1 used in this study was a conjugate comprising two molecules of Antigen 2 fused to a human IgG1 Fc. The Fc portion of the target was glycosylated. The target was formulated at a concentration of 3 mg/ml in 10mM acetic acid (pH 5.2), 100mM NaCl, with 9% Sucrose, 0.38 EU/mg. MW=61,058.5 Da.

APP1 was stressed by changing the formulation pH from 4.2 to 7.0 by dilution in PBS followed by incubation at 37 °C for 2 weeks. The stress was performed on APP1 formulated at a concentration of 1.1 mg/mL in G42SuT in a 0.5 mL vial. APP1 and target were mixed at a 1:1 molar ratio. The mixture was incubated at 37 °C in an HPLC vial for 10 minutes.

### Results

To assess the relevance of the formed attributes to the interaction between APP1 and its target, an APP1 sample was mixed with its target and then fractions were separated by SEC. In SEC, proteins elute in the order of the size and molecular weight from larger sized molecules to smaller sized molecules. The first eluting SEC peak (13-14 minutes) contained the bound fraction comprising APP1 bound to its target (Figure 5A). The second eluting SEC peak (16 minutes) was the binding target species (unbound to APP1), which eluted before APP1 (which has a MW of 106 kDa), suggesting that the binding target species formed a non-covalent dimer having a molecular weight of 122 kDa and a configuration of 2x target.

A large number of modifications was identified and placed on the statistical significance versus fold change volcano plot (Figure 5B). The pH stress followed by 2 weeks of incubation at pH 7 at 37°C induced the formation of several attributes, especially deamidation of asparagine residues. The criterion for unbound/bound fold change was set up as described in Example 1 to identify that FC > 2 (log2 FC > 1) and corrected p-value < 0.05/100 (negative log10 p-value > 3.3) should be statistically significant in decreasing binding. Three modifications on different residues satisfied the criterion and appeared as dots in the top right quadrant (Figure 5B). Out of two target binding domains of APP1, only the Antigen 2 binding domain was assessed in this study. Three residues with attributes with the highest statistical significance and fold change combination were from CDR3 regions of the Antigen 2 binding domain of APP1: (1) HC CDR3 N357, (2) HC CDR3 N360 and (3) LC CDR1 W431. The fact that these changes occurred in the Antigen 2 binding domain validates the accuracy of this method. From their positions along the fold change axis, these three residues should have the following strength of binding N357 > W431 > N360. Fold change values FC N357 = 142 and FC N360 = 3 indicate that N357 plays a much greater role in binding. Several dots (attributes) with relatively high fold change were below the significance threshold of negative log10 (p*) = 3.3. Many of them were from the target-binding and Fc region of the protein, suggesting that were induced during the SEC fraction collection and sample preparation. This probably happened because the concentration of the protein in unbound fraction was lower than in bound, and the unbound protein was subjected to oxidizing species and other impurities. Because these modifications varied from one sample preparation to the other, statistical significance was lower.

### EXAMPLE 3

This example describes an exemplary method of the present disclosures wherein an affinity chromatography - precipitation technique is used. The technique comprises the use of beads labeled with the target or precipitation with cells that express targets at its surface.

In this example, affinity separation (affinity enrichment) of strongly-bound therapeutic protein-target complexes (K_{D} = 10⁻¹¹-10⁻⁹ M) and unbound modified therapeutic protein is performed using binding targets immobilized onto resin beads or binding targets expressed as cell surface proteins (Figure 6A). In a first group, binding target proteins are immobilized onto Actigel ALD Superflow resin (Sterogene Bioseparations, Inc., Carlsbad, CA) according to the manufacturer's protocol. The activated resin comprises a free carbonyl group which conjugates to a primary amine on the target proteins. In a first subgroup, the beads are placed in an affinity chromatography column (Figure 6B). In a second subgroup, the beads are placed in centrifuge tubes with gentle rocking. In a second group, cells expressing the binding targets as cell surface proteins are used in place of binding target proteins immobilized onto beads.

Therapeutic proteins are stressed and subsequently mixed with the beads or cells covered with the binding targets. For the first subgroup, an aliquot of the mixture is injected onto the affinity chromatography column. For the second subgroup, the centrifuge tubes are gently rocked overnight. For the second group, the cells expressing the binding targets at the cell surface are mixed with the stressed therapeutic proteins and incubated in centrifuge tubes with gentle rocking. After the mixing, the centrifuge tubes are centrifuged to separate the beads or cells comprising the bound fraction comprising therapeutic protein-binding target complexes (located in the pellet) from the unbound fraction comprising therapeutic proteins unbound to binding target stay (located in the supernatant) (Figure 6A). For the first subgroup of the first group, fractions are eluted off the column with an appropriate elution buffer so that the bound fraction comprising therapeutic protein-binding target complexes are retained on the column while the unbound fraction comprising therapeutic proteins not bound to binding targets elute from the column. For the first group involving the resin, the conditions for these steps is similar to those described for the purification of antibodies of interest from blood described in Liu et al., J.Biol.Chem. 283: 29266-29272 (2008) and/or Goetze et al., Glycobiology 21: 949-959 (2011), for example.

The separated bound and unbound fractions are subjected to LC-MS peptide mapping to identify the amino acid residues and their attributes affecting binding to produce results according to Figure 1 and as essentially described in Example 1.

Antibodies, BITEs, proteins, Fc-fusion proteins can be assessed by the method described in this example. This method is thought to be capable of fractionating larger amounts of bound and unbound therapeutic proteins as compared to the method of Example 1 using SEC. The method of this example also provides the advantage for instances in which production of extracellular, soluble regions of the target is challenging, and the target is only produced on the cell surface.

### EXAMPLE 4

This example describes an exemplary method of the present disclosures wherein the affinity purification technique comprises affinity chromatography, precipitation using binding target-labeled beads or precipitation with cell surface binding targets. In this example the therapeutic protein comprises an Fc of an antibody and the binding target is an Fc receptor.

In this example, affinity separation (affinity enrichment) of weakly-bound Fc-containing therapeutic protein-Fc receptor complexes (K_{D} = 10⁻⁸-10⁻⁶ M) and unbound modified Fc containing proteins is performed using Fc receptors immobilized onto resin beads or Fc receptors expressed by cells. In a first group, Fc receptors are immobilized onto Actigel ALD Superflow resin (Sterogene Bioseparations, Inc., Carlsbad, CA) according to the manufacturer's protocol. The activated resin comprises a free carbonyl group which conjugates to a primary amine on the Fc receptors. In a first subgroup, the Fc receptor-bound beads are placed in an affinity chromatography column (Figure 7B). In a second subgroup, the beads are placed in centrifuge tubes with gentle rocking (Figure 7A). In a second group, cells expressing the Fc receptors at the cell surface are used in place of beads.

Fc-containing therapeutic proteins are stressed and subsequently mixed with the beads or cells covered with the Fc receptors. For the first subgroup, an aliquot of the mixture is injected onto the affinity chromatography column. For the second subgroup, the centrifuge tubes are gently rocked overnight. For the second group, the cells expressing the Fc receptors are mixed with the stressed Fc containing therapeutic proteins and incubated in centrifuge tubes with gentle rocking. After the mixing, the centrifuge tubes are centrifuged to separate the beads or cells comprising the bound fraction comprising Fc containing therapeutic proteins-Fc receptor complexes (located in the pellet) from the unbound fraction comprising Fc containing therapeutic proteins unbound to Fc receptors stay (located in the supernatant). For the first subgroup of the first group, fractions are eluted off the column with an appropriate elution buffer so that the bound fraction comprising Fc containing therapeutic protein-Fc receptor complexes are retained on the column while the unbound fraction comprising Fc containing therapeutic proteins not bound to Fc receptors elute from the column. For the first group involving the resin, the conditions for these steps is similar to those described for the purification of antibodies of interest from blood described in Liu et al., J.Biol.Chem. 283: 29266-29272 (2008) and/or Goetze et al., Glycobiology 21: 949-959 (2011), for example.

The separated bound and unbound fractions are subjected to LC-MS peptide mapping to identify the amino acid residues and their modifications affecting binding to produce results according to Figure 1 and as essentially described in Example 1.

### EXAMPLE 5

This example describes an exemplary method of determining the criticality of an attribute of a therapeutic protein.

It is known that Fc-containing proteins bind to Fc receptors with an affinity characterized by a K_{D} of about 10⁻⁸ to about 10⁻⁶ M. In this experiment, the procedures described in Example 3 are carried out, except that a library of species of a therapeutic fusion protein comprising an Fc region with known attributes is used. Each member of the library is the same therapeutic fusion protein but has a unique attribute (relative to the other members of the library). Affinity purification is carried out with beads bound to Fc receptors. In some instances, the beads are coated with Protein A which binds to Fc receptors. The fraction comprising unbound Fc fusions (found in the supernatant) are analyzed via LC-MS peptide mapping side-by-side with the fraction comprising bound fusion protein-binding target complexes. For each species of each fraction, the abundance of the species of the fraction comprising unbound Fc fusions is compared to the abundance of the species in the fraction comprising bound fusion protein-target complexes. For those species having an abundance ratio greater than 1 are species considered as containing an attribute relevant to the binding interaction between the therapeutic protein and the target.

### EXAMPLE 6

This example demonstrates the identification of critical chemical modifications and paratope mapping by size exclusion chromatography (SEC) of stressed antibody-target complexes.

### Introduction

Therapeutic proteins including antibodies and Fc-fusion proteins undergo a large number of chemical modifications during manufacturing and storage including deamidation, oxidation, isomerization, hydroxylation, glycation, glycosylation, clips and others. They are also exposed to harsh conditions during stress studies, including forced oxidation, extremes of pH, UV light to study the possible degradation pathways and suitability of methods for detecting them. Some of these modifications are located on residues in binding regions, leading to loss of binding/potency and classified as critical quality attributes. Conventionally, criticality of modifications can be assessed by a laborious process of fractionating and characterizing intact protein variants one-by-one. Here we describe a method for large-scale, parallel, automated criticality assessment of potentially all modifications, and for paratope-epitope mapping of antibody-target binding sites.

### Methods

Therapeutic antibody sample was treated with UV light for several hours designed to yield multiple chemical modifications in an antibody population. Stressed antibody was then incubated with its target protein, and the mixture was fractionated by SEC on earlier eluting antibody-target complex and later eluting unbound antibody. The fractions were collected and the modifications of the antibody in each fraction were identified and quantified by LC-MS/MS peptide mapping using Agilent 1290 HPLC and Thermo QE-Biopharma MS. The procedure was repeated several times (n=3) for statistical significance. To distinguish the critical modifications affecting binding from noncritical modifications and artificial modifications caused by the sample preparation, a volcano plot was used for statistical significance versus the ratio (fold change) of modifications in unbound/bound antibody species (Figures 8A-8D).

### Preliminary Data

For typically strong therapeutic antibody - target binding affinity ranges of K_{D} = 10⁻¹¹-10⁻⁹ M, antibody and target elute together as a complex from SEC. When the affinity of a stressed antibody becomes weaker than K_{D}=10⁻⁸ M due to the chemical modifications, the unbound antibody elutes separately from its target. In this study, peptide mapping of the bound and unbound antibody molecules identified and quantified 275 chemical modifications, which were presented as the volcano plot (Figure 9). 27 modifications were indicated by high statistical significance (p-value < 0.006) and fold change (>1.7) in unbound/bound antibody species. These modifications occurred on 11 residues identified as paratope of the antibody (Figures 10A-13I). Paratope map was plotted as a modification score versus residue number (Figures 10A-10B). The modification score was chosen as a sum of logarithms for fold change and p-value (Log2 Fold Change + -log10 p-value), because the scales have similar values and both are important for separating signal from noise. Out of 11 residues with highest score, 10 were located at complementarity determining regions (CDRs), regions typically involved in binding. Residues in the conserved regions, typically not involved in binding, had significantly lower score. Correlation to crystallography data of the antibody-target complex revealed that 8 out of the 11 critical antibody residues were within 5A from the target, indicating that these antibody residues are close and likely to interact with the target. These results supported validity of the method. The only critical modification detected outside of CDRs was on a cysteine residue (C23) near CDR1, suggesting that long-range (allosteric) effects may play a role caused by the reduced disulfide bond. The method utilizes modifications on amino acid side chains for mapping protein-protein interactions, similar to Fast Photochemical Oxidation of Proteins (FPOP), but it does not require fast photochemical oxidation using lasers. This approach is especially relevant in situations when other structural data (from crystallography or NMR) are not available during early product development of the therapeutic antibody.

Accordingly, a method is provided for identification of critical chemical modifications and paratope mapping by SEC of stressed antibody-target complexes followed by peptide mapping of bound and unbound antibody species

### EXAMPLE 7

This example demonstrates a competitive SEC affinity separation for identification of antibody modifications impacting binding to target protein.

### Introduction

A large number of attributes are typically identified for therapeutic proteins (e.g., antibodies, BiTE^{®} molecules, therapeutic proteins, Fc-fusion proteins, etc.) including glycosylation, hydroxylation, glycation, deamidation, oxidation, isomerization, clips and other chemical modifications. Attributes include post-translational modifications of therapeutic proteins taking place during cell culture and chemical modifications/degradations occurring during production, purification, formulation and storage. Some of these modifications occur in binding regions and may impact binging to target and efficacy. Efficacy of therapeutic protein is one of the main criteria for attribute criticality assessment. The method describes the experimental criticality assessment of attributes by competitive affinity binding between an antibody and its target Her2, followed by SEC fractionation and LC-MS/MS peptide mapping characterization of bound and unbound therapeutic proteins (Figure 14).

### Methods

A soluble part of human recombinant Her2 receptor was used for binding to a stressed antibody. A Tosoh TSK Gel G3000SWXL column was used for SEC separation of bound antibody-antigen complexes and unbound antibody. The stressed antibody (45C10d)-receptor samples were also analyzed using SEC-UV-MALS system to determine the binding stoichiometry (Figures 16A-16C). Corresponding fractions from each SEC run were collected, digested by trypsin, and then analyzed using LC-MS/MS. Mass spectrometry data were searched against the mAb heavy chain and light chain, the receptor, and trypsin using MassAnalyzer. The volcano plot, a type of scatter-plot, was used to represent the peptide mapping results to identify changes in large data sets composed of replicate data.

### Preliminary data

In the method, the characterization includes identification and quantitation of modifications in the bound and unbound therapeutic protein faction with the goal to identify the critical quality attributes responsible for the loss of binding. Protein-ligand complexes, including antibody-target remain bound during SEC separation if equilibrium dissociation constant Kd < 10-8M. The method was applied to several antibody:target complexes by mixing them in a ratio defined by stoichiometry of interactions, typically 1:2. In case of antibody-Her2 complex, however, modifications after stress did not lead to loss of binding, suggesting that Kd < 10-8M even for stressed and degraded antibody molecules. When smaller amount of the receptor was provided (1:1), the antibody species with modifications reducing binding would elute as unbound from SEC (Figures 15A-15G). The fractions were collected (Figure 17) for two experiments: 1) CEX and 2) reduced peptide mapping. For peptide mapping, protein fraction were collected, denatured high guanidine, reduced, alkylated and digested.

The CEX data showed that bound fraction contained the main species, while unbound fraction included a large percentage of acidic and basic variants (Figures 18A-18D). Peptide mapping revealed ratios of modifications and their p-values after triplicate measurements (n=3) shown as volcano plot (Figures 19, 20A-20E). Top-right region should contain modifications impacting binding the most. Several modifications with ratio > 1.5 were summarized in the abundance plot (Figure 21). HC D102 isomerization and LC N30 deamidation were four-fold higher in unbound fraction and with high statistical significance. Although HC N55 deamidation and M107 oxidation were also abundant, they were not statistically different between unbound and bound.

Our findings are in agreement with previously published potency measurements (Figures 22A-22B). Several modifications on HC and LC N-termini correlated to non-binding. Close review of antibody-Her2 crystal structure from Cho et al 2003 indicated that HC and LC N-termini come close to target, so the terminal modifications may indeed lead to loss of binding (Figures 22A-22B).

Thus, it is shown that competitive SEC of stressed antibody/target mixture followed by peptide mapping revealed residues and modifications reducing, but not eliminating binding.

### REFERENCES

The following references are cited in Example 6:
Abbott WM, Damschroder MM, Lowe DC. 2014. Current approaches to fine mapping of antigen-antibody interactions. Immunology 142: 526-535.
Blech M, Peter D, Fischer P, Bauer MM, Hafner M, Zeeb M, Nar H. 2013. One target-two different binding modes: structural insights into gevokizumab and canakinumab interactions to interleukin-1beta. J Mol.Biol. 425: 94-111.
Dhungana S, Fessler MB, Tomer KB. 2009. Epitope mapping by differential chemical modification of antigens. Methods Mol.Biol. 524: 119-134.
Englander SW. 2006. Hydrogen exchange and mass spectrometry: A historical perspective. J Am.Soc.Mass Spectrom 17: 1481-1489.
Goswami D, Zhang J, Bondarenko PV, Zhang Z. 2018. MS-based conformation analysis of recombinant proteins in design, optimization and development of biopharmaceuticals, https://doi.org/10.1016/j.ymeth.2018.04.011. Methods 144: 134-151.
Jones LM, Sperry B, Carroll A, Gross ML. 2011. Fast photochemical oxidation of proteins for epitope mapping. Anal Chem 83: 7657-7661.
Luo J, Liu Z, Guo Y, Li M. 2015. A structural dissection of large protein-protein crystal packing contacts. Sci.Rep. 5: 14214.
Opuni KFM, Al-Majdoub M, Yefremova Y, El-Kased RF, Koy C, Glocker MO. 2018. Mass spectrometric epitope mapping. Mass Spectrom Rev. 37: 229-241.
Sevy AM, Healey JF, Deng W, Spiegel PC, Meeks SL, Li R. 2013. Epitope mapping of inhibitory antibodies targeting the C2 domain of coagulation factor VIII by hydrogen-deuterium exchange mass spectrometry. J Thromb.Haemost. 11: 2128-2136.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

Preferred embodiments of this disclosure are described herein, including the best mode known to the inventors for carrying out the disclosure. Variations of those preferred embodiments may become apparent to those of ordinary skill in the art upon reading the foregoing description. The inventors expect skilled artisans to employ such variations as appropriate, and the inventors intend for the disclosure to be practiced otherwise than as specifically described herein. Accordingly, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

In view of the above, it will be appreciated that the invention also encompasses the following items:
1. A method of identifying structures of a therapeutic protein or a target that affect an interaction between the therapeutic protein and the target, said method comprising:
   a. applying a stress to a first sample comprising therapeutic proteins or targets;
   b. contacting the first sample with a second sample comprising therapeutic proteins or targets to form a mixture comprising (i) therapeutic protein-target complexes, (ii) unbound therapeutic proteins, and (iii) unbound targets, wherein the first sample comprises the therapeutic proteins and the second sample comprises the targets, or
      wherein the first sample comprises the targets and the second sample comprises the therapeutic proteins;
   c. separating the mixture into at least two fractions, wherein an unbound fraction comprises unbound therapeutic proteins or targets and a bound fraction comprises therapeutic protein-target complexes;
   d. for each of the unbound fraction and bound fraction, identifying and quantifying the abundance of a structure present on a species of the therapeutic protein or target, wherein, when the abundance of a structure in the unbound fraction is greater than the abundance of the structure in the bound fraction, the structure negatively affects the interaction between the therapeutic protein and the target.
2. The method of item 1, wherein the structure comprises or consists of an attribute of the target.
3. The method of item 2, wherein, when the structure has a negative effect on the interaction between the therapeutic protein and the target, the structure is a candidate for being part of an epitope on the target.
4. The method of item 1, wherein the structure comprises or consists of an attribute of the therapeutic protein.
5. The method of item 4, wherein, when the structure has a negative effect on the interaction between the therapeutic protein and the target, the structure is a candidate for being part of a paratope of the therapeutic protein.
6. The method of item 4 or 5, comprising:
   a. applying a stress to a first sample comprising therapeutic proteins;
   b. contacting the first sample with a second sample comprising targets to form a mixture comprising (i) therapeutic protein-target complexes, (ii) unbound therapeutic proteins, and (iii) unbound targets;
   c. separating the mixture into at least two fractions, wherein an unbound fraction comprises unbound therapeutic proteins and a bound fraction comprises therapeutic protein-target complexes;
   d. for each of the unbound fraction and bound fraction, identifying and quantifying the abundance of structures present on a species of the therapeutic protein, wherein, when the abundance of an structure in the unbound fraction is greater than the abundance of the attribute in the bound fraction, the structure negatively affects the interaction between the therapeutic protein and the target.
7. The method of item 6, wherein, when the structure on the species the therapeutic protein negatively affects the interaction between the therapeutic protein and the target, the therapeutic protein is a candidate for being part of a paratope of the therapeutic protein.
8. A method of determining an effect of a known structure present on a species of a therapeutic protein or a target on an interaction between the therapeutic protein and the target, said method comprising:
   a. contacting a first sample comprising a therapeutic protein or target with a known structure with a second sample comprising targets or therapeutic proteins to form a mixture comprising (i) therapeutic protein- target complexes, (ii) unbound therapeutic proteins, and (iii) unbound targets,
      wherein the first sample comprises the therapeutic proteins and the second sample comprises the targets, or
      wherein the first sample comprises the targets and the second sample comprises the therapeutic proteins;
   b. separating the mixture into at least two fractions, wherein an unbound fraction comprises unbound therapeutic proteins or targets and a bound fraction comprises therapeutic protein-target complexes;
   c. for each of the unbound fraction and bound fraction, quantifying the abundance of the known structure, wherein, when the abundance of the known structure in the unbound fraction is greater than the abundance of the known structure in the bound fraction, the known structure has a negative effect on the interaction between the therapeutic protein and the target.
9. The method of item 8, wherein the known structure comprises or consists of an attribute of the target.
10. The method of item 9, wherein, when the known structure has a negative effect on the interaction between the therapeutic protein and the target, the structure is a candidate for being part of an epitope on the target.
11. The method of item 8, wherein the known structure comprises or consists of an attribute of the therapeutic protein.
12. The method of item 11, wherein, when the known structure has a negative effect on the interaction between the therapeutic protein and the target, the structure is a candidate for being part of a paratope of the therapeutic protein.
13. The method of item 11 or 12, comprising:
   a. contacting a first sample comprising a therapeutic protein with a known attribute with a second sample comprising targets to form a mixture comprising (i) therapeutic protein- target complexes, (ii) unbound therapeutic proteins, and (iii) unbound targets;
   b. separating the mixture into at least two fractions, wherein an unbound fraction comprises unbound therapeutic proteins and a bound fraction comprises therapeutic protein-target complexes;
   c. for each of the unbound fraction and bound fraction, quantifying the abundance of the known attribute, wherein, when the abundance of the known attribute in the unbound fraction is greater than the abundance of the known attribute in the bound fraction, the known attribute has a negative effect on the interaction between the therapeutic protein and the target.
14. The method of any one of the preceding claims, wherein the first sample comprises a population of species of the therapeutic protein.
15. The method of item 14, wherein the population comprises more than one species of the therapeutic protein having a unique attribute profile.
16. The method of item 14 or 15, wherein the species of the therapeutic protein are the only proteins present in the first sample.
17. The method of item 16, wherein the first sample comprises (i) the population of therapeutic proteins and (ii) a pharmaceutically-acceptable carrier, diluent, excipient, or a combination thereof.
18. The method of item 14 or 15, wherein the first sample comprises the species of the therapeutic protein and serum, or a serum fraction.
19. The method of item 18, wherein the serum or serum fraction comprises one or more serum proteins.
20. The method of any one of the preceding claims, wherein the second sample comprises a population of targets and the targets are proteins.
21. The method of item 20, wherein each of the targets of the second sample has the same amino acid sequence.
22. The method of item 21, wherein the targets are the only proteins present in the second sample.
23. The method of item 21, wherein the second sample comprises the targets and serum or a serum fraction.
24. The method of item 23, wherein the serum or serum fraction comprises one or more serum proteins.
25. The method of any one of the preceding claims, wherein the mixture is separated into at least two fractions using a technique that separates components of a mixture based on size, charge, hydrophobicity, affinity for a capture molecule, or a combination thereof.
26. The method of item 25, wherein the technique is size exclusion chromatography (SEC), affinity chromatography, precipitation using beads or cells, free flow fractionation (FFF), ion exchange chromatography (IEX), hydrophobic interaction chromatography (HIC), or ultracentrifugation (UC).
27. The method of item 25 or 26, wherein the mixture is separated into at least two fractions using a technique that separates components of a mixture based on size, optionally, wherein the technique is size exclusion chromatography (SEC).
28. The method of item 25 or 26, wherein the mixture is separated into at least two fractions using a technique that separates components of a mixture based on affinity for a capture molecule bound to a solid support, optionally, a bead or a cell.
29. The method of item 28, wherein the mixture is separated by (i) adding the mixture to a container comprising beads bound to the capture molecule or cells expressing at its surface the capture molecule, (ii) centrifuging the container to obtain a supernatant and a pellet, (iii) collecting the supernatant from the pellet to obtain the unbound fraction, (iv) releasing the bound fraction from the pellet with a solution, (v) centrifuging the container comprising the pellet and the solution to obtain a second supernatant comprising the bound fraction and a second pellet comprising the beads or cells, and (vi) collecting the second supernatant to obtain the bound fraction.
30. The method of item 28, wherein the mixture is separated by (i) adding the mixture to a column comprising beads bound to the capture molecule to obtain a flow-through and a bound fraction (ii) collecting the flow-through to obtain the unbound fraction, (iii) releasing the bound fraction from the beads with a solution and collecting the solution comprising the bound fraction.
31. The method of any one of items 1-7 and 14-30, comprising using a mass spectrometer to identify and quantify the abundance of each attribute of the species of the therapeutic protein in each of the unbound fraction and bound fraction.
32. The method of any one of items 8-30, comprising using a mass spectrometer to quantify the abundance of the known attribute in each of the unbound fraction and bound fraction.
33. The method of item 31 or 32, wherein each of the unbound fraction and bound fraction are subjected to a peptide digestion step, optionally, a trypsin digestion step, prior to the quantifying step or identifying and quantifying step.
34. The method of any one of items 31-33, comprising carrying out liquid chromatography/mass spectrometry (LC/MS).
35. The method of any one of the preceding items, comprising determining, for each attribute, a ratio of the abundance of the attribute in the unbound fraction to the abundance of the attribute in the bound fraction.
36. The method of item 35, wherein, when the ratio is greater than about 1, the attribute negatively affects the interaction between the therapeutic protein and the target.
37. The method of item 35, wherein when the ratio is greater than about 1.5, the attribute affects the interaction between the therapeutic protein and the target.
38. The method of item 35, wherein when the ratio is greater than about 2.0, the attribute affects the interaction between the therapeutic protein and the target.
39. The method of item 35, wherein the ratio is expressed as log₂.
40. The method of item 39, wherein, when the log₂ ratio is greater than about 0.58, the attribute affects the interaction between the therapeutic protein and the target.
41. The method of item 39, wherein, when the log₂ ratio is greater than about 1.0, the attribute affects the interaction between the therapeutic protein and the target.
42. The method of any one of the preceding items, wherein the method comprises repeating steps of the method, optionally, the separating and quantifying steps, wherein, each time the steps are repeated, a ratio is determined for each attribute to obtain a plurality of ratios and the method comprises determining the statistical significance of the plurality of ratios.
43. The method of item 42, comprising repeating the steps at least two, three, or four times.
44. The method of item 43, comprising repeating the steps at least five or more times.
45. The method of any one of items 42-44, wherein determining the statistical significance of the plurality of ratios comprises calculating the p-value, optionally, wherein the p-value is calculated by a t-test.
46. The method of item 45, wherein, when the *p*-value is less than 0.05 or the negative log₁₀ *p-*value is greater than 1.3, the attribute negatively affects the interaction between the therapeutic protein and the target.
47. The method of any one of items 45 or 46, wherein determining the statistical significance of the plurality of ratios comprises calculating the corrected *p*-value (*p**) of the ratios, wherein *p** = *p*/N, wherein *p* is the *p*-value and N is the total number of tested attributes.
48. The method of any one of items 42-47, further comprising plotting the average ratio or mean ratio for each attribute in a volcano plot, wherein the statistical significance of the ratio is plotted as a function of the ratio.
49. The method of any one of items 1-7 and 14-48, wherein the stress applied to the first sample leads to formation of at least one species of the therapeutic protein having a unique attribute profile relative to the attribute profile of the therapeutic protein prior the applied stress.
50. The method of any one of items 8-46, wherein the known attribute was engineered at a known amino acid position of the therapeutic protein prior to being contacted with the second sample.
51. The method of any one of the preceding items, wherein the attribute forms as a result of a chemical modification, optionally, wherein the chemical modification alters the mass-to-charge ratio (m/z) of charged ions of an amino acid of the therapeutic protein.
52. The method of item 51, wherein the chemical modification is a glycosylation, hydroxylation, glycation, deamidation, oxidation, reduction, isomerization, aggregation, degradation, acetylation, or clipping due to hydrolysis proteolysis.
53. The method of any one of the preceding items, wherein the stress is an exposure to ultraviolet light, heat, air, freeze/thaw cycle, shaking, long-term storage, change in pH, or change in temperature, optionally, wherein the change in pH is greater than about 1.0 or greater than about 2.0, optionally, wherein the change in temperature is greater than or about 2 degrees Celsius or greater than or about 5 degrees Celsius.
54. The method of any one of the preceding items, wherein the therapeutic protein comprises an antibody, antigen-binding antibody fragment, or antibody protein product, optionally, a Bi-specific T cell engager (BiTE), bispecific antibody, trispecific antibody, Fc fusion protein.
55. The method of item 48, comprising a single chain Fragment variable (scFv) which binds to a target.
56. The method of any one of items 1-55, wherein the therapeutic protein has an equilibrium dissociation constant (K_{D}) for the target that is about 10⁻¹¹ to about 10⁻⁹ M.
57. The method of item 56, wherein the mixture is separated into at least two fractions by SEC or by precipitation using beads or cells.
58. The method of item 57, comprising adding the mixture to a container comprising beads bound to the capture molecule or cells expressing at its surface the capture molecule, (ii) centrifuging the container comprising the mixture and the beads or cells, and (iii) removing the supernatant from the pellet to obtain a first fraction and a second fraction, optionally, wherein the capture molecule is FcRn.
59. The method of any one of items 1-55, wherein the therapeutic protein has an equilibrium dissociation constant (K_{D}) for the target that is about 10⁻⁸ to about 10⁻⁶ M.
60. The method of item 59, wherein the mixture is separated into at least two fractions by precipitation using beads or cells bound to capture molecule.
61. The method of item 60, wherein the capture molecule is FcRn.
62. The method of item 60, where is the capture molecule in Fc gamma receptor.

## Claims

1. A method of determining an effect of a known structure present on a species of a therapeutic protein or a target on an interaction between the therapeutic protein and the target, said method comprising:
a. contacting a first sample comprising therapeutic proteins having a known structure with a second sample comprising targets to form a mixture comprising (i) therapeutic protein-target complexes, (ii) unbound therapeutic proteins, and (iii) unbound targets;
b. separating the mixture into at least two fractions, wherein an unbound fraction comprises unbound therapeutic proteins or targets and a bound fraction comprises therapeutic protein-target complexes;
c. for each of the unbound fraction and bound fraction, quantifying the abundance of the known structure, wherein the known structure comprises an attribute of the therapeutic protein and, when the abundance of the known structure in the unbound fraction is greater than the abundance of the known structure in the bound fraction, the known structure has a negative effect on the interaction between the therapeutic protein and the target.

2. The method of claim 1, wherein, when the structure has a negative effect on the interaction between the therapeutic protein and the target, the structure is a candidate for being part of a paratope of the therapeutic protein.

3. The method of claim 1 or 2, wherein the first sample comprises a population of species of the therapeutic protein, optionally more than one species of the therapeutic protein having a unique attribute profile.

4. The method of claim 3, wherein the first sample further comprises serum or a serum fraction.

5. The method of any one of the preceding claims, wherein the second sample comprises a population of targets and the targets are antigens, optionally wherein each of the antigens has the same amino acid sequence.

6. The method of any one of the preceding claims, wherein the mixture is separated into at least two fractions using size exclusion chromatography (SEC), affinity chromatography, precipitation using beads or cells, free flow fractionation (FFF), ion exchange chromatography (IEX), hydrophobic interaction chromatography (HIC), or ultracentrifugation (UC).

7. The method of any one of claims 1-6, comprising using a mass spectrometer to quantify the abundance of the attribute in each of the unbound fraction and bound fraction.

8. The method of any one of claims 1-7, comprising determining, for each attribute, a ratio of the abundance of the attribute in the unbound fraction to the abundance of the attribute in the bound fraction, wherein when the ratio is greater than 1, the attribute negatively affects the interaction between the therapeutic protein and the target.

9. The method of any one of claims 1-8, wherein the method comprises repeating steps of the method.

10. The method of any one of claims 1-9, wherein the known structure comprises an attribute that was engineered at a known amino acid position of the therapeutic protein prior to being contacted with the second sample.

11. The method of any one of claims 1-10, wherein the attribute forms as a result of a chemical modification, optionally, wherein the chemical modification alters the mass-to-charge ratio (m/z) of charged ions of an amino acid of the therapeutic protein.

12. The method of claim 11, wherein the chemical modification is a glycosylation, hydroxylation, glycation, deamidation, oxidation, reduction, isomerization, aggregation, degradation, acetylation, or clipping due to hydrolysis proteolysis.

13. The method of any one of the preceding claims, wherein the therapeutic protein comprises an antibody, an antigen-binding antibody fragment, a Bi-specific T cell engager molecule, a bispecific antibody, a trispecific antibody, or an Fc fusion protein.

14. The method of any one of claims 1-13, wherein the therapeutic protein comprises an IgG antibody.
